(19) Europäisches Patentamt
European Patent Office
Office européen des brevets

(11) EP 4 703 390 A1

(12) EUROPEAN PATENT APPLICATION
published in accordance with Art. 153(4) EPC

(43) Date of publication:
04.03.2026 Bulletin 2026/10

(21) Application number: 24796051.1

(22) Date of filing: 23.04.2024

(51) International Patent Classification (IPC):
C07K 16/46 (2006.01)        C07K 16/28 (2006.01)
C07K 16/30 (2006.01)        A61K 39/395 (2006.01)
C12N 15/13 (2006.01)        A61P 35/00 (2006.01)

(52) Cooperative Patent Classification (CPC):
A61K 39/395; A61P 35/00; C07K 16/00;
C07K 16/28; C07K 16/30; C07K 16/46

(86) International application number:
PCT/CN2024/089297

(87) International publication number:
WO 2024/222671 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 24.04.2023 CN 202310459592

(71) Applicant: Chia Tai Tianqing Pharmaceutical
Group Co., Ltd.
Lianyungang, Jiangsu 222062 (CN)

(72) Inventors:
• ZHANG, Bing
Lianyungang, Jiangsu 222062 (CN)
• DU, Min
Lianyungang, Jiangsu 222062 (CN)
• LU, Yamin
Lianyungang, Jiangsu 222062 (CN)
• ZHEN, Zipeng
Lianyungang, Jiangsu 222062 (CN)
• WANG, Xin
Lianyungang, Jiangsu 222062 (CN)
• DU, Xiuzhen
Lianyungang, Jiangsu 222062 (CN)

(74) Representative: Dehns
10 Old Bailey
London EC4M 7NG (GB)

Remarks:
The complete document including Reference Table(s) and the Sequence Listing(s) can be downloaded from the EPO website

(54) MULTISPECIFIC ANTIBODY TARGETING C-MET

(57) Provided is a multispecific antibody targeting c-Met. Specifically provided are multispecific antibodies, nucleic acids encoding the antibodies, vectors containing the nucleic acids, cells containing the vectors, and pharmaceutical compositions containing the multispecific antibodies, and further provided are uses thereof for treating related diseases.

FIG. 2

EP 4 703 390 A1

**Description**

**TECHNICAL FIELD**

The present disclosure relates to a multispecific antibody, and in particular, to a multispecific antibody targeting EGFR and c-Met. The present disclosure further relates to a method for preparing the multispecific antibody and use thereof.

**BACKGROUND**

Epidermal growth factor receptor (EGFR, also known as ErbB1 or HER1) is a 170 kDa type I transmembrane glycoprotein encoded by the proto-oncogene c-erbB 1, and is one of the receptor tyrosine kinases (RTKs). EGFR is a member of the human epidermal growth factor receptor (HER) family, which include HER2 (ErbB2), HER3 (ErbB3), and HER4 (ErbB4). Increased expression or kinase activity of EGFR is associated with a series of human tumors, making EGFR an attractive target for tumor therapy.

The c-mesenchymal-epithelial transition factor (c-Met) is one of the receptor tyrosine kinases and a heterodimer of approximately 190 kDa formed by linking a 50 kDa extracellular chain ($\alpha$ chain) and a 145 kDa transmembrane chain ($\beta$ chain). The transmembrane chain ($\beta$ chain) includes a SEMA domain (sema homology region, SEMA), a PSI domain (plexin semaphorin-integrin, PSI), 4 immunoglobulin-like repeat domains (immunoglobulin-like regions in plexins and transcription factors, IPT), a transmembrane domain, a juxtamembrane domain (JM), a tyrosine kinase domain (TK), and a carboxy-terminal tail region (carboxyl terminal, CT). c-Met is a receptor expressed on the cell surface, in which the SEMA domain is one of the important elements for ligand binding and is considered as a binding site of its ligand, hepatocyte growth factor (HGF). HGF is synthesized by mesenchymal cells, fibroblasts, and smooth muscle cells, and activates the HGF/c-Met signaling through a paracrine mechanism to exert its biological functions.

In various tumors, due to the overexpression of c-Met and HGF and the paracrine and autocrine positive feedback loops formed by HGF and c-Met, the HGF/c-Met signaling pathway is abnormally activated, and the growth, invasion, migration, and angiogenesis of tumor cells are promoted. The abnormal expression of the c-Met gene is present in many cancer types, such as brain cancer, breast cancer, colorectal cancer, gastric cancer, head and neck cancer, lung cancer, and liver cancer. Immunotherapeutic agents such as antibodies that bind to c-Met are able to block the binding of HGF to c-Met.

In addition to conventional IgG1 of a four-chain structure, IgG antibodies in Camelidae species also include IgG2 and IgG3 heavy-chain only antibodies (HcAbs) free of light chains. The single-variable-region domains ($V_HHs$) of heavy-chain only antibodies are capable of specifically binding to antigens and have relatively high affinity for antigens. Based on their uniqueness, $V_HH$ domains used as part of an antibody or an antigen-binding fragment have more significant advantages over conventional antibody fragments (such as scFvs and Fabs): for example, only a single domain is required to specifically bind to an antigen with high affinity; they can be easily transformed into polyvalent and multispecific formats; $V_HH$ domains are highly soluble and do not tend to aggregate; the $V_HH$ molecules are small and thus have relatively high tissue permeability; $V_HHs$ do not need to be paired with light chains, and thus there is no light and heavy chain mismatch problem about forming bispecific or multispecific antibodies.

About 60% of all tumor patients resistant to EGFR tyrosine kinase inhibitors have increased c-Met expression, c-Met amplification, or increased HGF, indicating that c-Met will be activated as a compensatory pathway in the case of EGFR inhibition.

Therefore, there is a need to develop multispecific antibodies targeting EGFR and c-Met.

**BRIEF SUMMARY**

The present disclosure provides a multispecific antibody targeting EGFR and c-Met, and further provides a related nucleic acid capable of encoding the provided antibody, a vector, a cell, a pharmaceutical composition, a method for preparing same, and use.

In one aspect, the present disclosure provides a multispecific antibody, comprising:

(i) a first antigen-binding moiety that binds to a first antigen;
(ii) a second antigen-binding moiety that binds to a second antigen; and
(iii) a third antigen-binding moiety that binds to the first antigen,

wherein the first antigen is c-Met, and the second antigen is EGFR; and the first antigen-binding moiety and the third antigen-binding moiety are both single variable domains and each independently comprise any one of the following:

(1) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;

(2) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is S or T;

(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;

(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;

(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;

(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;

(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

In some embodiments, the first antigen-binding moiety and the third antigen-binding moiety each independently comprise any one of the following:

(2) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is S or T;

(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

In some embodiments, the first antigen-binding moiety and the third antigen-binding moiety each independently comprise any one of the following:

(2) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is S or T; or

(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9. In some embodiments, the first antigen-binding moiety and the third antigen-binding moiety each independently comprise any one of the following:

(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

In some embodiments, the first antigen-binding moiety and the third antigen-binding moiety each independently comprise a CDR1, a CDR2, and a CDR3 of a single variable domain having the amino acid sequence set forth in SEQ ID NO: 25, 26, 27, 28, 29, 30, 31, 32, 33, 34, 35, 36, 37, 38, or 39. In some embodiments, the first antigen-binding moiety and the third antigen-binding moiety each independently comprise a CDR1, a CDR2, and a CDR3 of a single variable domain having the amino acid sequence set forth in SEQ ID NO: 35, 36, or 38.

In some embodiments, the first antigen-binding moiety and the third antigen-binding moiety each independently comprise a CDR1, a CDR2, and a CDR3 of a single variable domain having the amino acid sequence set forth in SEQ ID NO: 35 or 36. In some other embodiments, the first antigen-binding moiety and the third antigen-binding moiety each independently comprise a CDR1, a CDR2, and a CDR3 of a single variable domain having the amino acid sequence set forth in SEQ ID NO: 36 or 38.

In some specific embodiments, the first antigen-binding moiety comprises a CDR1, a CDR2, and a CDR3 of a single variable domain having the amino acid sequence set forth in SEQ ID NO: 36, and the third antigen-binding moiety comprises a CDR1, a CDR2, and a CDR3 of a single variable domain having the amino acid sequence set forth in SEQ ID NO: 35. In some other specific embodiments, the first antigen-binding moiety comprises a CDR1, a CDR2, and a CDR3 of a single variable domain having the amino acid sequence set forth in SEQ ID NO: 36, and the third antigen-binding moiety comprises a CDR1, a CDR2, and a CDR3 of a single variable domain having the amino acid sequence set forth in SEQ ID NO: 38.

In some embodiments, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is S or T. Further, in some embodiments, the first antigen-binding moiety comprises the amino acid sequence set forth in SEQ ID NO: 41, and the third antigen-binding moiety comprises the amino acid sequence set forth in SEQ ID NO: 40. In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 36, and the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35. In some specific embodiments, the first antigen-binding moiety comprises the amino acid sequence set forth in SEQ ID NO: 36, and the third antigen-binding moiety comprises the amino acid sequence set forth in SEQ ID NO: 35.

In some other embodiments, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24. Further, in some embodiments, the first antigen-binding moiety comprises the amino acid sequence set forth in SEQ ID NO: 41, and the third antigen-binding moiety comprises the amino acid sequence set forth in SEQ ID NO: 42. In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 36, and the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 38. In some specific embodiments, the first antigen-binding moiety comprises the amino acid sequence set forth in SEQ ID NO: 36, and the third antigen-binding moiety comprises the amino acid sequence set forth in SEQ ID NO: 38.

In some embodiments, the first antigen-binding moiety and the third antigen-binding moiety bind to different epitopes of c-Met.

The second antigen-binding moiety provides the ability to bind to EGFR. In some embodiments, the second antigen-binding moiety comprises a heavy chain variable region and a light chain variable region; the heavy chain variable region comprises: an HCDR1 comprising the amino acid sequence set forth in SEQ ID NO: 63, an HCDR2 comprising the amino acid sequence set forth in SEQ ID NO: 64, and an HCDR3 comprising the amino acid sequence set forth in SEQ ID NO: 65; the light chain variable region comprises an LCDR1 comprising the amino acid sequence set forth in SEQ ID NO: 66, an LCDR2 comprising the amino acid sequence set forth in SEQ ID NO: 67, and an LCDR3 comprising the amino acid sequence set forth in SEQ ID NO: 68. Further, in some embodiments, the second antigen-binding moiety comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 70. In some specific embodiments, the second antigen-binding moiety comprises a heavy chain variable region and a light chain variable region; the heavy chain variable region comprises the amino acid sequence set forth in SEQ ID NO: 69, and the light chain variable region comprises the amino acid sequence set forth in SEQ ID NO: 70.

In some embodiments, the multispecific antibody further comprises an Fc domain composed of two Fc polypeptides. In some specific embodiments, in the multispecific antibody, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; the third antigen-binding moiety comprises: a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6; and the second antigen-binding moiety comprises a heavy chain variable region and a light chain variable region, the heavy chain variable region comprises: an HCDR1 comprising the amino acid sequence set forth in SEQ ID NO: 63, an HCDR2 comprising the amino acid sequence set forth in SEQ ID NO: 64, and an HCDR3 comprising the amino acid sequence set forth in SEQ ID NO: 65, and the light chain variable region comprises an LCDR1 comprising the amino acid sequence set forth in SEQ ID NO: 66, an LCDR2 comprising the amino acid sequence set forth in SEQ ID NO: 67, and an LCDR3 comprising the amino acid sequence set forth in SEQ ID NO: 68, wherein $X_1$ is S or T. In further specific embodiments, the first antigen-binding moiety comprises the amino acid sequence set forth in SEQ ID NO: 41, the third antigen-binding moiety comprises the amino acid sequence set forth in SEQ ID NO: 40, the heavy chain variable region of the second antigen-binding moiety comprises the amino acid sequence set forth in SEQ ID NO: 69, and the light chain variable region thereof comprises the amino acid sequence set forth in SEQ ID NO: 70. In further specific embodiments, the first antigen-binding moiety comprises the amino acid sequence set forth in SEQ ID NO: 36, the third antigen-binding moiety comprises the amino acid sequence set forth in SEQ ID NO: 35, the heavy chain variable region of the second antigen-binding moiety comprises the amino acid sequence set forth in SEQ ID NO: 69, and the light chain variable region thereof comprises the amino acid sequence set forth in SEQ ID NO: 70.

In some other specific embodiments, in the multispecific antibody, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; the third antigen-binding moiety comprises: a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24; and the second antigen-binding moiety comprises a heavy chain variable region and a light chain variable region, the heavy chain variable region comprises: an HCDR1 comprising the amino acid sequence set forth in SEQ ID NO: 63, an HCDR2 comprising the amino acid sequence set forth in SEQ ID NO: 64, and an HCDR3 comprising the amino acid sequence set forth in SEQ ID NO: 65, and the light chain variable region comprises an LCDR1 comprising the amino acid sequence set forth in SEQ ID NO: 66, an LCDR2 comprising the amino acid sequence set forth in SEQ ID NO: 67, and an LCDR3 comprising the amino acid sequence set forth in SEQ ID NO: 68. In further specific embodiments, the first antigen-binding moiety comprises the amino acid sequence set forth in SEQ ID NO: 41, the third antigen-binding moiety comprises the amino acid sequence set forth in SEQ ID NO: 42, the heavy chain variable region of the second antigen-binding moiety comprises the amino acid sequence set forth in SEQ ID NO: 69, and the light chain variable region thereof comprises the amino acid sequence set forth in SEQ ID NO: 70. In further specific embodiments, the first antigen-binding moiety comprises the amino acid sequence set forth in SEQ ID NO: 36, the third antigen-binding moiety comprises the amino acid sequence set forth in SEQ ID NO: 38, the heavy chain variable region of the second antigen-binding moiety comprises the amino acid sequence set forth in SEQ ID NO: 69, and the light chain variable region thereof comprises the amino acid sequence set forth in SEQ ID NO: 70.

In the embodiments, in one optional configuration, the first antigen-binding moiety and the third antigen-binding moiety are both single variable domains, and the second antigen-binding moiety is a Fab; and the first antigen-binding moiety, at the C-terminus, is fused to the N-terminus of one of the Fc polypeptides of the Fc domain, the second antigen-binding moiety, at the C-terminus of the Fab heavy chain or the Fab light chain, is fused to the N-terminus of the other Fc polypeptide of the Fc domain, and the third antigen-binding moiety, at the C-terminus, is fused to the N-terminus of the first antigen-binding moiety. For the embodiment, in further more specific embodiments, the multispecific antibody consists of three polypeptide chains, wherein a first polypeptide chain comprises the first antigen-binding moiety, the third antigen-binding moiety, and one of the Fc polypeptides of the Fc domain described above, a second polypeptide chain comprises the Fab heavy chain of the second antigen-binding moiety and the other Fc polypeptide of the Fc domain described above, and a third polypeptide chain is the Fab light chain of the second antigen-binding moiety described above.

In some embodiments, the multispecific antibody is trivalent.

In one aspect, the present disclosure provides an isolated nucleic acid comprising a nucleotide sequence encoding the multispecific antibody described in the present disclosure.

In one aspect, the present disclosure provides a vector comprising the nucleic acid described in the present disclosure. In one aspect, the present disclosure provides a host cell comprising the nucleic acid or the vector described in the present disclosure.

In another aspect, the present disclosure provides a method for preparing the multispecific antibody described in the present disclosure, comprising culturing the host cell to express the multispecific antibody, and isolating and purifying the multispecific antibody from the system.

In another aspect, the present disclosure provides a pharmaceutical composition comprising the multispecific antibody and a pharmaceutically acceptable excipient.

In another aspect, the present disclosure provides use of the multispecific antibody or the pharmaceutical composition for preparing a medicament for treating a disease expressing c-Met or/and EGFR.

In another aspect, the present disclosure provides a method for treating a tumor expressing c-Met or/and EGFR, comprising: administering to a subject a therapeutically effective amount of the multispecific antibody or the pharmaceutical composition.

In yet another aspect, the present disclosure provides a c-Met-binding antibody, comprising a single variable domain, wherein the single variable domain comprises:

(1) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;
(2) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is S or T;

(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;

(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;

(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;

(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;

(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

In some embodiments, the single variable domain comprises the amino acid sequence set forth in SEQ ID NO: 40, 41, or 42, but does not comprise the amino acid sequence set forth in SEQ ID NO: 29, 30, or 31.

The multispecific antibody targeting EGFR and c-Met provided by the present disclosure exhibits good anti-tumor effects and/or safety.

## BRIEF DESCRIPTION OF THE DRAWINGS

FIGs. 1A-1G show binding curves of different anti-human c-Met $V_H$H-Fc chimeric antibodies to target cells with different c-Met expression levels as determined by flow cytometry;

FIG. 2 is a structural schematic of an exemplary multispecific antibody of the present disclosure;

FIGs. 3A-3C show binding curves of anti-EGFR/anti-c-Met multispecific antibodies to cells expressing EGFR and c-Met as determined by flow cytometry;

FIG. 4 shows the inhibition of c-Met phosphorylation and downstream signaling pathways by anti-EGFR/anti-c-Met multispecific antibodies as determined by Western Blotting, wherein the leftmost lane denotes the marker, the BM denotes amivantamab, the negative denotes hIgG1, the blank denotes no antibody, the HGF "-" or "+" denotes the absence or presence of HGF, and the p-cMet, p-Akt, p-Erk, and p-EGFR are phosphorylated c-Met, phosphorylated Akt, phosphorylated Erk, and phosphorylated EGFR, respectively;

FIG. 5 shows the inhibition of EGFR phosphorylation and downstream signaling pathways by anti-EGFR/anti-c-Met multispecific antibodies as determined by Western Blotting, wherein the leftmost lane denotes the marker, the BM denotes amivantamab, the negative denotes hIgG1, the blank denotes no antibody, the EGF "-" or "+" denotes the absence or presence of EGF, and the p-cMet, p-Akt, p-Erk, and p-EGFR are phosphorylated c-Met, phosphorylated Akt, phosphorylated Erk, and phosphorylated EGFR, respectively;

FIGs. 6A-6C show curves of anti-EGFR/anti-c-Met multispecific antibodies competing with HGF ligand at different concentrations for binding to c-Met as determined by ELISA;

FIGs. 7A and 7B show the inhibition of proliferation of tumor cells expressing EGFR and c-Met by anti-EGFR/antic-Met multispecific antibodies;

FIGs. 8A and 7B show the killing effects of anti-EGFR/anti-c-Met multispecific antibodies on tumor cells expressing EGFR and c-Met; and

FIGs. 9A-9C show the endocytic activity of anti-EGFR/anti-c-Met multispecific antibodies on cells expressing EGFR and c-Met as determined by flow cytometry.

## SUMMARY

### *Definitions and Description*

Unless otherwise stated, the following terms used in the present disclosure shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

The term "antibody" is used in its broadest sense and encompasses natural antibodies and artificial antibodies of various structures, including, but not limited to, various antibody structures such as monoclonal antibodies, polyclonal antibodies, multispecific antibodies (e.g., bispecific antibodies or trispecific antibodies), and single-chain antibodies, so long as they exhibit the desired antigen-binding activity.

The term "multispecific" means that the antibody is capable of specifically binding to a plurality of different antigenic determinants, e.g., capable of specifically binding to two or more different antigenic determinants. The antigenic determinant is synonymous with an antigen epitope herein. Generally, a bispecific antibody comprises two antigen-binding sites, each of which is specific for a different antigenic determinant. The different antigenic determinants may be expressed on the same or different cells. The different antigenic determinants may be different due to different antigens (e.g., binding to antigens EGFR and c-Met) or may be present on the same antigen. An antigenic determinant is a special chemical group with a certain composition and structure on the surface or other parts of the antigenic substance molecule, which can specifically bind to its corresponding antibody or sensitized lymphocyte. An example of the antigenic determinant is c-Met, which is an antigenic substance having various structurally defined or structurally undefined antigenic determinants. Antibodies which bind to two different antigenic determinants on an antigen are referred to herein as bispecific antibodies. One particular bispecific antibody can, for example, bind to EGFR and c-Met.

The term "N valent antibody" is intended to indicate the presence of N antigen-binding sites in the antibody. For example, "bivalent antibody" or "antibody is bivalent" refers to the presence of two antigen-binding sites in the antibody, and "trivalent antibody" or "antibody is trivalent" refers to the presence of three antigen-binding sites in the antibody. A natural human immunoglobulin molecule typically has two antigen-binding sites, and a Fab typically has a single antigen-binding site. A single variable domain and an scFv typically have a single antigen-binding site. The term "antigen-binding moiety" refers to a polypeptide molecule specifically binding to an antigenic determinant. A specific antigen-binding moiety may be a Fab, an scFv, or a single variable domain.

The term "first", "second", or "third" used herein with respect to an antigen-binding moiety, an antigen, an Fc polypeptide, a peptide linker, a polypeptide chain, and the like are used for ease of distinction when more than one part of each type is present. Unless specifically stated, the use of these terms is not intended to confer a particular order or orientation.

The term "fusion" refers to that components (e.g., an antigen-binding moiety, an Fc polypeptide, etc.) are linked either directly or by peptide bonds via one or more peptide linkers. For example, some peptide linkers consist of 1 to 50 amino acids linked by peptide bonds, wherein the amino acids may be selected from 20 naturally occurring amino acids; in a more preferred embodiment, the 1 to 50 amino acids are selected from glycine, alanine, proline, serine, asparagine, glutamine, and lysine.

The term "variable domain" or "variable region" refers to a domain of an antibody that is involved in the binding of the antibody to an antigen. For example, a natural four-chain antibody (e.g., one derived from a human, a murine, etc.) has a heavy chain variable region (also referred to as heavy chain variable domain, VH, or VH domain) and a light chain variable region (also referred to as light chain variable domain, VL, or VL domain), while a heavy-chain antibody derived from an animal such as a camelid or shark has a single variable domain. In most cases, each variable domain of a natural antibody consists substantially of four "framework regions (FRs)" and three "complementarity determining regions (CDRs)". The four framework regions are referred to as framework region 1 (or FR1), framework region 2 (or FR2), framework region 3 (or FR3), and framework region 4 (or FR4). The framework regions are separated by three complementarity determining regions referred to in the art and hereinafter as complementarity determining region 1 (or CDR1), complementarity determining region 2 (or CDR2), and complementarity determining region 3 (or CDR3). Thus, the general structure of a variable domain can be represented as follows: FR1-CDR1-FR2-CDR2-FR3-CDR3-FR4. Variable domains impart specificity for an antigen to an antibody by virtue of having an antigen-binding site.

The term "single variable domain" refers to a variable domain capable of specifically binding to an antigen epitope without being paired with other variable domains. The single variable domain typically has three CDRs (CDR1, CDR2, and CDR3) present on a single domain. In some cases, the single variable domain may be a heavy chain variable domain (e.g., VH), so long as it can form a single antigen-binding unit (i.e., a functional antigen-binding unit consisting essentially of a single variable domain; in this way, a single antigen-binding domain can form a functional antigen-binding unit without interacting with another variable domain). Another example of a single variable domain is "$V_H$H domain" of camelids (abbreviated as "$V_H$H" or "VHH").

The term "$V_H$H domain" is used to distinguish these variable domains from the heavy chain variable domains present in conventional four-chain antibodies and the light chain variable domains present in conventional four-chain antibodies. The $V_H$H domain specifically binds to epitopes without additional antigen-binding domains involved (different from the VH or VL domains of conventional four-chain antibodies where VL and VH domains jointly recognize epitopes). The $V_H$H domain is a small, stable, and efficient antigen recognition unit formed from a single domain.

The term "complementarity determining region" (CDR) is also referred to as "hypervariable region" (HVR). A natural four-chain antibody typically comprises six CDRs, among which three are in the heavy chain variable region, i.e., heavy chain CDR1 (HCDR1), heavy chain CDR2 (HCDR2), and heavy chain CDR3 (HCDR3), and three are in the light chain variable region, i.e., light chain CDR1 (LCDR1), light chain CDR2 (LCDR2), and light chain CDR3 (LCDR3). A heavy-chain antibody or a single variable domain typically has three CDRs (CDR1, CDR2, and CDR3).

There are currently many ways to define CDRs. The Kabat scheme defines CDRs based on the sequence variability and is the most commonly used (Elvin A. Kabat, et al., Sequences of Proteins of Immunological Interest, 5th Edition, Public Health Service, National Institutes of Health, Bethesda, Md. (1991)), while the Chothia scheme defines CDRs based on the positions of structural loops (Cyrus Chothia, et al., Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. The "Contact" defines CDRs based on the analysis of the crystal structure of available complexes. In addition, the CCG definition is also an option. However, it should be noted that boundaries of the CDRs of variable regions of the same antibody based on different methods and definitions may differ, that is, CDR sequences of the variable regions of the same antibody defined by different methods may differ. Thus, when reference is made to an antibody defined by specific CDR sequences, the scope of the antibody also encompasses antibodies defined by CDR sequences under any other definitions (e.g., one of or a combination of several of the definitions such as Kabat, IMGT, Chothia, Contact, AbM, CCG).

The term "Fab" refers to a protein consisting of VH and CH1 domains of the heavy chain and VL and CL domains of the light chain of an immunoglobulin. The Fab herein refers to a Fab molecule in a natural or modified form, i.e., comprising a Fab heavy chain composed of a heavy chain variable region VH and a constant region CH1 (VH-CH1, in a direction from N-terminus to C-terminus), and a Fab light chain composed of a light chain variable region and a constant region CL (VL-CL, in a direction from N-terminus to C-terminus). The modified Fab may be, for example, a Fab in which amino acid substitutions are introduced into the CH1/CL domain or/and the VH/VL domain. In one specific example, the modified Fab may be a Fab in which amino acid substitutions are introduced into the CL domain.

The term "scFv" comprises the VH and VL domains of an immunoglobulin, wherein these domains are present in a single polypeptide chain. In some embodiments, scFv further comprises a peptide linker between the VH and VL domains that enables the scFv to form the required structure for antigen-binding.

The term "Fc domain" or "Fc" is used herein to define the C-terminal region of an immunoglobulin heavy chain, which comprises at least part of the constant region. The term includes natural sequence Fc and variant Fc. The C-terminal lysine (Lys447) of the Fc may or may not be present. Unless otherwise stated, amino acid residues in the Fc or constant region are numbered according to the EU numbering system, also referred to as the EU index, as described in Kabat, E.A. et al., Sequences of Proteins of Immunological Interest, 5th Ed., Public Health Service, National Institutes of Health, Bethesda, MD (1991), NIH Publication 91-3242. As used herein, one of the "Fc polypeptides" of an Fc domain refers to one of the two polypeptides that form a dimeric Fc domain. For example, the Fc polypeptide of an IgG Fc domain comprises IgG CH2 and IgG CH3.

The term "treating" or "treatment" means administering the compound or pharmaceutical composition described herein to prevent, ameliorate, or eliminate a disease or one or more symptoms associated with the disease, including but not limited to: (i) preventing the occurrence of a disease or disease state in a mammal, particularly when such a mammal is predisposed to the disease state but has not yet been diagnosed with it; (ii) inhibiting a disease or disease state, i.e., arresting its progression; (iii) alleviating a disease or disease state, i.e., causing its regression; and (iv) reducing any direct or indirect pathological consequences of a disease or disease state.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying the onset of one or more symptoms of the specific disease, condition, or disorder described herein. The amount of the multispecific antibody, the antibody-drug conjugate, or the pharmaceutical composition of the present disclosure that constitutes a "therapeutically effective amount" may vary depending on factors such as the compound or the pharmaceutical composition and its ability to elicit a desired response in an individual, the disease state and its severity, the mode of administration, and the age, sex, and weight of the mammal to be treated. The therapeutically effective amount may also be determined routinely by those skilled in the art following their knowledge and the present disclosure. The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "excipient" refers to any ingredient other than the active ingredient (e.g., the antibody of the present disclosure). The selection of the excipient will largely depend on factors such as the particular mode of administration, the effect of the excipient on solubility and stability, and the nature of the dosage form.

The term "isolated" means that a target compound, such as an antibody or an antigen-binding fragment thereof, $V_HH$, or a nucleic acid, has been isolated from its natural environment.

The terms "Xn" and "Xaa" are equivalent and refer to an unspecified amino acid whose scope is specified by the subsequent definitions in the relevant description.

As used herein, the term "$EC_{50}$" refers to a concentration of an antibody that induces 50% of the maximal effective response. The $EC_{50}$ can be measured by ELISA or FACS analysis or any other method known in the art.

The "$K_D$" refers to the equilibrium dissociation constant, which is derived from the ratio of the dissociation rate constant ($k_d$) to the association rate constant ($k_a$) (i.e., $k_d/k_a$), and is expressed in molar concentration (M). The $K_D$ value of an antibody may be determined using methods well established in the art. A preferred method for determining the $K_D$ of an antibody is surface plasmon resonance (SPR), preferably using a biosensor system such as the Biacore surface plasmon resonance system for analysis.

The term "identity" is also referred to as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. The alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to acquire the maximum alignment for the full length of sequences being compared.

The term "subject" includes any human or non-human animal. The term "non-human animal" includes all vertebrates, e.g., mammals and non-mammals, such as non-human primates, sheep, dogs, cats, horses, cows, chickens, amphibians, and reptiles. Preferably, the subject according to the present disclosure is a human. The terms "patient" and "subject" are used interchangeably unless otherwise indicated. The "subjects in need" include those with a disease or condition, those at risk of developing a disease or condition, and those who may have a disease or condition and whose purpose is to prevent, delay, or alleviate the disease or condition.

As used herein, "about" means being within an acceptable error range determined by those of ordinary skill in the art for a specific value, which will depend in part on how the value is measured or determined, i.e., the limitation by the measurement system. For example, "about" may mean being within 1 or more than 1 standard deviation, as practiced in the art. Alternatively, "about" may mean a range of up to $\pm5\%$, for example, fluctuating within a specific numerical range given $\pm2\%$, $\pm1\%$, or $\pm0.5\%$. Where a specific value is given in the scope of the present disclosure, unless otherwise stated, "about" should be considered to mean being within an acceptable error range for that specific value. As used herein, unless otherwise stated, the values of the parameters or conditions in a step are all modified by "about" by default.

The terms "comprise", "comprises", "comprising", and equivalents thereof (e.g., contain, contains, containing, include, includes, and including) are to be construed as "including, but not limited to", meaning that additional unspecified elements, components, and steps may be included in addition to the enumerated elements, components, and steps.

As used herein, unless otherwise specified clearly in the context, singular terms encompass plural referents, and vice versa.

_Single Variable Domain_

The present disclosure provides a single variable domain that binds to c-Met (such as human c-Met). The single variable domain provides more available options for the development or drug construction of c-Met-targeting drugs. The single variable domain has good affinity for human c-Met, and can provide targeting property. In some certain cases, the single variable domain is capable of providing the ability to block the binding of the ligand HGF to c-Met. In particular, in some embodiments, the single variable domain has cross-reactions with monkey c-Met. Since monkeys are the ideal experimental animals for drug toxicology experiments, cross-reactions with monkeys will facilitate drug toxicology experiments.

The present disclosure provides a single variable domain that binds to c-Met, comprising:

(1) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;

(2) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is S or T;

(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;

(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;

(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;

(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;

(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

In some embodiments, the single variable domain comprises: a CDR1 having the amino acid sequence set forth in SEQ ID NO: 1, a CDR2 having the amino acid sequence set forth in SEQ ID NO: 2, and a CDR3 having the amino acid sequence set forth in SEQ ID NO: 3. In some embodiments, the single variable domain comprises: a CDR1 having the amino acid sequence set forth in SEQ ID NO: 4, a CDR2 having the amino acid sequence set forth in SEQ ID NO: 5, and a CDR3 having the amino acid sequence set forth in SEQ ID NO: 6, wherein $X_1$ is S or T. In some embodiments, the single variable domain comprises: a CDR1 having the amino acid sequence set forth in SEQ ID NO: 4, a CDR2 having the amino acid sequence set forth in SEQ ID NO: 5, and a CDR3 having the amino acid sequence set forth in SEQ ID NO: 6, wherein $X_1$ is S. In some embodiments, the single variable domain comprises: a CDR1 having the amino acid sequence set forth in SEQ ID NO: 4, a CDR2 having the amino acid sequence set forth in SEQ ID NO: 5, and a CDR3 having the amino acid sequence set forth in SEQ ID NO: 6, wherein $X_1$ is T. In some embodiments, the single variable domain comprises: a CDR1 having the amino acid sequence set forth in SEQ ID NO: 7, a CDR2 having the amino acid sequence set forth in SEQ ID NO: 8, and a CDR3 having the amino acid sequence set forth in SEQ ID NO: 9. In some embodiments, the single variable domain comprises: a CDR1 having the amino acid sequence set forth in SEQ ID NO: 10, a CDR2 having the amino acid sequence set forth in SEQ ID NO: 11, and a CDR3 having the amino acid sequence set forth in SEQ ID NO: 12. In some embodiments, the single variable domain comprises: a CDR1 having the amino acid sequence set forth in SEQ ID NO: 13, a CDR2 having the amino acid sequence set forth in SEQ ID NO: 14, and a CDR3 having the amino acid sequence set forth in SEQ ID NO: 15. In some embodiments, the single variable domain comprises: a CDR1 having the amino acid sequence set forth in SEQ ID NO: 16, a CDR2 having the amino acid sequence set forth in SEQ ID NO: 17, and a CDR3 having the amino acid sequence set forth in SEQ ID NO: 18. In some embodiments, the single variable domain is derived from a camelid. In some embodiments, the single variable domain is humanized. In some embodiments, the single variable domain comprises: a CDR1 having the amino acid sequence set forth in SEQ ID NO: 19, a CDR2 having the amino acid sequence set forth in SEQ ID NO: 20, and a CDR3 having the amino acid sequence set forth in SEQ ID NO: 21. In some embodiments, the single variable domain comprises: a CDR1 having the amino acid sequence set forth in SEQ ID NO: 22, a CDR2 having the amino acid sequence set forth in SEQ ID NO: 23, and a CDR3 having the amino acid sequence set forth in SEQ ID NO: 24.

In some embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, 39, 40, 41, or 42.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, and the single variable domain comprises: a CDR1 comprising the amino acid sequence set forth in SEQ ID NO: 1, CDR2 comprising the amino acid sequence set forth in SEQ ID NO: 2, and CDR3 comprising the amino acid sequence set forth in SEQ ID NO: 3.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40, and the single variable domain comprises: a CDR1 comprising the amino acid sequence set forth in SEQ ID NO: 4, CDR2 comprising the amino acid sequence set forth in SEQ ID NO: 5, and CDR3 comprising the amino acid sequence set forth in SEQ ID NO: 6, wherein, $X_1$ is S or T.

In some more specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 26, 33, or 34, and the single variable domain comprises: a CDR1 comprising the amino acid sequence set forth in SEQ ID NO: 4, CDR2 comprising the amino acid sequence set forth in SEQ ID NO: 5, and CDR3 comprising the amino acid sequence set forth in SEQ ID NO: 6, wherein, $X_1$ is S.

In some more specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35, and the single variable domain comprises: a CDR1 comprising the amino acid sequence set forth in SEQ ID NO: 4, CDR2 comprising the amino acid sequence set forth in SEQ ID NO: 5, and CDR3 comprising the amino acid sequence set forth in SEQ ID NO: 6, wherein, $X_1$ is T.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41, and the single variable domain comprises: a CDR1 comprising the amino acid sequence set forth in SEQ ID NO: 7, CDR2 comprising the amino acid sequence set forth in SEQ ID NO: 8, and CDR3 comprising the amino acid sequence set forth in SEQ ID NO: 9.

In some more specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 27, 36, or 37, and the single variable domain comprises: a CDR1 comprising the amino acid sequence set forth in SEQ ID NO: 7, CDR2 comprising the amino acid sequence set forth in SEQ ID NO: 8, and CDR3 comprising the amino acid sequence set forth in SEQ ID NO: 9.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 28, and the single variable domain comprises: a CDR1 comprising the amino acid sequence set forth in SEQ ID NO: 10, CDR2 comprising the amino acid sequence set forth in SEQ ID NO: 11, and CDR3 comprising the amino acid sequence set forth in SEQ ID NO: 12.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 29, and the single variable domain comprises: a CDR1 comprising the amino acid sequence set forth in SEQ ID NO: 13, CDR2 comprising the amino acid sequence set forth in SEQ ID NO: 14, and CDR3 comprising the amino acid sequence set forth in SEQ ID NO: 15.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 30, and the single variable domain comprises: a CDR1 comprising the amino acid sequence set forth in SEQ ID NO: 16, CDR2 comprising the amino acid sequence set forth in SEQ ID NO: 17, and CDR3 comprising the amino acid sequence set forth in SEQ ID NO: 18.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and the single variable domain comprises: a CDR1 comprising the amino acid sequence set forth in SEQ ID NO: 19, CDR2 comprising the amino acid sequence set forth in SEQ ID NO: 20, and CDR3 comprising the amino acid sequence set forth in SEQ ID NO: 21.

In some specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 42, and the single variable domain comprises: a CDR1 comprising the amino acid sequence set forth in SEQ ID NO: 22, CDR2 comprising the amino acid sequence set forth in SEQ ID NO: 23, and CDR3 comprising the amino acid sequence set forth in SEQ ID NO: 24.

In some more specific embodiments, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32, 38, or 39, and the single variable domain comprises: a CDR1 comprising the amino acid sequence set forth in SEQ ID NO: 22, CDR2 comprising the amino acid sequence set forth in SEQ ID NO: 23, and CDR3 comprising the amino acid sequence set forth in SEQ ID NO: 24.

In some embodiments, an amino acid sequence having at least 85%, 86%, 87%, 88%, 89%, 90%, 91%, 92%, 93%, 94%, 95%, 96%, 97%, 98%, or 99% identity comprises substitutions (e.g., conservative substitutions), insertions or deletions compared to a reference sequence; however, single variable domains comprising the sequence retain the ability to bind to c-Met. In some embodiments, a total of 1-18, 1-16, 1-14, 1-13, 1-12, 1-11, 1-10, 1-9, 1-8, 1-7, 1-6, 1-5, 1-4, 1-3, or 1-2 amino acids are substituted, inserted, and/or deleted in an amino acid sequence selected from SEQ ID NOs: 25, 26, 27, 28, 29, 30, 31, 32, 33, 34, 35, 36, 37, 38, 39, 40, 41, and 42. In some embodiments, the substitutions, insertions, or deletions occur in regions outside of CDRs (i.e., in FRs). In some embodiments, the substitutions, insertions, or deletions occur in CDR regions, for example, in one, two, or three of CDR1, CDR2, and CDR3. In some embodiments, the substitutions, insertions, or deletions occur in CDR regions and non-CDR regions. In some embodiments, the amino acid sequence of the single variable domain comprises the amino acid sequence set forth in SEQ ID NO: 25, 26, 27, 28, 29, 30, 31, 32, 33, 34, 35, 36, 37, 38, 39, 40, 41, or 42.

In some embodiments, the amino acid sequence of the single variable domain is set forth in SEQ ID NO: 25, 26, 27, 28, 29, 30, 31, 32, 33, 34, 35, 36, 37, 38, 39, 40, 41, or 42.

In some embodiments, the single variable domain is a V$_H$H. In some embodiments, the V$_H$H is humanized. A single variable domain of non-human origin may be "humanized" by replacing one or more amino acid residues in the amino acid sequence of the original single variable domain sequence with one or more amino acid residues present at corresponding positions in the VH domain of a human antibody. Humanization may be desirable to reduce immunogenicity. Generally, the single variable domain has the following structure from N-terminus to C-terminus: FR1-CDR1-FR2-CDR2-FR3-CDR3-FR4.

In some embodiments, the present disclosure provides a single variable domain that binds to the same epitope as any one of the single variable domains described in the present disclosure. In some specific embodiments, the present disclosure provides a single variable domain that binds to the same epitopes as a single variable domain comprising the amino acid sequence of SEQ ID NO: 25, 26, 27, 28, 29, 30, 31, 32, 33, 34, 35, 36, 37, 38, 39, 40, 41, or 42. In some embodiments, the single variable domain that binds to the same epitopes is derived from a camelid or is humanized.

Based on the binding to the same epitopes, competitive screening of single variable domains can be carried out using conventional techniques known to those skilled in the art. Thus, in some embodiments, the present disclosure provides a single variable domain that competes for binding to c-Met with any one of the single variable domains described in the present disclosure. In some specific embodiments, provided is a single variable domain that competes for binding to c-Met with a single variable domain comprising an amino acid sequence of SEQ ID NO: 25, 26, 27, 28, 29, 30, 31, 32, 33, 34, 35, 36, 37, 38, 39, 40, 41, or 42. The binding to c-Met can be measured by ELISA, flow cytometry, or surface plasmon resonance (SPR) assay, or any other method known in the art. In some embodiments, the single variable domain that competes for binding to c-Met is derived from a camelid or is humanized.

The present disclosure provides some exemplary single variable domains that bind to c-Met. The CDR (CDR1, CDR2 and CDR3) and full-length amino acid sequences of the exemplary single variable domains provided herein are provided in Table S1 below.

Table S1. CDR and full-length amino acid sequences of single variable domains (SEQ ID NO.)

| Name | CDR1 | CDR2 | CDR3 | Full length |
|---|---|---|---|---|
| 1B-3B11 single variable domain | 1 | 2 | 3 | 25 |
| 1B-1B6 single variable domain | | | | 26 |
| 1B-1B6-V1 single variable domain | 4 | 5, $X_1$ is S | 6 | 33 |
| 1B-1B6-V2 single variable domain | | | | 34 |
| 1B-1B6-V3 single variable domain | 4 | 5, $X_1$ is T | 6 | 35 |
| 1B-1C7 single variable domain | | | | 27 |
| 1B-1C7-V1 single variable domain | 7 | 8 | 9 | 36 |
| 1B-1C7-V2 single variable domain | | | | 37 |
| 1B-1A8 single variable domain | 10 | 11 | 12 | 28 |
| 1B-1B2 single variable domain | 13 | 14 | 15 | 29 |
| 3B-1C7 single variable domain | 16 | 17 | 18 | 30 |
| 4&5B-2F01 single variable domain | 19 | 20 | 21 | 31 |
| 4&5C-12B04 single variable domain | | | | 32 |
| 12B04-V1 single variable domain | 22 | 23 | 24 | 38 |
| 12B04-V2 single variable domain | | | | 39 |

The present disclosure provides a c-Met-binding antibody comprising the single variable domain.

In some embodiments, the c-Met-binding antibody may be a monospecific antibody or a multispecific antibody. In one embodiment, the monospecific antibody comprises an Fc domain; preferably, the Fc is that of human IgG1, IgG2, IgG3, or IgG4.

In some embodiments, in the above c-Met-binding antibody, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, 39, 40, 41, or 42.

In some embodiments, the c-Met-binding antibody comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 38, 39, 43, 44, 45, 46, 47, 48, 49, 50, 51, 53, 55, 57, or 59.

The present disclosure provides exemplary monospecific antibodies (e.g., 1B-1B6-V1, 1B-1B6-V2, 1B-1B6-V3, 1B-1C7-V1, 1B-1C7-V2, 12B04-V1, 12B04-V2, etc.), in which the single variable domain is fused with the Fc of a human IgG1, thus forming a homodimer through Fc.

The present disclosure provides an isolated nucleic acid comprising a polynucleotide encoding the c-Met-binding antibody described in the present disclosure.

The present disclosure provides a vector comprising the nucleic acid described in the present disclosure.

The present disclosure provides a host cell comprising the nucleic acid or the vector described in the present disclosure.

The present disclosure provides a method for preparing the c-Met-binding antibody described in the present disclosure, comprising culturing the host cell described in the present disclosure to express the c-Met-binding antibody, and isolating and purifying the c-Met-binding antibody from the system.

The present disclosure provides a pharmaceutical composition comprising the c-Met-binding antibody described in the present disclosure and a pharmaceutically acceptable excipient.

The present disclosure provides a method for treating a disease related to c-Met expression, comprising administering to a subject in need the c-Met-binding antibody or the pharmaceutical composition described in the present disclosure.

The present disclosure provides a method for treating a disease related to c-Met expression, comprising administering to a subject in need a therapeutically effective amount of the c-Met-binding antibody or the pharmaceutical composition described in the present disclosure.

*Multispecific Antibody*

The present disclosure provides a multispecific antibody comprising a single variable domain that can target c-Met and EGFR expressed on the surface of a tumor cell and possesses tumor killing activity.

With the single variable domain, the mismatches between the light and heavy chains can be reduced or avoided compared to forms with a number of (e.g., two) Fab.

The multispecific antibody of the present disclosure exhibits excellent anti-tumor properties such as lysing tumor cells or inhibiting the proliferation of tumor cells. In some embodiments, the multispecific antibody of the present disclosure is capable of inducing the killing of various tumor cells through ADCC and/or CDC effects. The multispecific antibody of the present disclosure exhibits good tumor killing activity against tumor cells with different c-Met expression levels and/or different EGFR expression levels.

The multispecific antibody of the present disclosure can inhibit the downstream signaling of c-Met or/and EGFR. The multispecific antibody of the present disclosure exhibits excellent antigen endocytotic or/and antigen degradation effects. In some embodiments, the multispecific antibody of the present disclosure, after binding to c-Met and/or EGFR, mediates the endocytosis of c-Met and/or EGFR and reduces the expression of c-Met and/or EGFR on the surface of tumor cells.

The multispecific antibody may be constructed using any one of the single variable domains described in "single variable domain". Thus, the present disclosure provides a multispecific antibody, comprising:

(i) a first antigen-binding moiety that binds to a first antigen;
(ii) a second antigen-binding moiety that binds to a second antigen; and
(iii) a third antigen-binding moiety that binds to the first antigen,

wherein the first antigen is c-Met, and the second antigen is EGFR; and the first antigen-binding moiety and the third antigen-binding moiety are both single variable domains and each independently comprise any one of the following:

(1) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;
(2) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is S or T;
(3) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;
(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;
(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;
(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;
(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or
(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

In the present disclosure, the first antigen-binding moiety and the third antigen-binding moiety are both single variable domains that bind to c-Met. In some embodiments, the first antigen-binding moiety comprises the complementarity determining regions described in any one of (1)-(8) above. In some embodiments, the third antigen-binding moiety comprises the complementarity determining regions described in any one of (1)-(8) above. In the multispecific antibody, the first antigen-binding moiety and the third antigen-binding moiety may be identical or different, and any single variable domain described in the present disclosure may be independently selected for combination. In some specific embodiments, the first antigen-binding moiety and the third antigen-binding moiety are identical. In some other specific embodiments, the first antigen-binding moiety and the third antigen-binding moiety are different.

Multispecific antibodies constructed with different combinations of the first antigen-binding moiety and the third antigen-binding moiety with defined CDR features are illustratively listed in Table S2. In some embodiments, the first antigen-binding moiety comprises the complementarity determining regions described in (3) above, and the third antigen-binding moiety comprises the complementarity determining regions described in (2) above, that is, the first antigen-binding moiety comprises a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9, and the third antigen-binding moiety comprises a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is S or T. In some specific embodiments, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is T. In a more specific embodiment, the first antigen-binding moiety comprises the CDR1 set forth in SEQ ID NO: 7, the CDR2 set forth in SEQ ID NO: 8, and the CDR3 set forth in SEQ ID NO: 9; and the third antigen-binding moiety comprises the CDR1 set forth in SEQ ID NO: 4, the CDR2 set forth in SEQ ID NO: 5, and the CDR3 set forth in SEQ ID NO: 6, wherein $X_1$ is T. In some other embodiments, the first antigen-binding moiety comprises the complementarity determining regions described in (3) above, and the third antigen-binding moiety comprises the complementarity determining regions described in (8) above, that is, the first antigen-binding moiety comprises a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9, and the third antigen-binding moiety comprises a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24. In a more specific embodiment, the first antigen-binding moiety comprises the CDR1 set forth in SEQ ID NO: 7, the CDR2 set forth in SEQ ID NO: 8, and the CDR3 set forth in SEQ ID NO: 9; and the third antigen-binding moiety comprises the CDR1 set forth in SEQ ID NO: 22, the CDR2 set forth in SEQ ID NO: 23, and the CDR3 set forth in SEQ ID NO: 24.

Table S2. Exemplary multispecific antibodies with defined CDR features in the first antigen-binding moiety and the third antigen-binding moiety

| Third antigen-binding moiety \ First antigen-binding moiety | (1) | (2) | (3) | (4) | (5) | (6) | (7) | (8) |
|---|---|---|---|---|---|---|---|---|
| (1) | (1)+(1) | (1)+(2) | (1)+(3) | (1)+(4) | (1)+(5) | (1)+(6) | (1)+(7) | (1)+(8) |
| (2) | (2)+(1) | (2)+(2) | (2)+(3) | (2)+(4) | (2)+(5) | (2)+(6) | (2)+(7) | (2)+(8) |
| (3) | (3)+(1) | (3)+(2) | (3)+(3) | (3)+(4) | (3)+(5) | (3)+(6) | (3)+(7) | (3)+(8) |
| (4) | (4)+(1) | (4)+(2) | (4)+(3) | (4)+(4) | (4)+(5) | (4)+(6) | (4)+(7) | (4)+(8) |
| (5) | (5)+(1) | (5)+(2) | (5)+(3) | (5)+(4) | (5)+(5) | (5)+(6) | (5)+(7) | (5)+(8) |
| (6) | (6)+(1) | (6)+(2) | (6)+(3) | (6)+(4) | (6)+(5) | (6)+(6) | (6)+(7) | (6)+(8) |
| (7) | (7)+(1) | (7)+(2) | (7)+(3) | (7)+(4) | (7)+(5) | (7)+(6) | (7)+(7) | (7)+(8) |
| (8) | (8)+(1) | (8)+(2) | (8)+(3) | (8)+(4) | (8)+(5) | (8)+(6) | (8)+(7) | (8)+(8) |

Note: in the table, (X) + (Y) indicates the combination of the third antigen-binding moiety and the first antigen-binding moiety of the multispecific antibody. For example, (2) + (1) indicates that the third antigen-binding moiety of the multispecific antibody comprises the complementarity determining regions described in (2), while the first antigen-binding moiety comprises the complementarity determining regions described in (1).

Further, in some embodiments, the first antigen-binding moiety and the third antigen-binding moiety each independently comprise an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, 39, 40, 41, or 42. In some embodiments, the first antigen-binding moiety and the third antigen-binding moiety each independently comprise an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, or 39.

In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, 39, 40, 41, or 42. In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, or 39. In some specific embodiments, the first antigen-binding moiety comprises the amino acid sequence set forth in SEQ ID NO: 25, 26, 27, 28, 29, 30, 31, 32, 33, 34, 35, 36, 37, 38, 39, 40, 41, or 42. In some more specific embodiments, the first antigen-binding moiety comprises the amino acid sequence set forth in SEQ ID NO: 25, 26, 27, 28, 29, 30, 31, 32, 33, 34, 35, 36, 37, 38, or 39.

In some embodiments, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, 39, 40, 41, or 42. In some embodiments, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 25, 26, 27, 28, 29, 30, 31, 32, 33, 34, 35, 36, 37, 38, or 39. In some specific embodiments, the third antigen-binding moiety comprises the amino acid sequence set forth in SEQ ID NO: 25, 26, 27, 28, 29, 30, 31, 32, 33, 34, 35, 36, 37, 38, 39, 40, 41, or 42. In some more specific embodiments, the third antigen-binding moiety comprises the amino acid sequence set forth in SEQ ID NO: 25, 26, 27, 28, 29, 30, 31, 32, 33, 34, 35, 36, 37, 38, or 39.

In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41, and the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40. In some specific embodiments, the first antigen-binding moiety comprises the amino acid sequence set forth in SEQ ID NO: 41, and the third antigen-binding moiety comprises the amino acid sequence set forth in SEQ ID NO: 40. In some more specific embodiments, the first antigen-binding moiety comprises the amino acid sequence set forth in SEQ ID NO: 27, 36, or 37, and the third antigen-binding moiety comprises the amino acid sequence set forth in SEQ ID NO: 26, 33, 34, or 35.

In some embodiments, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41, and the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 42. In some specific embodiments, the first antigen-binding moiety comprises the amino acid sequence set forth in SEQ ID NO: 41, and the third antigen-binding moiety comprises the amino acid sequence set forth in SEQ ID NO: 42. In some more specific embodiments, the first antigen-binding moiety comprises the amino acid sequence set forth in SEQ ID NO: 27, 36, or 37, and the third antigen-binding moiety comprises the amino acid sequence set forth in SEQ ID NO: 32, 38, or 39.

In one example, the first antigen-binding moiety comprises the amino acid sequence set forth in SEQ ID NO: 27, and the third antigen-binding moiety comprises the amino acid sequence set forth in SEQ ID NO: 26, 32, 33, 34, 35, 38, or 39.

In one example, the third antigen-binding moiety comprises the amino acid sequence set forth in SEQ ID NO: 27, and the first antigen-binding moiety comprises the amino acid sequence set forth in SEQ ID NO: 26, 32, 33, 34, 35, 38, or 39.

In one example, the first antigen-binding moiety comprises the amino acid sequence set forth in SEQ ID NO: 36, and the third antigen-binding moiety comprises the amino acid sequence set forth in SEQ ID NO: 26, 32, 33, 34, 35, 38, or 39.

In one example, the third antigen-binding moiety comprises the amino acid sequence set forth in SEQ ID NO: 36, and the first antigen-binding moiety comprises the amino acid sequence set forth in SEQ ID NO: 26, 32, 33, 34, 35, 38, or 39.

In one example, the first antigen-binding moiety comprises the amino acid sequence set forth in SEQ ID NO: 37, and the third antigen-binding moiety comprises the amino acid sequence set forth in SEQ ID NO: 26, 32, 33, 34, 35, 38, or 39.

In one example, the third antigen-binding moiety comprises the amino acid sequence set forth in SEQ ID NO: 37, and the first antigen-binding moiety comprises the amino acid sequence set forth in SEQ ID NO: 26, 32, 33, 34, 35, 38, or 39.

In one example, the first antigen-binding moiety comprises the amino acid sequence set forth in SEQ ID NO: 27, and the third antigen-binding moiety comprises the amino acid sequence set forth in SEQ ID NO: 26.

In one example, the third antigen-binding moiety comprises the amino acid sequence set forth in SEQ ID NO: 27, and the first antigen-binding moiety comprises the amino acid sequence set forth in SEQ ID NO: 26.

In one example, the first antigen-binding moiety comprises the amino acid sequence set forth in SEQ ID NO: 36, and the third antigen-binding moiety comprises the amino acid sequence set forth in SEQ ID NO: 33.

In one example, the third antigen-binding moiety comprises the amino acid sequence set forth in SEQ ID NO: 36, and the first antigen-binding moiety comprises the amino acid sequence set forth in SEQ ID NO: 33.

In one example, the first antigen-binding moiety comprises the amino acid sequence set forth in SEQ ID NO: 36, and the third antigen-binding moiety comprises the amino acid sequence set forth in SEQ ID NO: 34.

In one example, the third antigen-binding moiety comprises the amino acid sequence set forth in SEQ ID NO: 36, and the first antigen-binding moiety comprises the amino acid sequence set forth in SEQ ID NO: 34.

In one example, the first antigen-binding moiety comprises the amino acid sequence set forth in SEQ ID NO: 36, and the third antigen-binding moiety comprises the amino acid sequence set forth in SEQ ID NO: 35.

In one example, the third antigen-binding moiety comprises the amino acid sequence set forth in SEQ ID NO: 36, and the first antigen-binding moiety comprises the amino acid sequence set forth in SEQ ID NO: 35.

In one example, the first antigen-binding moiety comprises the amino acid sequence set forth in SEQ ID NO: 36, and the third antigen-binding moiety comprises the amino acid sequence set forth in SEQ ID NO: 32.

In one example, the third antigen-binding moiety comprises the amino acid sequence set forth in SEQ ID NO: 36, and the first antigen-binding moiety comprises the amino acid sequence set forth in SEQ ID NO: 32.

In one example, the first antigen-binding moiety comprises the amino acid sequence set forth in SEQ ID NO: 36, and the third antigen-binding moiety comprises the amino acid sequence set forth in SEQ ID NO: 38.

In one example, the third antigen-binding moiety comprises the amino acid sequence set forth in SEQ ID NO: 36, and the first antigen-binding moiety comprises the amino acid sequence set forth in SEQ ID NO: 38.

In one example, the first antigen-binding moiety comprises the amino acid sequence set forth in SEQ ID NO: 36, and the third antigen-binding moiety comprises the amino acid sequence set forth in SEQ ID NO: 39.

In one example, the third antigen-binding moiety comprises the amino acid sequence set forth in SEQ ID NO: 36, and the first antigen-binding moiety comprises the amino acid sequence set forth in SEQ ID NO: 39.

In one example, the first antigen-binding moiety and the third antigen-binding moiety both comprise the amino acid sequence set forth in SEQ ID NO: 25, 26, 27, 28, 29, 30, 31, 32, 33, 34, 35, 36, 37, 38, or 39.

Multispecific antibodies constructed with different combinations of the first antigen-binding moiety and the third antigen-binding moiety with full-length amino acid sequence features are illustratively listed in Table S3.

Table S3. Exemplary multispecific antibodies with defined amino acid sequences in the first antigen-binding moiety and the third antigen-binding moiety

| First antigen-binding moiety / Third antigen-binding moiety | 1B-1B6-V1 single variable domain | 1B-1B6-V2 single variable domain | 1B-1B6-V3 single variable domain | 1B-1C7-V1 single variable domain | 1B-1C7-V2 single variable domain | 12B04-V1 single variable domain | 12B04-V2 single variable domain |
|---|---|---|---|---|---|---|---|
| 1B-1B6-V1 single variable domain | 1B-1B6-V1 + 1B-1B6-V1 single variable domains | 1B-1B6-V1 + 1B-1B6-V2 single variable domains | 1B-1B6-V1 + 1B-1B6-V3 single variable domains | 1B-1B6-V1 + 1B-1C7-V1 single variable domains | 1B-1B6-V1 + 1B-1C7-V2 single variable domains | 1B-1B6-V1 + 12B04-V1 single variable domains | 1B-1B6-V1 + 12B04-V2 single variable domains |
| 1B-1B6-V2 single variable domain | 1B-1B6-V2 + 1B-1B6-V1 single variable domains | 1B-1B6-V2 + 1B-1B6-V2 single variable domains | 1B-1B6-V2 + 1B-1B6-V3 single variable domains | 1B-1B6-V2 + 1B-1C7-V1 single variable domains | 1B-1B6-V2 + 1B-1C7-V2 single variable domains | 1B-1B6-V2 + 12B04-V1 single variable domains | 1B-1B6-V2 + 12B04-V2 single variable domains |
| 1B-1B6-V3 single variable domain | 1B-1B6-V3 + 1B-1B6-V1 single variable domains | 1B-1B6-V3 + 1B-1B6-V2 single variable domains | 1B-1B6-V3 + 1B-1B6-V3 single variable domains | 1B-1B6-V3 + 1B-1C7-V1 single variable domains | 1B-1B6-V3 + 1B-1C7-V2 single variable domains | 1B-1B6-V3 + 12B04-V1 single variable domains | 1B-1B6-V3 + 12B04-V2 single variable domains |
| 1B-1C7-V1 single variable domain | 1B-1C7-V1 + 1B-1B6-V1 single variable domains | 1B-1C7-V1 + 1B-1B6-V2 single variable domains | 1B-1C7-V1 + 1B-1B6-V3 single variable domains | 1B-1C7-V1 + 1B-1C7-V1 single variable domains | 1B-1C7-V1 + 1B-1C7-V2 single variable domains | 1B-1C7-V1 + 12B04-V1 single variable domains | 1B-1C7-V1 + 12B04-V2 single variable domains |
| 1B-1C7-V2 single variable domain | 1B-1C7-V2 + 1B-1B6-V1 single variable domains | 1B-1C7-V2 + 1B-1B6-V2 single variable domains | 1B-1C7-V2 + 1B-1B6-V3 single variable domains | 1B-1C7-V2 + 1B-1C7-V1 single variable domains | 1B-1C7-V2 + 1B-1C7-V2 single variable domains | 1B-1C7-V2 + 12B04-V1 single variable domains | 1B-1C7-V2 + 12B04-V2 single variable domains |
| 12B04-V1 single variable domain | 12B04-V1 + 1B-1B6-V1 single variable domains | 12B04-V1 + 1B-1B6-V2 single variable domains | 12B04-V1 + 1B-1B6-V3 single variable domains | 12B04-V1 + 1B-1C7-V1 single variable domains | 12B04-V1 + 1B-1C7-V2 single variable domains | 12B04-V1 + 12B04-V1 single variable domains | 12B04-V1 + 12B04-V2 single variable domains |
| 12B04-V2 single variable domain | 12B04-V2 + 1B-1B6-V1 single variable domains | 12B04-V2 + 1B-1B6-V2 single variable domains | 12B04-V2 + 1B-1B6-V3 single variable domains | 12B04-V2 + 1B-1C7-V1 single variable domains | 12B04-V2 + 1B-1C7-V2 single variable domains | 12B04-V2 + 12B04-V1 single variable domains | 12B04-V2 + 12B04-V2 single variable domains |

Note: in the table, (X) + (Y) indicates the combination of the third antigen-binding moiety and the first antigen-binding moiety of the multispecific antibody. For example, "1B-1B6-V1 + 1B-1C7-V1 single variable domains" indicates that the third antigen-binding moiety of the multispecific antibody comprises the amino acid sequence of the 1B-1B6-V1 single variable domain, while the first antigen-binding moiety comprises the amino acid sequence of the 1B-1C7-V1 single variable domain; the amino acid sequences of the single variable domains referred to in the table are shown in Table S1.

In the present disclosure, the first antigen-binding moiety may be derived from a camelid or be humanized. The third antigen-binding moiety may be derived from a camelid or be humanized. Humanization may reduce the immunogenicity, and in some embodiments, both the first antigen-binding moiety and the third antigen-binding moiety are humanized. In some embodiments, both the first antigen-binding moiety and the third antigen-binding moiety are derived from a camelid.

In the multispecific antibody of the present disclosure, the second antigen-binding moiety provides the ability to target EGFR. The second antigen-binding moiety may be a Fab, an scFv, or an ScFab (single-chain Fab). In some embodiments, the second antigen-binding moiety is a Fab that binds to EGFR.

In some embodiments, the second antigen-binding moiety is murine, chimeric, or humanized.

In some embodiments, the second antigen-binding moiety comprises a heavy chain variable region and a light chain variable region, wherein the heavy chain variable region comprises: an HCDR1 comprising the amino acid sequence set forth in SEQ ID NO: 63, an HCDR2 comprising the amino acid sequence set forth in SEQ ID NO: 64, and an HCDR3 comprising the amino acid sequence set forth in SEQ ID NO: 65, and the light chain variable region comprises: an LCDR1 comprising the amino acid sequence set forth in SEQ ID NO: 66, an LCDR2 comprising the amino acid sequence set forth in SEQ ID NO: 67, and an LCDR3 comprising the amino acid sequence set forth in SEQ ID NO: 68.

In one specific embodiment, the heavy chain variable region comprises the HCDR1, the HCDR2, and the HCDR3 of the variable region set forth in SEQ ID NO: 69, and the light chain variable region comprises the LCDR1, the HCDR2, and the LCDR3 of the variable region set forth in SEQ ID NO: 70.

In some embodiments, the second antigen-binding moiety comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 70. In some embodiments, the heavy chain variable region of the second antigen-binding moiety comprises the amino acid sequence set forth in SEQ ID NO: 69, and the light chain variable region thereof comprises the amino acid sequence set forth in SEQ ID NO: 70. In some embodiments, for the second antigen-binding moiety, the amino acid sequence of the heavy chain variable region is set forth in SEQ ID NO: 69, and the amino acid sequence of the light chain variable region is set forth in SEQ ID NO: 70.

The multispecific antibody of the present disclosure may have no Fc domain, and the first antigen-binding moiety, the second antigen-binding moiety, and the third antigen-binding moiety are connected by suitable linkers.

The multispecific antibody of the present disclosure may have an Fc domain, and the Fc domain may prolong the half-life, provide the Fc domain-related effector, and the like.

*Multispecific Antibody Configurations*

The antigen-binding moieties in the multispecific antibody of the present disclosure may be fused to one another in a variety of configurations. In some embodiments, the multispecific antibody further comprises (iv) an Fc domain composed of two Fc polypeptides.

In some embodiments, the third antigen-binding moiety and the first antigen-binding moiety are fused to each other, optionally via a peptide linker. In some more specific embodiments, the third antigen-binding moiety, at the C-terminus, is fused to the N-terminus of the first antigen-binding moiety. Further, in some embodiments, the first antigen-binding moiety, at the C-terminus, is fused to the N-terminus of one of the Fc polypeptides of the Fc domain, the second antigen-binding moiety is a Fab, and the second antigen-binding moiety, at the C-terminus of the Fab heavy chain or the Fab light chain, is fused to the N-terminus of the other Fc polypeptide of the Fc domain.

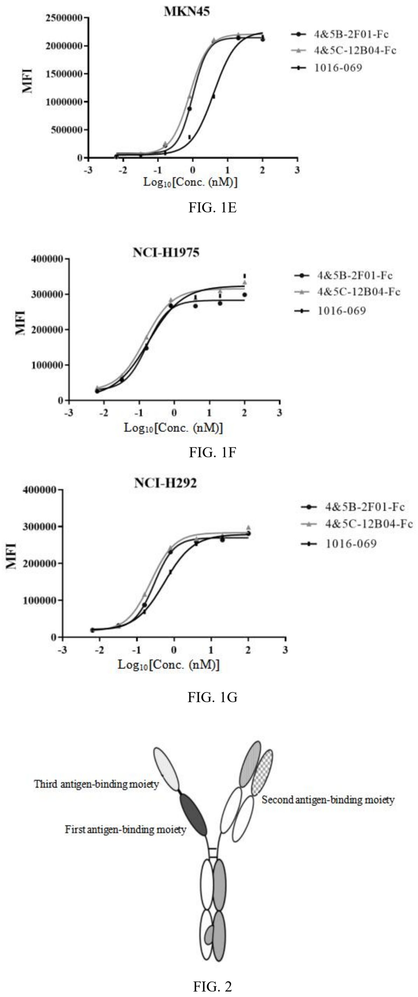

In one specific embodiment, the first antigen-binding moiety and the third antigen-binding moiety are both single variable domains, and the second antigen-binding moiety is a Fab; the first antigen-binding moiety, at the C-terminus, is fused to the N-terminus of one of the Fc polypeptides of the Fc domain, the second antigen-binding moiety, at the C-terminus of the Fab heavy chain, is fused to the N-terminus of the other Fc polypeptide of the Fc domain, and the third antigen-binding moiety, at the C-terminus, is fused to the N-terminus of the first antigen-binding moiety. Such configurations are schematically depicted in FIG. 2. For the embodiment, in further more specific embodiments, the multispecific antibody has three polypeptide chains, wherein a first polypeptide chain comprises the first antigen-binding moiety, the third antigen-binding moiety, and one of the Fc polypeptides of the Fc domain described above, a second polypeptide chain comprises the Fab heavy chain of the second antigen-binding moiety and the other Fc polypeptide of the Fc domain described above, and a third polypeptide chain is the Fab light chain of the second antigen-binding moiety described above.

In any one of the embodiments described above, the antigen-binding moieties of the multispecific antibody are operably linked, e.g., fused directly or via various peptide linkers (e.g., peptide linkers comprising one or more amino acids, typically about 1-50 amino acids) as described herein or known in the art and hinges, which can be properly selected by those skilled in the art.

The third antigen-binding moiety may be fused to the first antigen-binding moiety either directly or via a peptide linker. In one embodiment, the third antigen-binding moiety is fused to the first antigen-binding moiety via a peptide linker.

The peptide linkers may be each independently any suitable, e.g., electrically charged and/or flexible, linker polypeptide. In specific embodiments, the peptide linker consists of 1 to 50 amino acids linked by peptide bonds, wherein the amino acids may be selected from 20 naturally occurring amino acids; in a more preferred embodiment, the 1 to 50 amino acids are selected from glycine, alanine, proline, serine, asparagine, glutamine, and lysine. Thus, exemplary peptide linkers may be polyglycines (particularly $(Gly)_4$ and $(Gly)_5$), poly(Gly-Ser), $(Gly)_3AsnGlySer(Gly)_2$, $(Gly)_3Cys(Gly)_4$, GlyProAsnGlyGly, or those disclosed in Table 4 of Patent No. WO2019195535, etc.

In some embodiments, the peptide linker may be a peptide linker consisting of glycine and serine. In some embodiments, the peptide linker may comprise 0, 1, 2, 3, 4, 5, 6, 7, 8, 9, 10, 11, 12, 13, 14, 15, 16, 17, 18, 19, 20 or more amino acids. In some embodiments, the peptide linker is a peptide linker comprising GGGGS as a unit. In some embodiments, the peptide linker comprising GGGGS as a unit is (GGGGS)$_n$, wherein n is any number between 1 and 10, i.e., 1, 2, 3, 4, 5, 6, 7, 8, 9, and 10, or any range defined by any two of the aforementioned numbers, e.g., 1-5, 2-5, 3-6, 2-4, and 1-4. In some specific embodiments, the peptide linker is a linker polypeptide comprising GGGGS (SEQ ID NO: 91), (GGGGS)$_2$, (GGGGS)$_3$, or (GGGGS)$_4$. In some specific embodiments, the third antigen-binding moiety is fused to the first antigen-binding moiety via a peptide linker GGGGS (SEQ ID NO: 91) or (GGGGS)$_2$.

When the antigen-binding moiety is fused to an Fc, it is typically fused via a hinge region. In one embodiment, the first antigen-binding moiety is fused to one of the Fc polypeptides of the Fc domain via a first hinge, and the second antigen-binding moiety is fused to the other Fc polypeptides of the Fc domain via a second hinge. In some embodiments, the first hinge and the second hinge can form a covalent bond, such as a disulfide bond. The first hinge or/and the second hinge may comprise amino acids of the hinge region of a human IgG, and the hinge region of the human IgG comprises a natural hinge region or a variant thereof. In some embodiments, the first hinge or/and the second hinge comprises amino acids of the hinge region of a human IgG1. In some embodiments, the first hinge or/and the second hinge comprises amino acids of the hinge region of a human IgG4. In some specific embodiments, the first hinge comprises GEPKSSDKTHTCPPCP (SEQ ID NO: 89), and the second hinge comprises EPKSCDKTHTCPPCP (SEQ ID NO: 90).

*Fc Domain*

The Fc domain of the multispecific antibody consists of a pair of polypeptide chains comprising a heavy chain domain of an immunoglobulin molecule. For example, the Fc domain of immunoglobulin G (IgG) molecule is a dimer, and each of Fc polypeptides comprises CH2 and CH3 of the IgG heavy chain constant regions. The two Fc polypeptides of the Fc domain are capable of stable association with each other. In one embodiment, the multispecific antibody of the present disclosure comprises one Fc domain.

In some embodiments, the Fc domain of the multispecific antibody is an IgG Fc domain. In some embodiments, the Fc domain is an IgG1 Fc domain. In some embodiments, the Fc domain is a human Fc domain. In some specific embodiments, the Fc domain is a human IgG1 Fc domain.

In some embodiments, the Fc domain comprises a modification, such as an amino acid substitution. The modification may be, for example, a modification that promotes heterodimerization, a modification that alters the binding ability to protein A, etc.

In some embodiments, the Fc comprises a modification that promotes heterodimerization.

The multispecific antibody of the present disclosure comprises different antigen-binding moieties fused to one or the other of the two Fc polypeptides in the Fc domain. Therefore, the two Fc polypeptides are generally contained in two different polypeptide chains. Several possible combinations of the two polypeptides are generated by recombinant co-expression and subsequent dimerization of these polypeptides. In order to increase the yield and purity of the multispecific antibody in recombinant production, it would be advantageous to introduce a modification that promotes the binding of the desired polypeptides into the Fc domain of the multispecific antibody. Thus, in specific embodiments, the Fc domain comprises an amino acid substitution that promotes the association of the two Fc polypeptides of the Fc domain.

The site for the most extensive protein-protein interaction between the two Fc polypeptides of the human IgG Fc domain is in the CH3 domain of the Fc domain. Thus, in one embodiment, the modification is in the CH3 domain of the Fc domain.

In specific embodiments, the modification is a so-called "knob-into-hole" modification, which comprises a "knob" modification in one of the two Fc polypeptides of the Fc domain and a "hole" modification in the other one of the two Fc polypeptides of the Fc domain. Generally, the method involves introducing a protuberance ("knob") at the interface of one Fc polypeptide and a corresponding cavity ("hole") at the interface of the other Fc polypeptide, such that the protuberance can be positioned in the cavity to promote heterodimer formation and to interfere with homodimer formation. The protuberance is constructed by substituting a small amino acid side chain from the interface of one Fc polypeptide with a larger side chain (e.g., tyrosine, tryptophan, etc.). The complementary cavity having the same or similar size as the protuberance is produced in the interface of the other Fc polypeptide by substituting a large amino acid side chain with a smaller amino acid side chain (e.g., alanine or threonine, etc.). Thus, in specific embodiments, an amino acid residue is substituted with an amino acid residue having a larger side chain volume in the CH3 domain of one Fc polypeptide of the multispecific antibody, thereby producing a protuberance within the CH3 domain of the Fc polypeptide that can be positioned in a cavity within the CH3 domain of the other Fc polypeptide, and an amino acid residue is substituted with an amino acid residue having a smaller side chain volume in the CH3 domain of the other Fc polypeptide, thereby producing a cavity within the CH3 domain of the Fc polypeptide.

In some embodiments, according to the EU numbering, one Fc polypeptide of the Fc domain comprises amino acid substitutions 354C or/and 366Y/W, and the other Fc polypeptide comprises amino acid substitutions 349C, 366S, 368A, or/and 407T/V. In some specific embodiments, according to the EU numbering, one of the Fc polypeptides of the Fc domain comprises amino acid substitutions 354C and 366Y/W, and the other Fc polypeptide comprises amino acid substitutions 349C, 366S, 368A, and 407T/V. In some more specific embodiments, according to the EU numbering, one of the Fc polypeptides of the Fc domain comprises amino acid substitutions 354C and 366W, and the other Fc polypeptide comprises amino acid substitutions 349C, 366S, 368A, and 407V. In the above embodiments, the Fc may be an Fc of a human IgG1. In one specific embodiment, according to the EU numbering, one of the Fc polypeptides of the Fc domain comprises amino acid substitutions S354C and T366W, and the other Fc polypeptide comprises amino acid substitutions Y349C, T366S, L368A, and Y407V.

In some embodiments, the Fc domain comprises a modification that reduces or eliminates the binding of the CH3 region of one Fc polypeptide in the Fc domain to Protein A (from Staphylococcus aureus). In some embodiments, the Fc domain comprises an amino acid substitution that reduces or eliminates the binding of the CH3 region of one Fc polypeptide in the Fc domain to Protein A. In some embodiments, according to the EU numbering, the Fc domain comprises amino acid substitution(s): (a) 435R or (b) 435R and 436F that occur only in one Fc polypeptide but not in the other Fc polypeptide. In some embodiments, according to the EU numbering, the Fc domain comprises amino acid substitution(s): (a) 435R or (b) 435R and 436F that occur only in one of the Fc polypeptides. In some specific embodiments, according to the EU numbering, the Fc domain comprises amino acid substitutions H435R and Y436F that occur only in one of the Fc polypeptides. In some specific embodiments, according to the EU numbering, the Fc domain comprises an amino acid substitution H435R that occurs only in one of the Fc polypeptides. In the above embodiment, the Fc is an IgG1 Fc, particularly a human IgG1 Fc.

In the multispecific antibody of the present disclosure, the Fc domain may comprise (i) a modification that promotes heterodimerization and/or (ii) a modification that reduces or eliminates the binding of the CH3 region of one Fc polypeptide in the Fc domain to protein A. In some embodiments, the Fc comprises (i) a modification that promotes heterodimerization and (ii) a modification that reduces or eliminates the binding of the CH3 region of one Fc polypeptide in the Fc domain to protein A. For example, in one specific embodiment, according to the EU numbering, the Fc domain comprises the following groups of amino acid substitutions:

i. 349C, 366S, 368A, 407T/V, 354C, and 366Y/W, wherein amino acid substitutions 354C and 366Y/W are on the same Fc polypeptide, and are not on the same Fc polypeptide as the other amino acid substitutions in (i); and
ii. (a) 435R or (b) 435R and 436F that occur only in one of the Fc polypeptides.

In one more specific embodiment, according to the EU numbering, one of the Fc polypeptides of the Fc domain comprises amino acid substitutions Y349C, T366S, L368A, Y407V, H435R, and Y436F, and the other Fc polypeptide comprises amino acid substitutions S354C and T366W. In such a specific embodiment, the Fc is an IgG1 Fc, particularly a human IgG1 Fc.

In another more specific embodiment, according to the EU numbering, one of the Fc polypeptides of the Fc domain comprises amino acid substitutions Y349C, T366S, L368A, Y407V, and H435R, and the other Fc polypeptide comprises amino acid substitutions S354C and T366W. In such a specific embodiment, the Fc is an IgG1 Fc, particularly a human IgG1 Fc.

In the context of the present disclosure, amino acid substitutions are represented as: original amino acid-position-substituent amino acid, where the amino acid residues are represented by three-letter (Xaa) or single-letter (X) codes, and the original amino acid can be omitted. Thus, for example, the "H435R" or "435R" means that amino acid H or the original amino acid at position 435 is substituted with amino acid R. More than 1 substituted amino acid may be contained. For example, T366Y/W means that amino acid T at position 366 is substituted with amino acid Y or W.

In some embodiments, the Fc domain does not comprise fucose.

In some embodiments, the multispecific antibody of the present disclosure is afucosylated. The interaction of the multispecific antibody with FcγRIIIa can be improved by defucosylation, thereby enhancing the ADCC effect of the antibody. Methods of producing multispecific antibodies with little or no fucose at the glycosylation site in the Fc domain without altering the amino acid sequence are known in the art, for example, adjusting the composition of the medium containing the expressing cells, or knocking out fucose expression-related genes such as FUT8 in the expressing cells.

In some embodiments, the multispecific antibody described in the present disclosure is trivalent, i.e., the first antigen-binding moiety, the second antigen-binding moiety, and the third antigen-binding moiety each provide monovalent binding to the corresponding antigen.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

In some embodiments, the multispecific antibody consists of three polypeptide chains, wherein one polypeptide chain comprises the amino acid sequence set forth in SEQ ID NO: 71, another polypeptide chain comprises the amino acid sequence set forth in SEQ ID NO: 85, and the other polypeptide chain comprises the amino acid sequence set forth in SEQ ID NO: 87.

In some embodiments, the multispecific antibody consists of three polypeptide chains, wherein one polypeptide chain comprises the amino acid sequence set forth in SEQ ID NO: 73, another polypeptide chain comprises the amino acid sequence set forth in SEQ ID NO: 85, and the other polypeptide chain comprises the amino acid sequence set forth in SEQ ID NO: 87.

In some embodiments, the multispecific antibody consists of three polypeptide chains, wherein one polypeptide chain comprises the amino acid sequence set forth in SEQ ID NO: 75, another polypeptide chain comprises the amino acid sequence set forth in SEQ ID NO: 85, and the other polypeptide chain comprises the amino acid sequence set forth in SEQ ID NO: 87.

In some embodiments, the multispecific antibody consists of three polypeptide chains, wherein one polypeptide chain comprises the amino acid sequence set forth in SEQ ID NO: 77, another polypeptide chain comprises the amino acid sequence set forth in SEQ ID NO: 85, and the other polypeptide chain comprises the amino acid sequence set forth in SEQ ID NO: 87.

In some embodiments, the multispecific antibody consists of three polypeptide chains, wherein one polypeptide chain comprises the amino acid sequence set forth in SEQ ID NO: 79, another polypeptide chain comprises the amino acid sequence set forth in SEQ ID NO: 85, and the other polypeptide chain comprises the amino acid sequence set forth in SEQ ID NO: 87.

In some embodiments, the multispecific antibody consists of three polypeptide chains, wherein one polypeptide chain comprises the amino acid sequence set forth in SEQ ID NO: 81, another polypeptide chain comprises the amino acid sequence set forth in SEQ ID NO: 85, and the other polypeptide chain comprises the amino acid sequence set forth in SEQ ID NO: 87.

In some embodiments, the multispecific antibody consists of three polypeptide chains, wherein one polypeptide chain comprises the amino acid sequence set forth in SEQ ID NO: 83, another polypeptide chain comprises the amino acid sequence set forth in SEQ ID NO: 85, and the other polypeptide chain comprises the amino acid sequence set forth in SEQ ID NO: 87.

The present disclosure provides an exemplary trivalent multispecific antibody.

In one example, the multispecific antibody consists of three polypeptide chains, and the amino acid sequences of the three polypeptide chains are separately set forth in SEQ ID NO: 71, SEQ ID NO: 85, and SEQ ID NO: 87. In some embodiments, the nucleotide sequences encoding the three polypeptide chains are separately set forth in SEQ ID NO: 72, SEQ ID NO: 86, and SEQ ID NO: 88.

In one example, the multispecific antibody consists of three polypeptide chains, and the amino acid sequences of the three polypeptide chains are separately set forth in SEQ ID NO: 73, SEQ ID NO: 85, and SEQ ID NO: 87. In some embodiments, the nucleotide sequences encoding the three polypeptide chains are separately set forth in SEQ ID NO: 74, SEQ ID NO: 86, and SEQ ID NO: 88.

In one example, the multispecific antibody consists of three polypeptide chains, and the amino acid sequences of the three polypeptide chains are separately set forth in SEQ ID NO: 75, SEQ ID NO: 85, and SEQ ID NO: 87. In some embodiments, the nucleotide sequences encoding the three polypeptide chains are separately set forth in SEQ ID NO: 76, SEQ ID NO: 86, and SEQ ID NO: 88.

In one example, the multispecific antibody consists of three polypeptide chains, and the amino acid sequences of the three polypeptide chains are separately set forth in SEQ ID NO: 77, SEQ ID NO: 85, and SEQ ID NO: 87. In some embodiments, the nucleotide sequences encoding the three polypeptide chains are separately set forth in SEQ ID NO: 78, SEQ ID NO: 86, and SEQ ID NO: 88.

In one example, the multispecific antibody consists of three polypeptide chains, and the amino acid sequences of the three polypeptide chains are separately set forth in SEQ ID NO: 79, SEQ ID NO: 85, and SEQ ID NO: 87. In some embodiments, the nucleotide sequences encoding the three polypeptide chains are separately set forth in SEQ ID NO: 80, SEQ ID NO: 86, and SEQ ID NO: 88.

In one example, the multispecific antibody consists of three polypeptide chains, and the amino acid sequences of the three polypeptide chains are separately set forth in SEQ ID NO: 81, SEQ ID NO: 85, and SEQ ID NO: 87. In some embodiments, the nucleotide sequences encoding the three polypeptide chains are separately set forth in SEQ ID NO: 82, SEQ ID NO: 86, and SEQ ID NO: 88.

In one example, the multispecific antibody consists of three polypeptide chains, and the amino acid sequences of the three polypeptide chains are separately set forth in SEQ ID NO: 83, SEQ ID NO: 85, and SEQ ID NO: 87. In some embodiments, the nucleotide sequences encoding the three polypeptide chains are separately set forth in SEQ ID NO: 84, SEQ ID NO: 86, and SEQ ID NO: 88.

*Isolated Nucleic Acid*

The present disclosure provides an isolated nucleic acid comprising a nucleotide sequence encoding the multispecific antibody described in the present disclosure. Some nucleotide sequences encoding the multispecific antibody are illustratively listed in the sequence listing.

*Vector*

The present disclosure provides a vector comprising the nucleic acid. In some embodiments, the vector is a cloning vector; in some other embodiments, the vector is an expression vector; in one specific example, the expression vector is pcDNA3.1(+). The expression vector is optionally any expression vector capable of expressing the multispecific antibody described herein.

*Host Cell*

The present disclosure provides a host cell comprising the nucleic acid of the present disclosure or the vector of the present disclosure. In some embodiments, the host cell is a suitable host cell for cloning or expressing the multispecific antibody. In some embodiments, the host cell is a prokaryotic cell. In some other embodiments, the host cell is a eukaryotic cell. In some embodiments, the host cell is selected from a yeast cell, a mammalian cell, and other cells suitable for preparing a multispecific antibody. Mammalian cells are, for example, Chinese hamster ovary (CHO) cells or CHO-S cells.

*Antibody-Drug Conjuggate (ADC)*

The present disclosure provides an antibody-drug conjugate comprising the multispecific antibody of the present disclosure. In some embodiments, the antibody-drug conjugate comprises the multispecific antibody and a cytotoxic drug. The multispecific antibody and the cytotoxic drug are preferably linked by a linker, and the linker is a cleavable linker or a non-cleavable linker.

*Pharmaceutical Composition*

The present disclosure provides a pharmaceutical composition comprising the multispecific antibody of the present disclosure and further comprising one or more pharmaceutically acceptable excipients. The present disclosure provides a pharmaceutical composition comprising the antibody-drug conjugate of the present disclosure and further comprising one or more pharmaceutically acceptable excipients. The pharmaceutically acceptable excipient includes, for example, an excipient, a diluent, an encapsulating material, a filler, a buffering agent, or other reagents. In the pharmaceutical composition of the present disclosure, the multispecific antibody may be completely afucosylated (i.e., containing no detectable fucose) or may be partially afucosylated.

*Method for Preparing Multispecific Antibody*

In some embodiments, the present disclosure provides a method for preparing a multispecific antibody, comprising culturing the host cell to express the multispecific antibody, and optionally, isolating and purifying the multispecific antibody from the system. To produce the multispecific antibody, a nucleic acid encoding the multispecific antibody is isolated and inserted into one or more vectors for further use in cloning or/and expression in a host cell. The nucleic acid can be obtained using various methods known in the art, such as gene splicing and chemical synthesis. The prepared multispecific antibody can be purified by techniques known in the art, such as high-performance liquid chromatography, ion-exchange chromatography, gel chromatography, affinity chromatography, size-exclusion chromatography, ceramic hydroxyapatite (CHT) chromatography, etc. The actual conditions used to purify a particular protein depend in part on factors such as net charge, hydrophobicity, and hydrophilicity, which will be apparent to those skilled in the art. For affinity chromatography, antibodies, ligands, receptors, or antigens that bind to the multispecific antibody may be used for purification. For example, for affinity chromatography of the multispecific antibody of the present disclosure, purification may be performed using a matrix with protein A or protein G. The multispecific antibody of the present disclosure may be purified by affinity chromatography, ion-exchange chromatography, gel chromatography, or/and CHT chromatography sequentially.

The purity of the multispecific antibody can be determined by any of a variety of well-known analytical methods, such as gel electrophoresis, high-performance liquid chromatography, size-exclusion chromatography, and the like. The physicochemical properties or/and biological activity of the multispecific antibody described herein can be identified, screened, or characterized by a variety of assay methods known in the art.

*Use*

The present disclosure provides use of the multispecific antibody of the present disclosure. In some specific embodiments, the multispecific antibody used may be V12, V41, V35, V42, V67, V73, and/or V74.

The present disclosure provides use of the multispecific antibody, the antibody-drug conjugate, or the pharmaceutical composition for preparing a medicament for treating a disease expressing c-Met or/and EGFR. In some embodiments, the medicament is prepared using the multispecific antibody, the antibody-drug conjugate, or the pharmaceutical composition and one or more additional therapeutic agents. The additional therapeutic agent may be a therapeutic agent for a tumor known in the art.

The present disclosure provides a method for treating a disease expressing c-Met or/and EGFR, comprising: administering to a subject in need the multispecific antibody, the antibody-drug conjugate, or the pharmaceutical composition. The present disclosure provides a method for treating a disease expressing c-Met or/and EGFR, comprising: administering to a subject in need a therapeutically effective amount of the multispecific antibody, the antibody-drug conjugate, or the pharmaceutical composition. In some embodiments, the method further comprises: further administering to the subject in need one or more additional therapeutic agents. The additional therapeutic agent may be a therapeutic agent for a tumor known in the art. In some embodiments, the disease is a tumor.

In some embodiments, the tumor is epithelial cell cancer, breast cancer, ovarian cancer, lung cancer, lung adenocarcinoma, colorectal cancer, anal cancer, prostate cancer, kidney cancer, liver cancer, bladder cancer, head and neck cancer, gastric cancer, pancreatic cancer, skin cancer, oral cancer, pharyngeal cancer, nasal cancer, tongue cancer, esophageal cancer, testicular cancer, vaginal cancer, cervical cancer, spleen cancer, testicular cancer, thyroid cancer, salivary gland cancer, and/or thymus cancer. In some embodiments, the lung cancer includes non-small cell lung cancer (NSCLC) and small cell lung cancer (SCLC).

## DETAILED DESCRIPTION

The present disclosure further provides the following specific embodiments, which, however, are not intended to limit the scope of the present disclosure:

Embodiment 1. A multispecific antibody, comprising

(i) a first antigen-binding moiety binding to a first antigen;
(ii) a second antigen-binding moiety binding to a second antigen; and
(iii) a third antigen-binding moiety binding to the first antigen,

wherein the first antigen is c-Met, and the second antigen is EGFR; and the first antigen-binding moiety and the third antigen-binding moiety are both single variable domains and each independently comprise any one of the following:

(1) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is selected from S and T, preferably T;
(2) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;
(3) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;
(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;
(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;
(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;
(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or
(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

Embodiment 2. The multispecific antibody according to embodiment 1, wherein the first antigen-binding moiety and the third antigen-binding moiety each independently comprise any one of the following:

(1) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is selected from S and T, preferably T;
(2) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; or
(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

Embodiment 3. The multispecific antibody according to embodiment 2, wherein the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is selected from S and T; preferably, $X_1$ is T.

Embodiment 4. The multispecific antibody according to embodiment 2, wherein the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

Embodiment 5. A multispecific antibody, comprising

> (i) a first antigen-binding moiety binding to a first antigen;
> (ii) a second antigen-binding moiety binding to a second antigen; and
> (iii) a third antigen-binding moiety binding to the first antigen,

wherein the first antigen is c-Met, and the second antigen is EGFR; and the first antigen-binding moiety and the third antigen-binding moiety are both single variable domains and each independently comprise a CDR1, a CDR2, and a CDR3 of a single variable domain having the amino acid sequence set forth in SEQ ID NO: 35, 36, 26, 27, 25, 28, 29, 30, 31, 32, 33, 34, 37, 38, or 39.

Embodiment 6. The multispecific antibody according to embodiment 5, wherein the first antigen-binding moiety and the third antigen-binding moiety each independently comprise a CDR1, a CDR2, and a CDR3 of a single variable domain having the amino acid sequence set forth in SEQ ID NO: 35, 36, or 38.

Embodiment 7. The multispecific antibody according to embodiment 6, wherein the first antigen-binding moiety comprises a CDR1, a CDR2, and a CDR3 of a single variable domain having the amino acid sequence set forth in SEQ ID NO: 36, and the third antigen-binding moiety comprises a CDR1, a CDR2, and a CDR3 of a single variable domain having the amino acid sequence set forth in SEQ ID NO: 35.

Embodiment 8. The multispecific antibody according to embodiment 6, wherein the first antigen-binding moiety comprises a CDR1, a CDR2, and a CDR3 of a single variable domain having the amino acid sequence set forth in SEQ ID NO: 36, and the third antigen-binding moiety comprises a CDR1, a CDR2, and a CDR3 of a single variable domain having the amino acid sequence set forth in SEQ ID NO: 38.

Embodiment 9. The multispecific antibody according to any one of embodiments 1-8, wherein the first antigen-binding moiety and the third antigen-binding moiety each independently comprise an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40, 41, 35, 36, 26, 27, 25, 28, 29, 30, 31, 32, 33, 34, 37, 38, 39, or 42.

Embodiment 10. The multispecific antibody according to any one of embodiments 1-9, wherein the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41.

Embodiment 11. The multispecific antibody according to embodiment 10, wherein the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 27, 36, or 37; preferably, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 36.

Embodiment 12. The multispecific antibody according to any one of embodiments 1-11, wherein the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40.

Embodiment 13. The multispecific antibody according to embodiment 12, wherein the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 26, 33, 34, or 35; preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35.

Embodiment 14. The multispecific antibody according to any one of embodiments 1-11, wherein the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 42.

Embodiment 15. The multispecific antibody according to embodiment 14, wherein the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32, 38, or 39; preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 38.

Embodiment 16. The multispecific antibody according to any one of embodiments 1-8, wherein the first antigen-binding moiety and the third antigen-binding moiety are selected from any one of the following:

(1) the first antigen-binding moiety comprises the amino acid sequence set forth in SEQ ID NO: 36, and the third antigen-binding moiety comprises the amino acid sequence set forth in SEQ ID NO: 35;
(2) the first antigen-binding moiety comprises the amino acid sequence set forth in SEQ ID NO: 27, and the third antigen-binding moiety comprises the amino acid sequence set forth in SEQ ID NO: 26;
(3) the first antigen-binding moiety comprises the amino acid sequence set forth in SEQ ID NO: 36, and the third antigen-binding moiety comprises the amino acid sequence set forth in SEQ ID NO: 33;
(4) the first antigen-binding moiety comprises the amino acid sequence set forth in SEQ ID NO: 36, and the third antigen-binding moiety comprises the amino acid sequence set forth in SEQ ID NO: 34;
(5) the first antigen-binding moiety comprises the amino acid sequence set forth in SEQ ID NO: 36, and the third antigen-binding moiety comprises the amino acid sequence set forth in SEQ ID NO: 32;
(6) the third antigen-binding moiety comprises the amino acid sequence set forth in SEQ ID NO: 36, and the first antigen-binding moiety comprises the amino acid sequence set forth in SEQ ID NO: 38; or
(7) the third antigen-binding moiety comprises the amino acid sequence set forth in SEQ ID NO: 36, and the first antigen-binding moiety comprises the amino acid sequence set forth in SEQ ID NO: 39.

Embodiment 17. The multispecific antibody according to any one of embodiments 1-16, wherein the single variable domain is derived from a camelid or is humanized.

Embodiment 18. The multispecific antibody according to any one of embodiments 1-17, wherein the second antigen-binding moiety is a Fab, an scFv, or an scFab.

Embodiment 19. The multispecific antibody according to any one of embodiments 1-18, wherein the second antigen-binding moiety comprises a heavy chain variable region and a light chain variable region; the heavy chain variable region comprises: an HCDR1 comprising the amino acid sequence set forth in SEQ ID NO: 63, an HCDR2 comprising the amino acid sequence set forth in SEQ ID NO: 64, and an HCDR3 comprising the amino acid sequence set forth in SEQ ID NO: 65, and the light chain variable region comprises an LCDR1 comprising the amino acid sequence set forth in SEQ ID NO: 66, an LCDR2 comprising the amino acid sequence set forth in SEQ ID NO: 67, and an LCDR3 comprising the amino acid sequence set forth in SEQ ID NO: 68.

Embodiment 20. The multispecific antibody according to any one of embodiments 1-18, wherein the second antigen-binding moiety comprises a heavy chain variable region and a light chain variable region; the heavy chain variable region comprises the HCDR1, the HCDR2, and the HCDR3 of the variable region set forth in SEQ ID NO: 69, and the light chain variable region comprises the LCDR1, the HCDR2, and the LCDR3 of the variable region set forth in SEQ ID NO: 70.

Embodiment 21. The multispecific antibody according to any one of embodiments 1-20, wherein the second antigen-binding moiety comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 70.

Embodiment 22. The multispecific antibody according to embodiment 21, wherein the heavy chain variable region of the second antigen-binding moiety comprises the amino acid sequence set forth in SEQ ID NO: 69, and the light chain variable region thereof comprises the amino acid sequence set forth in SEQ ID NO: 70. Embodiment 23. The multispecific antibody according to any one of embodiments 1-22, wherein the second antigen-binding moiety is murine, chimeric, or humanized.

Embodiment 24. The multispecific antibody according to any one of embodiments 1-23, wherein the third antigen-binding moiety and the first antigen-binding moiety are fused to each other, optionally via a peptide linker. Embodiment 25. The multispecific antibody according to embodiment 24, wherein the third antigen-binding moiety, at the C-terminus, is fused to the N-terminus of the first antigen-binding moiety.

Embodiment 26. The multispecific antibody according to any one of embodiments 1-25, further comprising: (iv) an Fc domain composed of two Fc polypeptides.

Embodiment 27. The multispecific antibody according to embodiment 26, wherein the first antigen-binding moiety, at the C-terminus, is fused to the N-terminus of one of the Fc polypeptides of the Fc domain, the second antigen-binding moiety is a Fab, and the second antigen-binding moiety, at the C-terminus of the Fab heavy chain, is fused to the N-terminus of the other Fc polypeptide of the Fc domain.

Embodiment 28. The multispecific antibody according to embodiment 26 or 27, wherein the Fc domain is an IgG Fc domain, preferably an IgG1 Fc domain.

Embodiment 29. The multispecific antibody according to embodiment 28, wherein the IgG Fc domain is a human IgG Fc domain, preferably a human IgG1 Fc domain.

Embodiment 30. The multispecific antibody according to any one of embodiments 26-29, wherein the Fc domain comprises an amino acid substitution that promotes the association of the two Fc polypeptides of the Fc domain.

Embodiment 31. The multispecific antibody according to embodiment 30, wherein according to the EU numbering, one of the Fc polypeptides of the Fc domain comprises amino acid substitutions 354C and 366Y/W, and the other Fc polypeptide comprises amino acid substitutions 349C, 366S, 368A, and 407T/V.

Embodiment 32. The multispecific antibody according to any one of embodiments 26-31, wherein the Fc domain comprises an amino acid substitution that reduces or eliminates the binding of the CH3 region of one Fc polypeptide in the Fc domain to protein A.

Embodiment 33. The multispecific antibody according to embodiment 32, wherein according to the EU numbering, the Fc domain comprises amino acid substitution(s): (a) 435R or (b) 435R and 436F that occur only in one of the Fc polypeptides.

Embodiment 34. The multispecific antibody according to any one of embodiments 26-29, wherein according to the EU numbering, one of the Fc polypeptides of the Fc domain comprises amino acid substitutions 349C, 366S, 368A, 407V, 435R, and 436F, and the other Fc polypeptide comprises amino acid substitutions 354C and 366W. Embodiment 35. The multispecific antibody according to any one of embodiments 26-29, wherein according to the EU numbering, one of the Fc polypeptides of the Fc domain comprises amino acid substitutions 349C, 366S, 368A, 407V, and 435R, and the other Fc polypeptide comprises amino acid substitutions 354C and 366W. Embodiment 36. The multispecific antibody according to any one of embodiments 1-35, wherein the multispecific antibody is trivalent.

Embodiment 37. The multispecific antibody according to any one of embodiments 1-36, wherein the multispecific antibody consists of three polypeptide chains, wherein:

(1) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(2) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(3) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(4) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(5) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(6) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87; or

(7) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87.

Embodiment 38. A multispecific antibody, consisting of three polypeptide chains, wherein:

(1) one polypeptide chain comprises the amino acid sequence set forth in SEQ ID NO: 77, another polypeptide chain comprises the amino acid sequence set forth in SEQ ID NO: 85, and the other polypeptide chain comprises the amino acid sequence set forth in SEQ ID NO: 87;

(2) one polypeptide chain comprises the amino acid sequence set forth in SEQ ID NO: 71, another polypeptide chain comprises the amino acid sequence set forth in SEQ ID NO: 85, and the other polypeptide chain comprises the amino acid sequence set forth in SEQ ID NO: 87;

(3) one polypeptide chain comprises the amino acid sequence set forth in SEQ ID NO: 73, another polypeptide chain comprises the amino acid sequence set forth in SEQ ID NO: 85, and the other polypeptide chain comprises the amino acid sequence set forth in SEQ ID NO: 87;

(4) one polypeptide chain comprises the amino acid sequence set forth in SEQ ID NO: 75, another polypeptide chain comprises the amino acid sequence set forth in SEQ ID NO: 85, and the other polypeptide chain comprises the amino acid sequence set forth in SEQ ID NO: 87;

(5) one polypeptide chain comprises the amino acid sequence set forth in SEQ ID NO: 79, another polypeptide chain comprises the amino acid sequence set forth in SEQ ID NO: 85, and the other polypeptide chain comprises the amino acid sequence set forth in SEQ ID NO: 87;

(6) one polypeptide chain comprises the amino acid sequence set forth in SEQ ID NO: 81, another polypeptide chain comprises the amino acid sequence set forth in SEQ ID NO: 85, and the other polypeptide chain comprises the amino acid sequence set forth in SEQ ID NO: 87; or

(7) one polypeptide chain comprises the amino acid sequence set forth in SEQ ID NO: 83, another polypeptide chain comprises the amino acid sequence set forth in SEQ ID NO: 85, and the other polypeptide chain comprises the amino acid sequence set forth in SEQ ID NO: 87.

Embodiment 39. The multispecific antibody according to any one of embodiments 1-38, wherein the multispecific antibody is afucosylated.

Embodiment 40. An isolated nucleic acid, comprising a nucleotide sequence encoding the multispecific antibody according to any one of embodiments 1-39.

Embodiment 41. A vector, comprising the nucleic acid according to embodiment 40.

Embodiment 42. A host cell, comprising the nucleic acid according to embodiment 40 or the vector according to embodiment 41.

Embodiment 43. A method for preparing the multispecific antibody according to any one of embodiments 1-39, comprising: culturing the host cell according to embodiment 42 to express the multispecific antibody, and isolating and purifying the multispecific antibody from the system.

Embodiment 44. An antibody-drug conjugate, comprising the multispecific antibody according to any one of embodiments 1-39 and a cytotoxic drug.

Embodiment 45. A pharmaceutical composition, comprising the multispecific antibody according to any one of embodiments 1-39 or the antibody-drug conjugate according to embodiment 44, and a pharmaceutically acceptable excipient.

Embodiment 46. Use of the multispecific antibody according to any one of embodiments 1-39, the antibody-drug conjugate according to embodiment 44, or the pharmaceutical composition according to embodiment 45 for preparing a medicament for treating a disease expressing c-Met or/and EGFR.

Embodiment 47. The use according to embodiment 46, wherein the disease is a tumor.

Embodiment 48. The use according to embodiment 47, wherein the tumor is epithelial cell cancer, breast cancer, ovarian cancer, lung cancer, lung adenocarcinoma, colorectal cancer, anal cancer, prostate cancer, kidney cancer, liver cancer, bladder cancer, head and neck cancer, gastric cancer, pancreatic cancer, skin cancer, oral cancer, pharyngeal cancer, nasal cancer, tongue cancer, esophageal cancer, testicular cancer, vaginal cancer, cervical cancer, spleen cancer, testicular cancer, thyroid cancer, salivary gland cancer, or thymus cancer.

Embodiment 49. The use according to embodiment 48, wherein the lung cancer is non-small cell lung cancer or small cell lung cancer.

Embodiment 50. The use according to any one of embodiments 46-49, wherein the medicament is prepared using the multispecific antibody, the antibody-drug conjugate, or the pharmaceutical composition and one or more additional therapeutic agents.

Embodiment 51. A method for treating a disease expressing c-Met or/and EGFR, comprising: administering to a subject in need a therapeutically effective amount of the multispecific antibody according to any one of embodiments 1-39, the antibody-drug conjugate according to embodiment 44, or the pharmaceutical composition according to embodiment 45.

Embodiment 52. The method according to embodiment 51, wherein the disease is a tumor.

Embodiment 53. The method according to embodiment 52, wherein the tumor is epithelial cell cancer, breast cancer, ovarian cancer, lung cancer, lung adenocarcinoma, colorectal cancer, anal cancer, prostate cancer, kidney cancer, liver cancer, bladder cancer, head and neck cancer, gastric cancer, pancreatic cancer, skin cancer, oral cancer, pharyngeal cancer, nasal cancer, tongue cancer, esophageal cancer, testicular cancer, vaginal cancer, cervical cancer, spleen cancer, testicular cancer, thyroid cancer, salivary gland cancer, or thymus cancer.

Embodiment 54. The method according to embodiment 53, wherein the lung cancer is non-small cell lung cancer or small cell lung cancer.

Embodiment 55. A c-Met-binding antibody, comprising a single variable domain, wherein the single variable domain comprises:

(1) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is S or T;

(2) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;

(3) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;

(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;

(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;

(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;

(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

Embodiment 56. The c-Met-binding antibody according to embodiment 55, wherein the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40, 41, 35, 36, 26, 27, 25, 28, 29, 30, 31, 32, 33, 34, 37, 38, 39, or 42.

Embodiment 57. The c-Met-binding antibody according to embodiment 56, wherein the single variable domain comprises the amino acid sequence set forth in SEQ ID NO: 33, 34, 35, 36, 37, 38, or 39.

Embodiment 58. The c-Met-binding antibody according to embodiment 56, wherein the single variable domain comprises the amino acid sequence set forth in SEQ ID NO: 40, 41, or 42, but does not comprise the amino acid sequence set forth in SEQ ID NO: 29, 30, or 31.

Embodiment 59. The c-Met-binding antibody according to any one of embodiments 55-58, wherein the single variable domain is derived from a camelid or is humanized.

Embodiment 60. The c-Met-binding antibody according to any one of embodiments 55-59, wherein the c-Met-binding antibody comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 44, 45, 43, 46, 47, 48, 49, 50, 51, 53, 55, 57, or 59.

For clarity, the present disclosure is further described with the following examples, which are, however, not intended to limit the scope of the present disclosure. The reagents used in the present disclosure are commercially available and can be used without further purification. The amivantamab used in the examples was purchased from Janssen, NDC No. 57894-501-01.

## Example 1. Construction of anti-human c-Met $V_H$Hphage display library

A recombinant human c-Met-Fc fusion protein (SinoBiological, Cat. No. 10692-H02H) was mixed and emulsified with complete Freund's adjuvant in a volume ratio of 1:1. A bactrian camel was first immunized by subcutaneous multipoint injection, and then boosted with the recombinant human c-Met-Fc fusion protein mixed and emulsified with incomplete Freund's adjuvant in a volume ratio of 1:1 at intervals of 2 weeks. The serum was collected after the fourth or fifth immunization and assayed for anti-human c-Met antibody titer. After multiple rounds of immunization, the peripheral blood of the bactrian camel was collected, and peripheral blood mononuclear cells (PBMCs) were isolated. The total RNA of the PBMCs was extracted and then reversely transcribed into cDNA, and the variable region ($V_H$H) sequence of the camel antibody was amplified by nested PCR.

The $V_H$H coding fragment obtained from the amplification was digested with PstI/NotI endonuclease and inserted into a phagemid vector pMECS (NTCC Plasmid Vector, Strain, Cell and Gene Collection, Cat. No. pMECS) to construct a recombinant vector, which was then transferred into *Escherichia coli* TG1 (Lucigen, Cat. No. 60502-1) by electroporation to construct a library stock. The library stock was amplified to logarithmic growth phase. Library amplification was performed by adding M13KO7 helper phages (New England Biolabs, Cat. No. N0315S), with shaking overnight at 200 rpm at 28°C. The bacterial liquid was centrifuged, and the supernatant was collected. 1/4 supernatant volume of a PEG6000/NaCl solution (20% PEG6000 (w/v), 2.5 M NaCl) was added. The mixture was incubated on ice for 1-2 h to settle the phages. The settled phages were centrifuged and collected resuspended in PBS, and then 20% glycerol was added. The suspension was preserved at -80°C as a $V_H$H phage display library.

## Example 2. Anti-human c-Met $V_H$H screening

The $V_H$H phage display library was subjected to solid-phase panning, and the single clones obtained from the panning were cultured. Expression was induced by isopropyl-β-D-thiogalactoside (IPTG), and the supernatant was prepared.

The picked clones were subjected to positive identification by indirect ELISA for human c-Met-His (SinoBiological, Cat. No. 10692-H08H). Positive clones that bind only to human c-Met-His with relatively high signal values were selected for preservation and sequenced. Positive clones 1B-1B2, 1B-3B11, 1B-1C7, 1B-1B6, 1B-1A8, 3B-1C7, 4&5B-2F01, and 4&5C-12B04 were selected via the screening. As determined by sequence analysis, the amino acid sequence of the $V_H$H of 1B-3B11 is set forth in SEQ ID NO: 25, the amino acid sequence of the $V_H$H of 1B-1B6 is set forth in SEQ ID NO: 26, the amino acid sequence of the $V_H$H of 1B-1C7 is set forth in SEQ ID NO: 27, the amino acid sequence of the $V_H$H of 1B-1A8 is set forth in SEQ ID NO: 28, and the amino acid sequence of the $V_H$H of 1B-1B2 is set forth in SEQ ID NO: 29; the amino acid sequence of the $V_H$H of 3B-1C7 is set forth in SEQ ID NO: 30; the amino acid sequence of the $V_H$H of 4&5B-2F01 is set forth in SEQ ID NO: 31; the amino acid sequence of the $V_H$H of 4&5C-12B04 is set forth in SEQ ID NO: 32.

## Example 3. Preparation of anti-human c-Met $V_H$H-Fc chimeric antibodies

The $V_H$H sequences of the positive clones obtained from the screening were linked to a human Fc region to construct $V_H$H-Fc chimeric antibodies. Specifically, the $V_H$H sequences sequenced in Example 2 were inserted into a pcDNA3.1(+) eukaryotic expression vector containing a human IgG1 Fc region. These $V_H$H-Fc chimeric antibodies were expressed using an Expifectamine™ CHO Transfection Kit transient transfection and expression system (Thermo Fisher Scientific Inc., Cat. No. A29129). The sequences of VL1016-069 and VH1016-069 in Patent No. US20200079872A1 were separately inserted into a pcDNA3.1(+) eukaryotic expression vector containing a human IgG1 constant region (the amino acid sequence is SEQ ID NO: 92), and a chimeric antibody 1016-069 was expressed as a control using the same method.

As determined by sequence analysis, the amino acid sequence of 1B-3B11-Fc is set forth in SEQ ID NO: 43, the amino acid sequence of 1B-1B6-Fc is set forth in SEQ ID NO: 44, the amino acid sequence of 1B-1C7-Fc is set forth in SEQ ID NO: 45, the amino acid sequence of 1B-1A8-Fc is set forth in SEQ ID NO: 46, the amino acid sequence of 1B-1B2-Fc is set forth in SEQ ID NO: 47, the amino acid sequence of 3B-1C7-Fc is set forth in SEQ ID NO: 48, the amino acid sequence of chimeric antibody 4&5B-2F01-Fc is set forth in SEQ ID NO: 49, and the amino acid sequence of chimeric antibody 4&5C-12B04-Fc is set forth in SEQ ID NO: 50.

## Example 4. Affinity of anti-human V$_H$H-Fc chimeric antibodies for human and cynomolgus c-Met

4.1 Determination of affinity of antibodies for human and cynomolgus c-Met by surface plasmon resonance

The anti-human c-Met V$_H$H-Fc chimeric antibodies were tested for affinity using a biomolecular interaction analysis system (GE, Biacore T200 or Biacore 8K). An anti-hIgG (Fc) antibody (GE, Cat. No. BR-1008-39) was amino-coupled to a CM5 sensor chip. The anti-human c-Met V$_H$H-Fc chimeric antibodies were diluted to 1 μg/mL with a running buffer (137 mM NaCl, 2.7 mM KCl, 10 mM Na$_2$HPO$_4$·12H$_2$O, 1.8 mM KH$_2$PO$_4$, 0.05% surfactant P-20 (w/v), pH 7.4), and the capture was conducted by passing the dilutions through experimental channels at a flow rate of 30 μL/min. Human c-Met-His (SinoBiological, Cat. No. 10692-H08H) or cynomolgus c-Met-His (SinoBiological, Cat. No. 90304-C08H) was diluted with the running buffer to 100 nM, 50 nM, 25 nM, 12.5 nM, 6.25 nM and 3.125 nM, and the association was conducted for 200 s by passing the dilutions at a flow rate of 50 μL/min. Then dissociation was conducted for 600-1400 s by stopping sample injection. The data signals were collected by BiaControl Software 2.0 or Biacore 8K Control Software 3.0 in real time, and analyzed by BiaEvaluation Software 2.0 or Biacore Insight Evaluation Software 3.0. Fitting was performed using a Langmuir 1:1 model, and the association constant $k_a$ (1/Ms), the dissociation constant $k_d$ (1/s), and the equilibrium dissociation constant $K_D$ (M) were calculated. The results are shown in Tables 1-1 and 1-2. 1B-1B2-Fc, 1B-3B11-Fc, 1B-1C7-Fc, 1B-1B6-Fc, 1B-1A8-Fc, 3B-1C7-Fc, 4&5B-2F01-Fc, and 4&5C-12B04-Fc all have exhibited high affinity for human c-Met protein and cross-reactions with cynomolgus c-Met protein.

Table 1-1. Affinity of V$_H$H-Fc chimeric antibodies for human c-Met and cynomolgus c-Met

| V$_H$H-Fc Chimeric antibody | Human c-Met-His | | | Cynomolgus c-Met-His | | |
|---|---|---|---|---|---|---|
| | $k_a$(1/Ms) | $k_a$(1/s) | $K_D$(M) | $k_a$(1/Ms) | $k_a$(1/s) | $K_D$(M) |
| 1B-3B11-Fc | 5.03E+04 | 6.81E-05 | 1.35E-09 | 4.35E+04 | 2.88E-05 | 6.64E-10 |
| 1B-1B2-Fc | 1.41E+05 | 5.60E-04 | 3.99E-09 | 7.84E+04 | 2.15E-04 | 2.74E-09 |
| 1B-1C7-Fc | 2.21E+05 | 4.43E-05 | 2.00E-10 | 2.12E+05 | 3.99E-05 | 1.88E-10 |
| 1B-1B6-Fc | 8.44E+04 | 6.96E-04 | 8.25E-09 | 5.07E+04 | 3.47E-04 | 6.85E-09 |
| 1B-1A8-Fc | 1.73E+05 | 1.12E-04 | 6.48E-10 | 1.30E+05 | 8.80E-05 | 6.77E-10 |
| 3B-1C7-Fc | 1.76E+05 | 5.56E-05 | 3.17E-10 | 2.89E+05 | 7.35E-05 | 2.54E-10 |

Table 1-2. Affinity of V$_H$H-Fc chimeric antibodies for human c-Met and cynomolgus c-Met

| V$_H$H-Fc Chimeric antibody | Human c-Met-His | | | Cynomolgus c-Met-His | | |
|---|---|---|---|---|---|---|
| | $k_a$(1/Ms) | $k_a$(1/s) | $K_D$(M) | $k_a$(1/Ms) | $k_d$(1/s) | $K_D$(M) |
| 4&5B-2F01-Fc | 9.51E+04 | 6.44E-05 | 6.76E-10 | 5.21E+04 | 5.44E-04 | 1.04E-08 |
| 4&5C-12B04-Fc | 1.74E+05 | 8.96E-05 | 5.16E-10 | 1.45E+05 | 7.22E-05 | 4.98E-10 |
| 1016-069 | 2.83E+05 | 2.52E-04 | 8.91E-10 | 4.30E+05 | 1.97E-04 | 4.59E-10 |

4.2 Assay of binding of antibodies to cells by flow cytometry

The binding of the anti-human c-Met V$_H$H-Fc chimeric antibodies to target cells with different c-Met expression levels was determined by flow cytometry. The NCI-H1993 cells (BeNa Culture Collection, Cat. No. BNCC342186) are human lung adenocarcinoma cells with a high c-Met expression level, the MKN45 cells (Nanjing Cobioer Biosciences Co., Ltd., Cat. No. CBP60541) are human gastric cancer cells with a medium c-Met expression level, the KP4 cells (Nanjing Cobioer Biosciences Co., Ltd., Cat. No. CBP60541) are human pancreatic cancer cells with a low c-Met expression level, the NCI-H1975 cells (BeNa Culture Collection, Cat. No. BNCC100690) are human non-small cell lung adenocarcinoma cells with a low to medium c-Met expression level, and the NCI-H292 cells (BeNa Culture Collection, Cat. No.

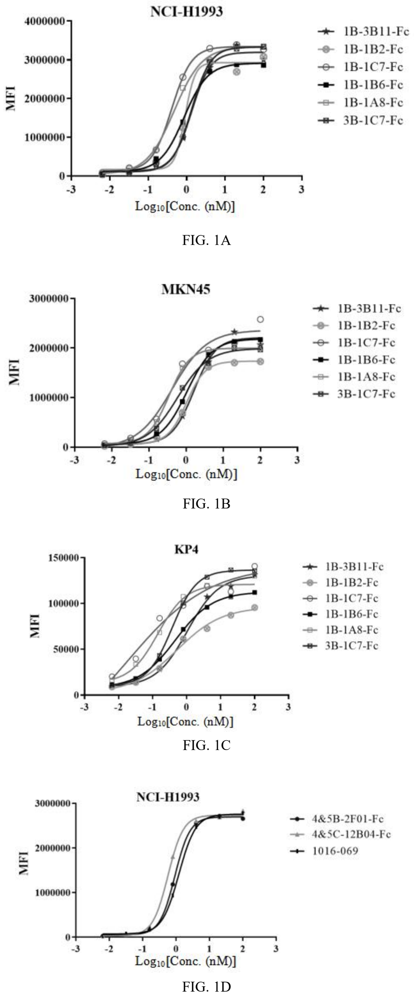

BNCC100671) are human epidermal lung carcinoma cells with a low to medium c-Met expression level. After $2 \times 10^5$ target cells were incubated with a serially diluted anti-human c-Met $V_H$H-Fc chimeric antibodies (serially diluted 5-fold from the initial concentration of 100 nM to 7 concentrations) on ice for 1 h, the cells were washed. A PE-labeled anti-human IgG Fc antibody (Jackson Immuno Research, Cat. No. 109-116-170) was added, and the mixture was incubated on ice for 0.5 h. The cells were washed and then tested using a flow cytometer (Thermo Fisher Scientific Inc., Attune NXT). The results are shown in FIGs. 1A-1G and Tables 2-1 and 2-2. 1B-1B2-Fc, 1B-3B11-Fc, 1B-1C7-Fc, 1B-1B6-Fc, 1B-1A8-Fc, 3B-1C7-Fc, 4&5B-2F01-Fc, and 4&5C-12B04-Fc all exhibited high binding ability to target cells with different c-Met expression levels, among which 4&5B-2F01-Fc and 4&5C-12B04-Fc were superior to the control 1016-069.

Table 2-1. Binding of anti-human c-Met $V_H$H-Fc chimeric antibodies to target cells

| $V_H$H-Fc Chimeric antibody | NCI-H1993 cell | MKN45 cell | KP4 cell |
|---|---|---|---|
| | $EC_{50}$(nM) | $EC_{50}$(nM) | $EC_{50}$(nM) |
| 1B-3B11-Fc | 1.333 | 1.615 | 1.049 |
| 1B-1B2-Fc | 0.910 | 1.034 | 0.526 |
| 1B-1C7-Fc | 0.407 | 0.421 | 0.019 |
| 1B-1B6-Fc | 0.854 | 1.056 | 0.471 |
| 1B-1A8-Fc | 0.477 | 0.345 | 0.143 |
| 3B-1C7-Fc | 1.352 | 0.592 | 0.375 |

Table 2-2. Binding of anti-human c-Met $V_H$H-Fc chimeric antibodies to target cells

| $V_H$H-Fc Chimeric antibody | NCI-H1993 cell $EC_{50}$ (nM) | MKN45 cell $EC_{50}$ (nM) | NCI-H1975 cell $EC_{50}$ (nM) | NCI-H292 cell $EC_{50}$ (nM) |
|---|---|---|---|---|
| 4&5B-2F01-Fc | 0.924 | 0.988 | 0.166 | 0.292 |
| 4&5C-12B04-Fc | 0.574 | 0.809 | 0.144 | 0.236 |
| 1016-069 | 1.217 | 3.950 | 0.185 | 0.550 |

## Example 5. Epitope difference analysis between different clones of anti-human c-Met $V_H$H

Epitope competition relationship analysis was performed using a biomolecular interaction system (Fortebio, Cat. No. Octet RED96). An Anti-Penta-HIS (HIS1K) sensor (Fortebio, Cat. No. 18-5120) was used, and the c-Met-His protein (SinoBiological, Cat. No. 10692-H08H) was diluted to about 5 μg/mL using a running buffer. The sensor was immersed in the diluted antigen sample. The immobilization height was controlled to about 1 nm by adjusting the binding time. Then the antigen sample interacted with a first antibody A and a second antibody B in sequence, and the B antibody binding signal was detected to determine whether the two antibodies recognize the same epitope. The results are shown in Tables 3-1, 3-2, and 3-3. The determination criteria were: a value > 60% indicated that the 2 antibodies do not compete at all; a value between 20% and 60% indicated a partial competition of the 2 antibodies (there may be a crossover at the epitopes); a value < 20% indicated a complete competition of the 2 antibodies. If the self-reaction signal (underlined) was < 20%, the data were determined valid.

As can be seen from the data in Table 3-1, the two antibodies 1B-3B11-Fc and 1B-1A8-Fc exhibited a complete competition; 1B-3B11-Fc and 1B-1A8-Fc demonstrated no significant competitive relationship with the other three candidate antibodies, suggesting different epitopes. Thus, the antibodies 1B-3B11-Fc and 1B-1B2-Fc, 1B-3B11-Fc and 1B-1C7-Fc, 1B-3B11-Fc and 1B-1B6-Fc, 1B-1A8-Fc and 1B-1B2-Fc, 1B-1A8-Fc and 1B-1C7-Fc, 1B-1A8-Fc and 1B-1B6-Fc, 1B-1B2-Fc and 1B-1C7-Fc, 1B-1B2-Fc and 1B-1B6-Fc, and 1B-1C7-Fc and 1B-1B6-Fc can simultaneously bind to different epitopes of the c-Met antigen. As can be seen from the data in Table 3-2, the two antibodies 3B-1C7-Fc and 1B-1B2-Fc exhibited a complete competition. Thus, by combining the data from Table 3-1, it can be seen that: the antibodies 1B-1A8-Fc and 3B-1C7-Fc, 3B-1C7-Fc and 1B-1C7-Fc, and 3B-1C7-Fc and 1B-1B6-Fc can simultaneously bind to different epitopes of the c-Met antigen.

As can be seen from the data in Table 3-3, 4&5B-2F01-Fc and 4&5C-12B04-Fc exhibited a complete competition with 1016-069.

Table 3-1. Epitope difference analysis

| AB# | 1B-3B11-Fc | 1B-1B2-Fc | 1B-1C7-Fc | 1B-1B6-Fc | 1B-1A8-Fc |
|---|---|---|---|---|---|
| **1B-3B11-Fc** | <u>9.83%</u> | 132.28% | 118.99% | 126.72% | 8.16% |

(continued)

| AB# | 1B-3B11-Fc | 1B-1B2-Fc | 1B-1C7-Fc | 1B-1B6-Fc | 1B-1A8-Fc |
|---|---|---|---|---|---|
| 1B-1B2-Fc | 120.47% | 7.44% | 120.97% | 122.01% | 117.16% |
| 1B-1C7-Fc | 91.78% | 87.01% | 0.00% | 97.85% | 91.06% |
| 1B-1B6-Fc | 125.54% | 119.93% | 118.44% | 13.35% | 125.90% |
| 1B-1A8-Fc | 20.31% | 122.78% | 116.44% | 122.38% | 17.76% |

Table 3-2. Epitope difference analysis

| AB# | 1B-3B11-Fc | 1B-1B2-Fc | 1B-1C7-Fc | 1B-1B6-Fc | 3B-1C7-Fc |
|---|---|---|---|---|---|
| 1B-3B11-Fc | 8.85% | 100.96% | 94.39% | 136.09% | 135.70% |
| 1B-1B2-Fc | 126.48% | 0.12% | 91.08% | 120.10% | 7.11% |
| 1B-1C7-Fc | 126.48% | 95.31% | 7.51% | 126.45% | 122.76% |
| 1B-1B6-Fc | 133.73% | 122.60% | 121.83% | 5.41% | 117.22% |
| 3B-1C7-Fc | 137.10% | 12.03% | 141.34% | 161.78% | 13.40% |

Table 3-3. Epitope difference analysis

| AB# | 4&5B-2F01-Fc | 4&5C-12B04-Fc | 1016-069 |
|---|---|---|---|
| 4&5B-2F01-Fc | -4.7% | -3.6% | 0.8% |
| 4&5C-12B04-Fc | 4.4% | -1.0% | 3.0% |
| 1016-069 | -1.9% | 1.5% | 1.2% |

## Example 6. Construction, expression, purification and affinity assay of anti-human c-Met $V_H$H-Fc humanized antibodies

The anti-human c-Met $V_H$H-Fc chimeric antibodies 1B-1B6-Fc, 1B-1C7-Fc, and 4&5C-12B04-Fc were subjected to humanization. The humanized $V_H$H sequences were inserted into a pcDNA3.1(+) eukaryotic expression vector containing a human IgG1 constant region. These $V_H$H-Fc humanized antibodies were expressed using the Expifectamine™ CHO Transfection Kit transient transfection and expression system (Thermo Fisher Scientific Inc., Cat. No. A29129). Among them, the humanization of chimeric antibody 1B-1B6-Fc gave three humanized antibodies 1B-1B6-V1, 1B-1B6-V2, and 1B-1B6-V3 with full-length amino acid sequences set forth in SEQ ID NOs: 51, 53, and 55, respectively, and full-length nucleotide sequences set forth in SEQ ID NOs: 52, 54, and 56, respectively; the humanization of chimeric antibody 1B-1C7-Fc gave two humanized antibodies 1B-1C7-V1 and 1B-1C7-V2 with full-length amino acid sequences set forth in SEQ ID NOs: 57 and 59, respectively, and full-length nucleotide sequences set forth in SEQ ID NOs: 58 and 60, respectively; the humanization of chimeric antibody 4&5C-12B04-Fc gave two humanized $V_H$Hs: 12B04-V1 and 12B04-V2, with amino acid sequences set forth in SEQ ID NOs: 38 and 39, respectively.

Through the surface plasmon resonance technology, the affinity of the acquired humanized antibodies and chimeric antibodies for human c-Met protein was detected on a biomolecular interaction analysis system (GE, Biacore 8K). An anti-hIgG (Fc) antibody (GE, Cat. No. BR-1008-39) was amino-coupled to a CM5 sensor chip. The anti-human c-Met $V_H$H-Fc chimeric antibodies were diluted to 2 $\mu$g/mL with a running buffer (137 mM NaCl, 2.7 mM KCl, 10 mM $Na_2HPO_4 \cdot 12H_2O$, 1.8 mM $KH_2PO_4$, 0.05% surfactant P-20 (w/v), pH 7.4), and the capture was conducted for 90 s by passing the dilutions through experimental channels at a flow rate of 30 $\mu$L/min. Human c-Met-His protein (SinoBiological, Cat. No. 10692-H08H) was diluted with the running buffer to 100 nM, 50 nM, 25 nM, 12.5 nM, 6.25 nM and 3.125 nM, and the association was conducted at a flow rate of 50 $\mu$L/min. The association curve was recorded.

The affinity data of the humanized antibodies (1B-1B6-V1, 1B-1B6-V2, 1B-1B6-V3, 1B-1C7-V1, and 1B-1C7-V2) and the chimeric antibodies (1B-1B6-Fc and 1B-1C7-Fc) for human c-Met protein are shown in Table 4.

Table 4. Binding affinity of anti-human c-Met V$_H$H-Fc chimeric antibodies and humanized antibodies for human c-Met-His

|  | k$_a$(1/Ms) | k$_a$(1/s) | K$_D$(M) |
|---|---|---|---|
| 1B-1C7-Fc | 1.39E+05 | 3.11E-05 | 2.24E-10 |
| 1B-1C7-V1 | 3.68E+05 | 1.30E-02 | 3.54E-08 |
| 1B-1C7-V2 | 3.36E+05 | 1.52E-03 | 4.51E-09 |
| 1B-1B6-Fc | 6.72E+04 | 7.91E-04 | 1.18E-08 |
| 1B-1B6-V1 | 4.83E+05 | 1.11E-02 | 2.31E-08 |
| 1B-1B6-V2 | 1.18E+05 | 2.05E-03 | 1.74E-08 |
| 1B-1B6-V3 | 1.11E+05 | 2.03E-03 | 1.82E-08 |

The assay demonstrated that 1B-1B6-Fc and its humanized antibodies and 1B-1C7-Fc and its humanized antibodies can specifically bind to c-Met-His protein.

**Example 7. Construction, expression, and purification of anti-EGFR/anti-c-Met multispecific antibodies**

The anti-EGFR/anti-c-Met multispecific antibody was prepared by connecting 2 humanized V$_H$H molecules targeting c-Met in series. The multispecific antibody Fc domain was subjected to knob-into-hole engineering (according to the EU numbering, mutations Y349C, T366S, L368A, and Y407V were introduced into one of the Fc polypeptides, and mutations S354C and T366W were introduced into the other Fc polypeptide), and a mutation that hinders the binding to protein A was further introduced into one of the Fc polypeptides of the Fc domain (according to the EU numbering, H435R and Y436F, or H435R).

The anti-EGFR/anti-c-Met multispecific antibodies were constructed according to the configuration shown in FIG. 2, and designated as V12, V41, V35, V42, V67, V73, and V74, respectively, wherein the second antigen-binding moiety was an antigen-binding domain binding to EGFR in a Fab form. Specifically, the anti-EGFR/anti-c-Met multispecific antibody had three polypeptide chains. One polypeptide chain (designated as anti-c-Met-V$_H$H-Fc) contained two antigen-binding domains binding to c-Met connected in series (derived from chimeric antibodies 1B-1B6-Fc, 1B-1C7-Fc, and 4&5C-12B04-Fc, and their humanized antibodies, as shown in Table 5), and for V12, V41, V35, V42, V67, V73, and V74, this polypeptide chain had the amino acid sequences set forth in SEQ ID NOs: 71, 73, 75, 77, 79, 81, and 83, respectively, and nucleotide sequences set forth in SEQ ID NOs: 72, 74, 76, 78, 80, 82, and 84, respectively. The other two polypeptide chains formed an antigen-binding domain binding to EGFR in a Fab form (the variable region sequence binding to EGFR is derived from Patent No. CN100497389C), in which one polypeptide chain was designated as anti-EGFR-HC-Fc (with its amino acid sequence being set forth in SEQ ID NO: 85 and nucleotide sequence set forth in SEQ ID NO: 86), and the other polypeptide chain was designated as anti-EGFR-LC (with its amino acid sequence set forth in SEQ ID NO: 87 and nucleotide sequence set forth in SEQ ID NO: 88). The nucleotide sequences encoding anti-c-Met-V$_H$H-Fc, anti-EGFR-HC-Fc, and anti-EGFR-LC were separately inserted into pcDNA3.1(+) eukaryotic expression vectors to give expression vectors expressing the corresponding polypeptide chains. The expression vectors described above were co-transfected into FUT8 gene-knockout CHO-S cells (designated as CHO FUT8$^{-/-}$ cells) in a transfection ratio of vectors anti-c-Met-V$_H$H-Fc:vector anti-EGFR-HC-Fc:vector anti-EGFR-LC = 1.5:1:1.5 using a CHOgro® high-yield expression system (Cat. No. MIR 6270), with a transfected cell density of $4 \times 10^6$ cells/mL. The culture was continued until day 10 after transfection, and the cell culture supernatant was collected by centrifugation. The protein purification was performed by protein A affinity chromatography, CHT chromatography, and gel chromatography using the ÄKTA pure protein purification system (GE Healthcare). The protein concentration was measured using a UV-Vis spectrophotometer (NanoDrop One C, Thermo Scientific). Electrophoresis (reducing SDS-PAGE), molecular weight (TOF MS), and sequence analysis confirmed that multispecific antibodies with expected structures and sequences were obtained.

Table 5. Sources of components of antigen-binding domains binding to c-Met in anti-EGFR/anti-c-Met multispecific antibodies

| Name | Third antigen-binding moiety | First antigen-binding moiety |
|---|---|---|
| V12 | 1B-1B6 | 1B-1C7 |
| V41 | 1B-1B6-V1 | 1B-1C7-V1 |
| V35 | 1B-1B6-V2 | 1B-1C7-V1 |

(continued)

| Name | Third antigen-binding moiety | First antigen-binding moiety |
|------|------------------------------|------------------------------|
| V42 | 1B-1B6-V3 | 1B-1C7-V1 |
| V67 | 4&5C-12B04 | 1B-1C7-V1 |
| V73 | 12B04-V1 | 1B-1C7-V1 |
| V74 | 12B04-V2 | 1B-1C7-V1 |

## Example 8. Affinity of anti-EGFR/anti-c-Met multispecific antibodies for human c-Met

Referring to the method in Example 6, the binding of chimeric multispecific antibodies V12 and V67 and humanized multispecific antibodies V35, V41, V42, V73, and V74 to human c-Met protein was detected by surface plasmon resonance. The tests showed that as shown in Table 6, all the antibodies can specifically bind to human c-Met-His protein, and the affinity of the humanized multispecific antibodies is comparable to that of the chimeric multispecific antibodies.

Table 6. Binding affinity of anti-EGFR/anti-c-Met multispecific antibodies for human c-Met-His

| | $k_a$(1/Ms) | $k_a$(1/s) | $K_D$(M) |
|------|------------|-----------|----------|
| V12 | 1.42E+05 | 4.62E-05 | 3.25E-10 |
| V41 | 9.70E+04 | 3.57E-05 | 3.68E-10 |
| V35 | 1.05E+05 | 3.34E-05 | 3.18E-10 |
| V42 | 1.00E+05 | 4.39E-05 | 4.38E-10 |
| V67 | 2.54E+05 | 7.01E-05 | 2.76E-10 |
| V73 | 1.97E+05 | 9.03E-05 | 4.58E-10 |
| V74 | 2.34E+05 | 1.28E-04 | 5.48E-10 |

## Example 9. Binding of anti-EGFR/anti-c-Met multispecific antibodies to tumor cells expressing EGFR and c-Met

The binding of the anti-EGFR/anti-c-Met multispecific antibodies (including amivantamab, V42, and V73) to A431 cells expressing EGFR and c-Met (high EGFR expression and low c-Met expression, from Center of Excellence in Molecular Cell Science, CAS), NCI-H1975 cells (medium EGFR expression and low to medium c-Met expression, from BeNa Culture Collection), and NCI-H1993 cells (medium EGFR expression and high c-Met expression, from BeNa Culture Collection) was analyzed by flow cytometry, with an hIgG1 (Biointron, Cat. No. B117901) as the negative control.

A431 cells, NCI-H1975 cells, and NCI-H1993 cells in the logarithmic growth phase were adjusted to viable cell densities of $5 \times 10^6$ to $1 \times 10^7$ cells/mL using an RPMI medium containing 2% of fetal bovine serum (Hyclon, Cat. No. SH30809.01), and seeded in 96-well U-bottom cell culture plates (Costar, Cat. No. 3799) at 50 μL/well. Different concentrations of the anti-EGFR/anti-c-Met multispecific antibodies were prepared using the medium described above by 5-fold serial dilution to obtain 10 concentration gradients with the highest concentration being 500 nM. The antibodies at different concentrations were added to the 96-well cell culture plate described above at 50 μL/well. The mixture was mixed thoroughly and incubated at 4°C for 1 h. The cells were washed with a pre-cooled running buffer (MACS, Cat. No. 130-091-221), and the supernatant was discarded. A pre-cooled fluorescent labeled goat anti-human IgG antibody (Jackson, Cat. No. 109-116-170) was added, and the cells were resuspended at 100 μL/well, mixed thoroughly, and incubated at 4°C for 30 min. After washing, the pre-cooled running buffer was added at 40 μL/well to resuspend the cells. The mixture was mixed thoroughly, and the cells were collected while flowing on the iQue3 flow cytometer. The data were analyzed using software.

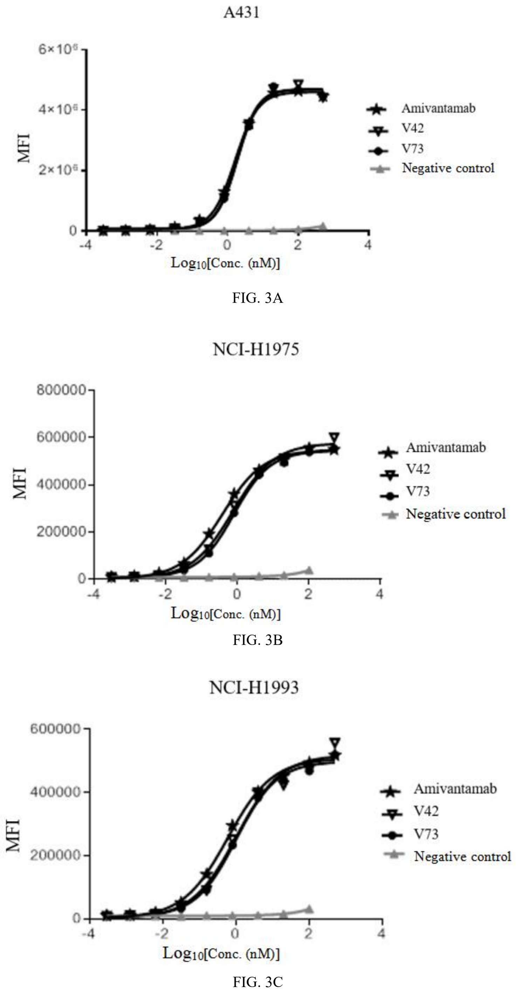

Table 7 and FIGs. 3A-3C show the binding ability of the anti-EGFR/anti-c-Met multispecific antibodies to A431 cells, NCI-H1975 cells, and NCI-H1993 cells. The results showed that the binding ability of V42 and V73 to the three cell lines is substantially comparable to that of amivantamab.

Table 7. The binding $EC_{50}$ values and maximum binding amounts of the anti-EGFR/anti-c-Met multispecific antibodies to EGFR and c-Met on the surface of tumor cells

| | A431 | | NCI-H1975 | | NCI-H1993 | |
|---|---|---|---|---|---|---|
| | $EC_{50}$(nM) | Top(MFI) | $EC_{50}$(nM) | Top(MFI) | $EC_{50}$(nM) | Top(MFI) |
| V42 | 1.852 | 4423651 | 0.755 | 600865 | 1.029 | 557298 |
| V73 | 1.905 | 4467149 | 0.7941 | 554727 | 1.018 | 520132 |
| Amivantamab | 1.62 | 4461530 | 0.3776 | 550125 | 0.5957 | 518725 |
| Negative control | / | 168103 | / | 38741 | / | 31204 |
| "/" denotes that the curve could not give a value by fitting. | | | | | | |

## Example 10. Inhibition of HGF-stimulated c-Met phosphorylation and downstream signaling pathways by anti-EGFR/anti-c-Met multispecific antibodies

The inhibition of HGF stimulation-induced c-Met phosphorylation and downstream signaling pathways by anti-EGFR/anti-c-Met multispecific antibodies (including amivantamab, V42, and V73) was analyzed by Western Blotting, with an hIgG1 (Biointron, Cat. No. B117901) as the negative control.

The coding sequences of HGF-$\alpha$ (with its amino acid sequence set forth in SEQ ID NO: 61) and HGF-$\beta$ (with its amino acid sequence set forth in SEQ ID NO: 62) were separately inserted into pcDNA3.1(+) eukaryotic expression vectors, expressed using the Expifectamine™ CHO Transfection Kit transient transfection and expression system (Thermo Fisher Scientific Inc., Cat. No. A29129), and purified using the Ni Sepharose excel nickel column (GE, Cat. No. 17-3712-01) to give HGF.

A549 cells (medium EGFR expression and medium c-Met expression, non-small cell lung cancer, from Cell Resource Center, Institute of Basic Medical Sciences, Chinese Academy of Medical Sciences) in the logarithmic growth phase were collected, counted, and then adjusted to a viable cell density of $4 \times 10^5$ cells/mL using an RPMI medium (Hyclon, Cat. No. SH30809.01) containing 10% of fetal bovine serum. The cells were added to 6-well cell culture plates at 2 mL/well. The plates were incubated in a cell incubator at 37°C and 5% $CO_2$ for 6 h, and then the medium was discarded. The RPMI medium was added at 1 mL/well, and the cells were starved overnight. The 6-well cell culture plates after overnight starvation were taken out, and the original medium was discarded. The anti-EGFR/anti-c-Met multispecific antibodies at a final concentration of 100 nM, or both HGF at a final concentration of 100 ng/mL and the anti-EGFR/anti-c-Met multispecific antibodies at a final concentration of 100 nM, were added. The plates were incubated in a cell incubator at 37°C and 5% $CO_2$ for 15 min. The cell culture plates were placed on ice and washed with pre-cooled PBS. A lysis buffer containing a protease inhibitor and a phosphatase inhibitor was added for lysis for 30 min. The protein was collected and quantified by the BCA method. The c-Met phosphorylation and downstream signaling pathways were detected by Western Blotting.

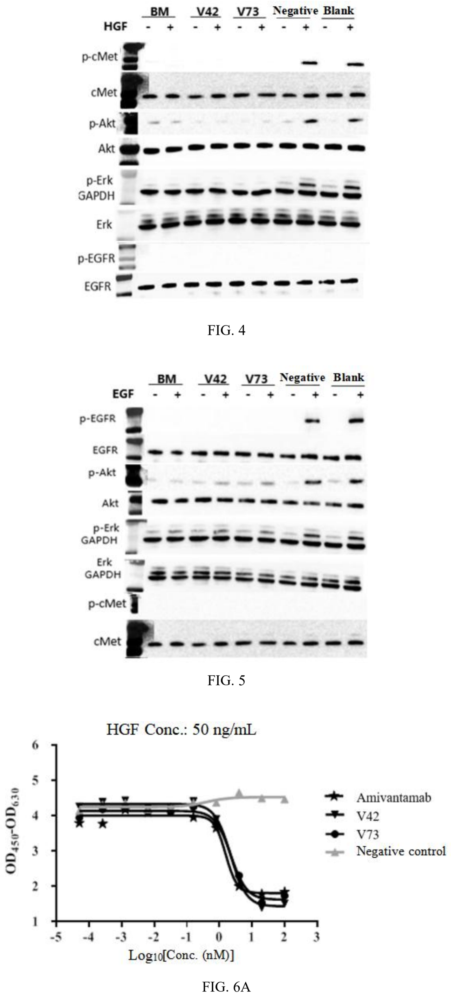

FIG. 4 shows the inhibitory effects of anti-EGFR/anti-c-Met multispecific antibodies on c-Met phosphorylation and downstream signaling pathways. The results demonstrated that the inhibitory effects of V42 and V73 on HGF-stimulated c-Met phosphorylation and downstream signaling pathways are comparable to those of amivantamab (BM in FIG. 4).

## Example 11. Inhibition of EGF-stimulated EGFR phosphorylation and downstream signaling pathways by anti-EGFR/anti-c-Met multispecific antibodies

The inhibition of EGF stimulation-induced EGFR phosphorylation and downstream signaling pathways by anti-EGFR/anti-c-Met multispecific antibodies (including amivantamab, V42, and V73) was analyzed by Western Blotting, with an hIgG1 (Biointron, Cat. No. B117901) as the negative control.

A549 cells in the logarithmic growth phase were collected, counted, and then adjusted to a viable cell density of $4 \times 10^5$ cells/mL using a complete medium. The cells were added to 6-well cell culture plates at 2 mL/well. The plates were incubated in a cell incubator at 37°C and 5% $CO_2$ for 6 h, and then the complete medium was discarded. An RPMI medium (Hyclon, Cat. No. SH30809.01) was added at 1 mL/well, and the cells were starved overnight. The 6-well cell culture plates after overnight starvation were taken out, and the original medium was discarded. The anti-EGFR/anti-c-Met multispecific antibodies at a final concentration of 200 nM, or both EGF (R&D, Cat. No. 236-EG) at a final concentration of 40 ng/mL and the anti-EGFR/anti-c-Met multispecific antibodies at a final concentration of 200 nM, were added. The plates were incubated in a cell incubator at 37°C and 5% $CO_2$ for 15 min. The cell culture plates were placed on ice and washed with pre-cooled PBS. A lysis buffer containing a protease inhibitor and a phosphatase inhibitor was added for lysis for 30 min.

The protein was collected and quantified by the BCA method. The EGFR phosphorylation and downstream signaling pathways were detected by Western Blotting.

FIG. 5 shows the inhibitory effects of anti-EGFR/anti-c-Met multispecific antibodies on EGFR phosphorylation and downstream signaling pathways. The results demonstrated that the inhibitory effects of V42 and V73 on EGF-stimulated EGFR phosphorylation and downstream signaling pathways are comparable to those of amivantamab (BM in FIG. 5).

### Example 12. Activity of anti-EGFR/anti-c-Met multispecific antibodies in competing with HGF ligand for binding to c-Met

The activity of the anti-EGFR/anti-c-Met multispecific antibodies (including amivantamab, V42, and V73) in competing with the HGF ligand for binding to c-Met was analyzed by ELISA, with an hIgG1 (Biointron, Cat. No. B117901) as the negative control.

2.0 mg of biotin (Thermo, Cat. No. 20217) was dissolved in 590 $\mu$L of DMSO (Sigma, Cat. No. D2650). The mixture was mixed thoroughly. The biotin solution was added in a ratio of 2.7 $\mu$L of the biotin solution per 100 $\mu$L of HGF protein (prepared according to Example 10, at a concentration of 2 mg/mL). The mixture was mixed and incubated at room temperature for 40 min to prepare biotin-labeled HGF. A high-adsorption 96-well plate coated with c-Met-His (Corning, Cat. No. 9018) incubated overnight at 4°C was taken out, and the solution in the plate was discarded. The plate was washed 3 times with PBST, blocked with 3% BSA at room temperature for 2 h, and then washed 3 times with PBST. An equal-volume mixture solution of biotin-labeled HGF and anti-EGFR/anti-c-Met multispecific antibodies (the final concentration of the anti-EGFR/anti-c-Met multispecific antibody was 100 nM, and the final concentration of the HGF protein was 50 ng/mL, 10 ng/mL, or 1 ng/mL) was added, and the mixture was incubated at room temperature for 2 h. The plate was washed 3 times with PBST before an HRP-Avidin antibody (Invitrogen, Cat. No. 18-4100-51) was added. The plate was then incubated at room temperature for 1 h. The plate was washed 5 times with PBST, before TMB (Thermo, 00-4201-56) was added. The mixture was incubated at room temperature in the dark for 10 min. A 1 M sulfuric acid stop solution was added, and the mixture was left to stand at room temperature for 5 min. The absorbance values at 450 nm were read on a microplate reader with 630 nm as the reference wavelength.

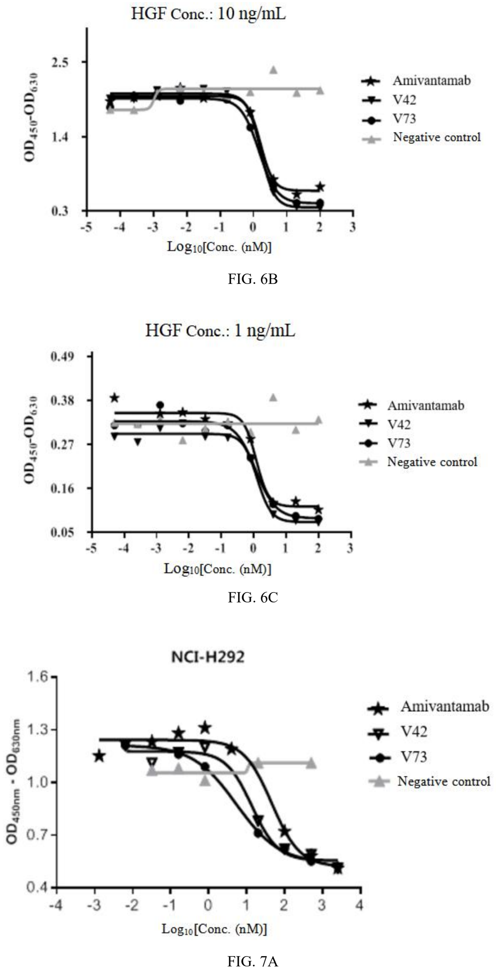

FIGs. 6A-6C show the activity of anti-EGFR/anti-c-Met multispecific antibodies in competing with HGF ligand for binding to c-Met. The results demonstrated that the activity of V42 and V73 in competing with HGF ligand for binding to c-Met is substantially comparable to that of amivantamab.

### Example 13. Inhibitory activity of anti-EGFR/anti-c-Met multispecific antibodies against tumor cell proliferation

The inhibitory activity of the anti-EGFR/anti-c-Met multispecific antibodies (including amivantamab, V42, and V73) against tumor cell proliferation was analyzed on a microplate reader, with an hIgG1 (Biointron, Cat. No. B117901) as the negative control.

NCI-H292 cells (medium EGFR expression and low to medium c-Met expression, from BeNa Culture Collection) and KP4 (medium EGFR expression and low c-Met expression, from Cobioer Biosciences) in the logarithmic growth phase were taken, adjusted to a viable cell density of $1.5 \times 10^4$ to $2 \times 10^4$ cells/mL using an RPMI medium (Hyclon, Cat. No. SH30809.01) containing 2% of fetal bovine serum, and added to a 96-well plate (Costar, Cat. No. 3599) at 100 $\mu$L/well. The anti-EGFR/anti-c-Met multispecific antibodies were prepared using an RPMI medium by 5-fold serial dilution to obtain 10 concentration gradients with the highest concentration being 2500 nM for NCI-H292 cells and by 3-fold serial dilution to obtain 8 concentration gradients with the highest concentration being 555 nM for KP4. The antibodies at different concentrations were added to the 96-well cell culture plate at 100 $\mu$L/well. The plate was incubated in a thermostat incubator at 37°C and 5% $CO_2$. For NCI-H292 cells, 50 $\mu$L of HGF was added to the culture medium at a final concentration of 1 ng/mL, and the plate was incubated for 144 h. For KP4, the plate was incubated for 120 h. A CCK8 working solution was added to the cell culture plate at 20 $\mu$L/well, and the plate was incubated in a thermostat incubator at 37°C and 5% $CO_2$ for 2 h. The absorbance values at 450 nm were read on a microplate reader with 630 nm as the reference wavelength.

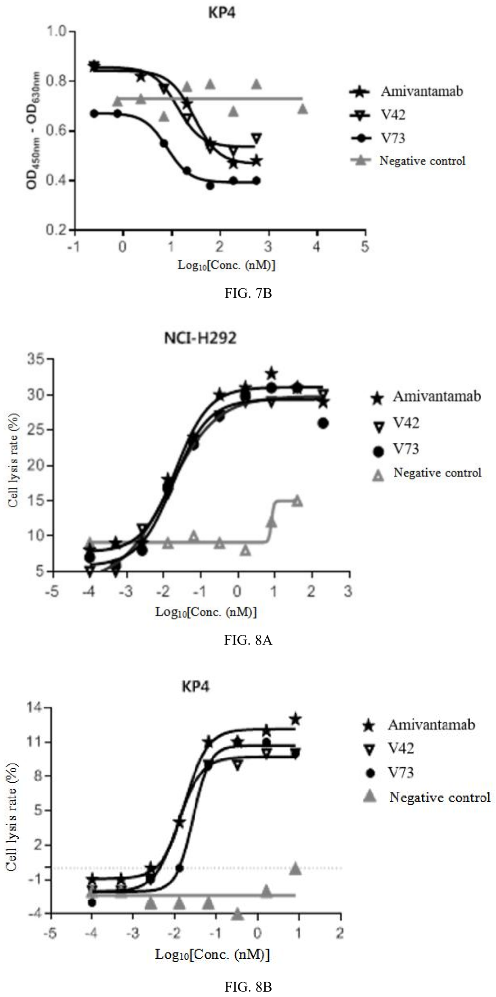

FIGs. 7A and 7B and Table 8 show the inhibitory activity of anti-EGFR/anti-c-Met multispecific antibodies against tumor cell proliferation. The results demonstrated that in NCI-H292 cells and KP4 cells, V73 and V42 possess superior inhibitory activity against tumor cell proliferation to amivantamab.

Table 8. $IC_{50}$ values for inhibiting the proliferation of NCI-H292 and KP4 cells by the antibodies

|  | NCI-H292 | KP4 |
|---|---|---|
|  | $IC_{50}$(nM) | $IC_{50}$(nM) |
| V42 | 13.72 | 13.09 |

(continued)

| | NCI-H292 | KP4 |
|---|---|---|
| | IC$_{50}$(nM) | IC$_{50}$(nM) |
| V73 | 5.356 | 7.985 |
| Amivantamab | 45.78 | 28.96 |

## Example 14. ADCC effect of anti-EGFR/anti-c-Met multispecific antibodies on tumor cells

The antibody-dependent cell-mediated cytotoxicity (ADCC) effects of the anti-EGFR/anti-c-Met multispecific antibodies (including amivantamab, V42, and V73) on tumor cells were explored using human PBMCs (peripheral blood mononuclear cells), with an hIgG1 (Biointron, Cat. No. B117901) as the negative control.

NCI-H292 and KP4 cells in the logarithmic growth phase were taken, and adjusted to a viable cell density of 3 × 10$^5$ cells/mL with an RPMI medium (Hyclon, Cat. No. SH30809.01) containing 2% of fetal bovine serum to give target cells. The anti-EGFR/anti-c-Met multispecific antibodies were prepared in an RPMI medium by 5-fold serial dilution to obtain 8-10 concentration gradients with the highest concentration ranging from 8 to 200 nM. PBMC cells were thawed, counted, and adjusted to a PBMC viable cell density of 1.5 ×10$^6$ cells/mL with an RPMI medium to give effector cells. An administration group (50 μL of target cells + 100 μL of effector cells + 50 μL of antibody), a target cell group (50 μL of target cells + 150 μL of culture medium), an effector cell group (100 μL of effector cells + 100 μL of culture medium), a target + effector cell group (50 μL of target cells + 100 μL of effector cells + 50 μL of culture medium), a blank control group (200 μL of culture medium), a lysis buffer control group (200 μL of culture medium + 20 μL of lysis buffer), and a target cell maximum release group (50 μL of target cells + 150 μL of culture medium + 20 μL of lysis buffer) were set. The groups were added to a 96-well plate (Costar, Cat. No. 3599) in an effector-to-target cell ratio of 10:1. The cells were incubated in a thermostat incubator at 37°C and 5% CO$_2$ for 24 h. The cell lysis rates were measured using a CytoTox96® nonradioactive cytotoxicity assay kit (Promega, Cat. No. G1780). Finally, the absorbance at 490 nm was measured on a microplate reader.

$$\text{Cell lysis rate}(\%) = \frac{\text{OD}_{\text{Administration group}} - \text{OD}_{\text{Target+effector cell group}}}{\text{OD}_{\text{Target cell maximum release group}} - \text{OD}_{\text{Target cell group}}} \times 100\%$$

FIGs. 8A and 8B and Table 9 show the ADCC effects of anti-EGFR/anti-c-Met multispecific antibodies on tumor cells.

Table 9. Lysis EC$_{50}$ value and maximum lysis rate of the antibodies to NCI-H292 and KP4 cells

| | NCI-H292 | | KP4 | |
|---|---|---|---|---|
| | EC$_{50}$(nM) | Maximum lysis rate | EC$_{50}$(nM) | Maximum lysis rate |
| V42 | 0.01346 | 30% | 0.01166 | 11% |
| V73 | 0.01813 | 31% | 0.02699 | 11% |
| Amivantamab | 0.02105 | 33% | 0.01708 | 13% |

## Example 15. Endocytic activity of anti-EGFR/anti-c-Met multispecific antibodies against tumor cells

The endocytic activity of the anti-EGFR/anti-c-Met multispecific antibodies (including amivantamab, V42, and V73) to A431 cells expressing EGFR and c-Met, NCI-H1975 cells, and NCI-H441 cells (medium EGFR expression and medium c-Met expression, from BeNa Culture Collection) was analyzed by flow cytometry, with an hIgG1 (Biointron, Cat. No. B117901) as the negative control.

A431 cells, NCI-H1975 cells, and NCI-H441 cells in the logarithmic growth phase were separately adjusted to a viable cell density of 2 × 10$^6$ cells/mL using a complete medium, and seeded in 96-well V-bottom cell culture plates (Costar, Cat. No. 3894) at 20 μL/well. A complex of the anti-EGFR/anti-c-Met multispecific antibody and the Antibody Internalization Human Reagent (Sartorius, Cat. No. 90564) was prepared according to the instructions of the Antibody Internalization Human Reagent, such that the final concentration of the anti-EGFR/anti-c-Met multispecific antibody was 100-167 nM. The mixture was incubated at 37°C for 15 min, and 5-fold serially diluted to give a total of 10 concentration gradients. The complex solution was added to the cell culture plate at 20 μL/well. The mixture was mixed thoroughly and then incubated in a thermostat incubator at 37°C and 5% CO$_2$ for 2 h. The changes in fluorescence values were detected in the RL1 channel of the iQue3 flow cytometer, and the data were analyzed using software.

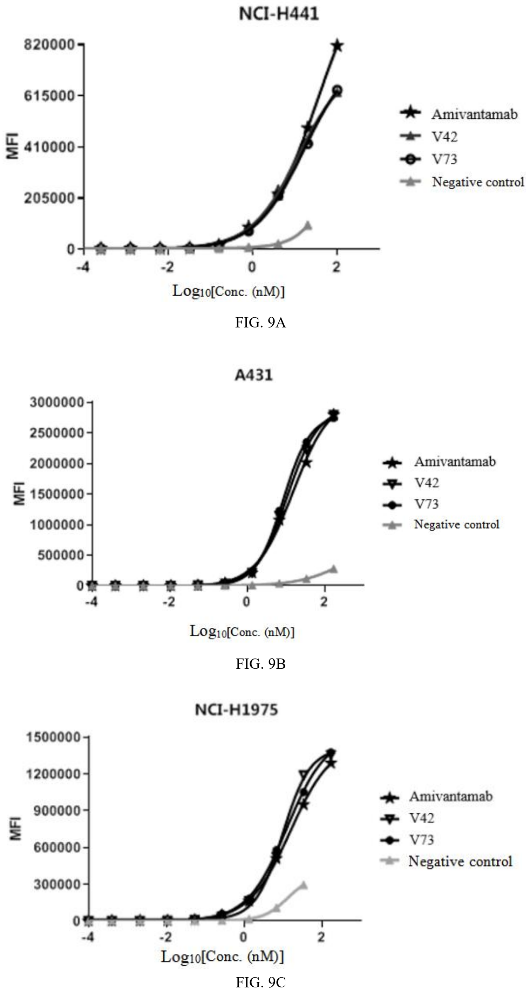

FIGs. 9A-9C and Table 10 show the endocytic activity of the anti-EGFR/anti-c-Met multispecific antibodies to A431 cells, NCI-H1975 cells, and NCI-H441 cells.

Table 10. Endocytic activity of anti-EGFR/anti-c-Met multispecific antibodies to A431 cells, NCI-H1975 cells, and NCI-H441 cells

| | NCI-H441 | | A431 | | NCI-H1975 | |
|---|---|---|---|---|---|---|
| | $EC_{50}$(nM) | Top(MFI) | $EC_{50}$(nM) | Top(MFI) | $EC_{50}$(nM) | Top(MFI) |
| V42 | 11.81 | 629221 | 10.54 | 2797694 | 9.662 | 1356725 |
| V73 | 16.72 | 638113.5 | 8.333 | 2745828 | 12.43 | 1376844 |
| Amivantamab | 35.63 | 815804 | 15.06 | 2799466.5 | 15.16 | 1290047 |
| Negative control | / | 97506 | / | 117841.5 | / | 110471 |

**Example 16. Pharmacodynamic evaluation of anti-EGFR/anti-c-Met Multispecific antibodies in U-87 MG human neuroglioma nude mouse subcutaneous xenograft tumor model**

SPF-grade female BALB/c-nu nude mice (purchased from Beijing HFK Bioscience Co., Ltd.) were subcutaneously grafted with U-87 MG cells (ATCC HTB-14™). After the tumor grew to 100-150 mm³, the mice were divided into a modeling group (normal saline), V42 groups, and V73 groups, with 8 mice in each group.

The day of the first dose was day 0 (D0). The drug (0.25 mg/kg or 0.75 mg/kg) was administered intravenously (i.v.) twice a week for a total of 6 doses at an injection volume of 0.1 mL/10 g mouse body weight. The tumors were measured twice weekly with a vernier caliper. The pharmacodynamic evaluation was conducted as per the tumor growth inhibition (TGI).

The measurement indexes and the calculation formulas are as follows:

$$\text{Tumor volume (mm}^3) = 1/2 \times (a \times b^2)$$

(where a represents the long diameter and b represents the short diameter of the tumor).

$$\text{Relative tumor proliferation rate T/C (\%)} = (T - T_0)/(C - C_0) \times 100\%,$$

TGI (%) = 100% - T/C; where T and C are the tumor volumes of the treatment and modeling groups, respectively, at the end of the study; $T_0$ and $C_0$ are the tumor volumes of the treatment and modeling groups, respectively, at the start of the study.

If the tumor volume was smaller than the tumor volume at the start of the study, i.e., $T < T_0$ or $C < C_0$, it was defined as partial regression (PR) of the tumor. When the tumor regressed, TGI (%) = 100 - (T - To)/To × 100%.

The results of the index measurement are shown in Table 11. In the U-87 MG human neuroglioma nude mouse subcutaneous xenograft tumor model, V42 and V73 both exhibited significant anti-tumor effects.

Table 11. Effect of anti-EGFR/anti-c-Met multispecific antibodies on U-87 MG human neuroglioma subcutaneous xenograft tumors in nude mice

| Group | Tumor volume on D0 (mm³) (Mean ± SEM) | Tumor volume on D20 (mm³) (Mean ± SEM) | D20 T/C | D20 TGI | P value |
|---|---|---|---|---|---|
| Modeling group | 115.4±1.7 | 2014±234.4 | - | - | - |
| V42 group (0.25 mg/kg) | 115.7±1.5 | 1352.6±228.4 | 65% | 35% | 0.062 |
| V42 group (0.75 mg/kg) | 115.8±1.4 | 210.9±38.4 | 5% | 95% | <0.001 |
| V73 group (0.25 mg/kg) | 116.1±1.5 | 1774.3±134.4 | 87% | 13% | 0.388 |
| V73 group (0.75 mg/kg) | 116.6±1.2 | 207.7±69 | 5% | 95% | <0.001 |

P value is determined by comparison with the modeling group.

The sequence information of the present disclosure is summarized in Table S4 below.

Table S4. Sequence information

| Description / Sequence (SEQ ID NO:) |
|---|
| CDR1 of 1B-3B11<br>SVA (SEQ ID NO: 1) |
| CDR2 of 1B-3B11<br>VINPGGDNTDYTYAVKG (SEQ ID NO: 2) |
| CDR3 of 1B-3B11<br>GRTFGGTWMDRNKWKY (SEQ ID NO: 3) |
| CDR1 of 1B-1B6, 1B-1B6-V1, 1B-1B6-V2, and 1B-1B6-V3<br>SGCMG (SEQ ID NO: 4) |
| CDR2 of 1B-1B6, 1B-1B6-V1, 1B-1B6-V2, and 1B-1B6-V3<br>TIYTRNDFTYYSDX$_1$VKG (SEQ ID NO: 5, wherein X$_1$ is selected from S and T) |
| CDR2 of 1B-1B6, 1B-1B6-V1, and 1B-1B6-V2<br>TIYTRNDFTYYSDX$_1$VKG (SEQ ID NO: 5, X$_1$ is S), i.e.,<br>TIYTRNDFTYYSDSVKG |
| CDR2 of 1B-1B6-V3<br>TIYTRNDFTYYSDX$_1$VKG (SEQ ID NO: 5, X$_1$ is T), i.e.,<br>TIYTRNDFTYYSDTVKG |
| CDR3 of 1B-1B6, 1B-1B6-V1, 1B-1B6-V2, and 1B-1B6-V3<br>GSPFRYAGAWNVCGLKEEDYNY (SEQ ID NO: 6) |
| CDR1 of 1B-1C7, 1B-1C7-V1, and 1B-1C7-V2<br>ANCVG (SEQ ID NO: 7) |
| CDR2 of 1B-1C7, 1B-1C7-V1, and 1B-1C7-V2<br>AINTGGETATYADFVKG (SEQ ID NO: 8) |
| CDR3 of 1B-1C7, 1B-1C7-V1, and 1B-1C7-V2<br>VGTRKYWDECSLSQHAYKN (SEQ ID NO: 9) |
| CDR1 of 1B-1A8<br>SYAMD (SEQ ID NO: 10) |
| CDR2 of 1B-1A8<br>GINSAGSPSYEASVKG (SEQ ID NO: 11) |
| CDR3 of 1B-1A8<br>CRDFRSPYCR (SEQ ID NO: 12) |
| CDR1 of 1B-1B2<br>MG (SEQ ID NO: 13) |
| CDR2 of 1B-1B2<br>SISSDGNSDYLDSVKG (SEQ ID NO: 14) |
| CDR3 of 1B-1B2<br>NGAGHLVLRPLCGSNAVDA (SEQ ID NO: 15) |
| CDR1 of 3B-1C7<br>GTTMG (SEQ ID NO: 16) |
| CDR2 of 3B-1C7<br>DISSDGNTYYSDSVKG (SEQ ID NO: 17) |
| CDR3 of 3B-1C7<br>AEFRLLLRPLCRQDDYGY (SEQ ID NO: 18) |
| CDR1 of 4&5B-2F01<br>YKYLA (SEQ ID NO: 19) |

(continued)

| Description<br>Sequence (SEQ ID NO:) |
|---|
| CDR2 of 4&5B-2F01<br>GLNTGGGSAYYADSVKG (SEQ ID NO: 20) |
| CDR3 of 4&5B-2F01<br>RRDYIHMTNIQALGLRPQWFSD (SEQ ID NO: 21) |
| CDR1 of 4&5C-12B04, 12B04-V1, and 12B04-V2<br>SNFMA (SEQ ID NO: 22) |
| CDR2 of 4&5C-12B04, 12B04-V1, and 12B04-V2<br>TITTGAGNTAYADSVKG (SEQ ID NO: 23) |
| CDR3 of 4&5C-12B04, 12B04-V1, and 12B04-V2<br>GRGGFWRPAEYDF (SEQ ID NO: 24) |
| $V_HH$ of 1B-3B11<br>QVQLVESGGGSVQAGGSLRLSCVVSGYTYSSVAWFRQTPGKEREGVAVINPGGDNTDYTYAVKGR FTISRDSAKSTVFLQMNNLESEDTAMYYCAAGRTFGGTWMDRNKWKYWGQGTLVTVSS (SEQ ID NO: 25) |
| $V_HH$ of 1B-1B6<br>QVQLVESGGDSVQAGGSLRLSCAASAHAYSSGCMGWFRQAPGKGREGVATIYTRNDFTYYSDSVK GRFTISRDDAKNTVYLQVNSLKAEDTAMYYCAAGSPFRYAGAWNVCGLKEEDYNYWGQGTLVT VSS (SEQ ID NO: 26) |
| $V_HH$ of 1B-1C7<br>QVQLVESGGGLVQPGGSLRLSCAASGYSYSANCVGWFYQAPGKEREGVAAINTGGETATYADFVK GRFTISQDYAKNTVYLQMNSLKSEDTGMYYCAAVGTRKYWDECSLSQHAYKNWGQGTLVTVSS (SEQ ID NO: 27) |
| $V_HH$ of 1B-1A8<br>EVQLVESGGGLVQPGGSLRLSCVASGFTFKSYAMDWVRQAPGKGLEWIAGINSAGSPSYEASVKG RFTISRDNAENTLYLQLNSLKTEDTAMYYCTKCRDFRSPYCRPGQGTLVTVSS (SEQ ID NO: 28) |
| $V_HH$ of 1B-1B2<br>EVQLVESGGGSVQAGGSLRLSCTASGFTFDMGWYRQAPGDECELVSSISSDGNSDYLDSVKGRFTI SQDNAKNTVYLQMNSLKPEDTAVYYCAANGAGHLVLRPLCGSNAVDAWGQGTLVTVSS (SEQ ID NO: 29) |
| $V_HH$ of 3B-1C7<br>QVQLVESGGDSVQAGGSLRLSCIASGFTFDGTTMGWYRQAPGDECELVSDISSDGNTYYSDSVKG RFTISQGNAKNTVYLQMNSLTPEDTAVYYCAAAEFRLLLRPLCRQDDYGYWGQGTLVTVSS (SEQ ID NO: 30) |
| $V_HH$ of 4&5B-2F01<br>QVQLQESGGGLVQPGGSLRLSCAASGTTGDYKYLAWFRQGPGKEREGIAGLNTGGGSAYYADSVK GRFTISQDNVKRLLYLQLNNLKPEDTAMYYCAARRDYIHMTNIQALGLRPQWFSDWGQGTQVTV SS (SEQ ID NO: 31) |
| $V_HH$ of 4&5C-12B04<br>EVQLVESGGGSVQAQGSLRLSCTVSGSTYSSNFMAWFRQAPGKEREWVATITTGAGNTAYADSVK GRFTISEDNAKRTAYLQMNSLKPEDTAVYYCAAGRGGFWRPAEYDFWGQGTQVTVSS (SEQ ID NO: 32) |
| $V_HH$ of 1B-1B6-V1 |

(continued)

| Description |
|---|
| Sequence (SEQ ID NO:) |
| QVQLLESGGGSVQAGGSLRLSCAASGFTFSSGCMGWFRQAPGKGREGVATIYTRNDFTYYSDSVK GRFTISRDNSKNTVYLQMNSLRAEDTAVYYCAAGSPFRYAGAWNVCGLKEEDYNYWGQGTLVTV SS (SEQ ID NO: 33) |
| V$_H$H of 1B-1B6-V2<br><br>QVQLLESGGGSVQAGGSLRLSCAASGHTYSSGCMGWFRQAPGKGREGVATIYTRNDFTYYSDSVK GRFTISRDNSKNTVYLQMNSLRAEDTAVYYCAAGSPFRYAGAWNVCGLKEEDYNYWGQGTLVTV SS (SEQ ID NO: 34) |
| V$_H$H of 1B-1B6-V3<br><br>QVQLLESGGGSVQAGGSLRLSCAASGHTYSSGCMGWFRQAPGKGREGVATIYTRNDFTYYSDTVK GRFTISRDNSKNTVYLQMNSLRAEDTAVYYCAAGSPFRYAGAWNVCGLKEEDYNYWGQGTLVTV SS (SEQ ID NO: 35) |
| V$_H$H of 1B-1C7-V1<br><br>QVQLVESGGGSVQPGGSLRLSCAASGYSYSANCVGWFRQAPGKGREGVSAINTGGETATYADFVK GRFTISRDNAKNTVYLQMNSLRAEDTAVYYCAAVGTRKYWDECSLSQHAYKNWGQGTLVTVSS (SEQ ID NO: 36) |
| V$_H$H of 1B-1C7-V2<br><br>QVQLVESGGGSVQPGGSLRLSCAASGFTFSANCVGWFRQAPGKGREGVSAINTGGETATYADFVK GRFTISRDNAKNTVYLQMNSLRAEDTAVYYCAAVGTRKYWDECSLSQHAYKNWGQGTLVTVSS (SEQ ID NO: 37) |
| 12B04-V1<br><br>EVQLVESGGGSVQAGGSLRLSCAVSGFTVSSNFMAWFRQAPGKGREWVSTITTGAGNTAYADSVK GRFTISEDNSKNTLYLQMNSLRAEDTAVYYCAAGRGGFWRPAEYDFWGQGTTVTVSS(SEQ ID NO: 38) |
| 12B04-V2<br><br>EVQLVESGGGSVQAGGSLRLSCAVSGFTVSSNFMAWFRQAPGKGREWVSTITTGAGNTAYADSVK GRFTISEDNSKNTAYLQMNSLRAEDTAVYYCAAGRGGFWRPAEYDFWGQGTTVTVSS(SEQ ID NO: 39) |
| V$_H$H of camel and humanized 1B-1B6<br><br>QVQLX$_2$ESGGX$_3$SVQAGGSLRLSCAASX$_4$X$_5$X$_6$X$_7$SSGCMGWFRQAPGKGREGVATIYTRNDFTYYS DX$_8$VKGRFTISRDX$_9$X$_{10}$KNTVYLQX$_{11}$NSLX$_{12}$AEDTAX$_{13}$YYCAAGSPFRYAGAWNVCGLKEEDYNY WGQGTLVTVSS (SEQ ID NO: 40, X$_2$ is selected from V and L, X$_3$ is selected from D and G, X$_4$ is selected from A and G, X$_5$ is selected from H and F, X$_6$ is selected from A and T, X$_7$ is selected from Y and F, X$_8$ is selected from S and T, X$_9$ is selected from D and N, X$_{10}$ is selected from A and S, X$_{11}$ is selected from V and M, X$_{12}$ is selected from K and R, X$_{13}$ is selected from M and V) |
| V$_H$H of camel and humanized 1B-1C7<br><br>QVQLVESGGGX$_{14}$VQPGGSLRLSCAASGX$_{15}$X$_{16}$X$_{17}$SANCVGWFX$_{18}$QAPGKX$_{19}$REGVX$_{20}$AINTGGET ATYADFVKGRFTISX$_{21}$DX$_{22}$AKNTVYLQMNSLX$_{23}$X$_{24}$EDTX$_{25}$X$_{26}$YYCAAVGTRKYWDECSLSQHAY KNWGQGTLVTVSS (SEQ ID NO: 41, X$_{14}$ is selected from L and S, X$_{15}$ is selected from Y and F, X$_{16}$ is selected from S and T, X$_{17}$ is selected from Y and F, X$_{18}$ is selected from Y and R, X$_{19}$ is selected from E and G, X$_{20}$ is selected from A and S, X$_{21}$ is selected from Q and R, X$_{22}$ is selected from Y and N, X$_{23}$ is selected from K and R, X$_{24}$ is selected from S and A, X$_{25}$ is selected from G and A, X$_{26}$ is selected from M and V) |
| V$_H$H of camel and humanized 4&5C-12B04 |

(continued)

| Description |
| --- |
| Sequence (SEQ ID NO:) |
| EVQLVESGGGSVQAX$_{27}$GSLRLSCX$_{28}$VSGX$_{29}$TX$_{30}$SSNFMAWFRQAPGKX$_{31}$REWVX$_{32}$TITTGAGNT AYADSVKGRFTISEDNX$_{33}$KX$_{34}$TX$_{35}$YLQMNSLX$_{36}$X$_{37}$EDTAVYYCAAGRGGFWRPAEYDFWGQGTX$_{38}$VTVSS (SEQ ID NO: 42, X$_{27}$ is selected from Q and G, X$_{28}$ is selected from T and A, X$_{29}$ is selected from S and F, X$_{30}$ is selected from Y and V, X$_{31}$ is selected from E and G, X$_{32}$ is selected from A and S, X$_{33}$ is |
| selected from A and S, X$_{34}$ is selected from R and N, X$_{35}$ is selected from A and L, X$_{36}$ is selected from K and R, X$_{37}$ is selected from P and A, X$_{38}$ is selected from Q and T) |
| 1B-3B11-Fc |
| QVQLVESGGGSVQAGGSLRLSCVVSGYTYSSVAWFRQTPGKEREGVAVINPGGDNTDYTYAVKGR FTISRDSAKSTVFLQMNNLESEDTAMYYCAAGRTFGGTWMDRNKWKYWGQGTLVTVSSEPKIPQ PQPKPQPQPQPQPKPQPKPEPECTCPKCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK(SEQ ID NO: 43) |
| 1B-1B6-Fc |
| QVQLVESGGDSVQAGGSLRLSCAASAHAYSSGCMGWFRQAPGKGREGVATIYTRNDFTYYSDSVK GRFTISRDDAKNTVYLQVNSLKAEDTAMYYCAAGSPFRYAGAWNVCGLKEEDYNYWGQGTLVT VSSEPKIPQPQPKPQPQPQPQPKPQPKPEPECTCPKCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKSLSLSPGK(SEQ ID NO: 44) |
| 1B-1C7-Fc |
| QVQLVESGGGLVQPGGSLRLSCAASGYSYSANCVGWFYQAPGKEREGVAAINTGGETATYADFVK GRFTISQDYAKNTVYLQMNSLKSEDTGMYYCAAVGTRKYWDECSLSQHAYKNWGQGTLVTVSSE PKIPQPQPKPQPQPQPQPKPQPKPEPECTCPKCPAPELLGGPSVFLFPPKPKDTLMISRTPEVTCVVVD VSHEDPEVKFNWYVDGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK(SEQ ID NO: 45) |
| 1B-1A8-Fc |
| EVQLVESGGGLVQPGGSLRLSCVASGFTFKSYAMDWVRQAPGKGLEWIAGINSAGSPSYEASVKG RFTISRDNAENTLYLQLNSLKTEDTAMYYCTKCRDFRSPYCRPGQGTLVTVSSEPKIPQPQPKPQPQ PQPQPKPQPKPEPECTCPKCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK(SEQ ID NO: 46) |
| 1B-1B2-Fc |
| EVQLVESGGGSVQAGGSLRLSCTASGFTFDMGWYRQAPGDECELVSSISSDGNSDYLDSVKGRFTI SQDNAKNTVYLQMNSLKPEDTAVYYCAANGAGHLVLRPLCGSNAVDAWGQGTLVTVSSEPKIPQP QPKPQPQPQPQPKPQPKPEPECTCPKCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPIEK TISKAKGQPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK(SEQ ID NO: 47) |
| 3B-1C7-Fc |

(continued)

| Description |
| --- |
| Sequence (SEQ ID NO:) |
| QVQLVESGGDSVQAGGSLRLSCIASGFTFDGTTMGWYRQAPGDECELVSDISSDGNTYYSDSVKG RFTISQGNAKNTVYLQMNSLTPEDTAVYYCAAAEFRLLLRPLCRQDDYGYWGQGTLVTVSSEPKIP QPQPKPQPQPQPQPKPQPKPEPECTCPKCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPI EKTISKAKGQPREPQVYTLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK (SEQ ID NO: 48) |
| 4&5B-2F01-Fc<br><br>QVQLQESGGGLVQPGGSLRLSCAASGTTGDYKYLAWFRQGPGKEREGIAGLNTGGGSAYYADSVK GRFTISQDNVKRLLYLQLNNLKPEDTAMYYCAARRDYIHMTNIQALGLRPQWFSDWGQGTQVTV SSEPKSSDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK(SEQ ID NO: 49) |
| 4&5C-12B04-Fc<br><br>EVQLVESGGGSVQAGGSLRLSCTVSGSTYSSNFMAWFRQAPGKEREWVATITTGAGNTAYADSVK GRFTISEDNAKRTAYLQMNSLKPEDTAVYYCAAGRGGFWRPAEYDFWGQGTQVTVSSEPKSSDKT HTCPPCPAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVYTLPPSR |
| DELTKNQVSLTCLVKGFYPSDIAVEWESNGQPENNYKTTPPVLDSDGSFFLYSKLTVDKSRWQQGN VFSCSVMHEALHNHYTQKSLSLSPGK(SEQ ID NO: 50) |
| 1B-1B6-V1<br><br>QVQLLESGGGSVQAGGSLRLSCAASGFTFSSGCMGWFRQAPGKGREGVATIYTRNDFTYYSDSVK GRFTISRDNSKNTVYLQMNSLRAEDTAVYYCAAGSPFRYAGAWNVCGLKEEDYNYWGQGTLVTV SSGEPKSSDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK(SEQ ID NO: 51)<br>caggtgcagctgctggagagcggcggcggaagcgtgcaggctggaggatccctgcggctgagctgtgctgcctccggcttcaccttcagctccggctgc atgggctggttcaggcaggcccctggcaagggccgggagggagttgctaccatctacacccggaatgacttcacctattactccgacagcgtgaagggca ggttcaccatcagccgggacaactccaagaacaccgtgtatctgcagatgaatagcctgcgggccgaggacaccgccgtgtactattgcgctgccggcag cccccttcaggtacgctggcgcttggaatgtgtgtggcctgaaggaggaggattacaattattggggccagggcaccctggtgaccgtgagcagcggagag ccaaagtccagcgataagacccacacctgccccccccttgccctgctcccgagctgctggggaggccctagcgtgttcctgttcccccctaagcctaaggacac cctgatgatcagccggacccccgaggtgacctgtgtggtggtggatgtgagccacgaggatcctgaggtgaagttcaactggtatgtggacggcgtggag gtgcacaacgccaagaccaagcctagggaggagcagtataactccacctataggtggtgagcgtgctgaccgtgctgcaccaggattggctgaatggca aggagtacaagtgcaaggtgagcaataaggctctgcctgctcctatcgagaagaccatctccaaggctaagggccagcccagggagcctcaggtgtacac cctgcctcccagcagggacgagctgaccaagaatcaggtgagcctgacctgcctggtgaagggcttctacccctccgatatcgctgtggagtgggagagc aacggccagcctgagaacaactacaagaccacccccccgtgctggattccgatggctccttcttcctgtacagcaagctgaccgtggataagagccggtg gcagcagggcaacgtgttcagctgctccgtgatgcacgaggccctgcacaaccactacacccagaagagcctgagcctgagccctggcaag(SEQ ID NO: 52) |
| 1B-1B6-V2 |

(continued)

| Description<br>Sequence (SEQ ID NO:) |
|---|
| QVQLLESGGGSVQAGGSLRLSCAASGHTYSSGCMGWFRQAPGKGREGVATIYTRNDFTYYSDSVK<br>GRFTISRDNSKNTVYLQMNSLRAEDTAVYYCAAGSPFRYAGAWNVCGLKEEDYNYWGQGTLVTV<br>SSGEPKSSDKTHTCPPCPAPELLGGPSVFLFPPKPKDTLMISRTPEVTCVVVDVSHEDPEVKFNWYV<br>DGVEVHNAKTKPREEQYNSTYRVVSVLTVLHQDWLNGKEYKCKVSNKALPAPIEKTISKAKGQPR<br>EPQVYTLPPSRDELTKNQVSLTCLVKGFYPSDIAVEWESNGQPENNYKTTPPVLDSDGSFFLYSKLT<br>VDKSRWQQGNVFSCSVMHEALHNHYTQKSLSLSPGK(SEQ ID NO: 53)<br>caggtgcagctgctggagtccggcggcggatccgtgcaggctggaggatccctgcggctgagctgcgctgcttccggccacacctactcctccggctgca<br>tgggctggttcaggcaggctcctggcaagggccgggagggagtggctaccatctataccaggaacgacttcacctactattccgattccgtgaagggcag<br>gttcaccatcagcagggataactccaagaacaccgtgtatctgcagatgaatagcctgcgggccgaggataccgccgtgtactactgcgccgccggcagc<br>cccttccggtacgctggagcttggaacgtgtgcggcctgaaggaggaggactacaactattggggccagggcaccctggtgaccgtgagctccggagag<br>ccaaagtccagcgataagacccacacctgcccccccttgccctgctcccgagctgctgggaggccctagcgtgttcctgttcccccctaagcctaaggacac<br>cctgatgatcagccggaccccccgaggtgacctgtgtggtggtggatgtgagccacgaggatcctgaggtgaagttcaactggtatgtggacggcgtggag<br>gtgcacaacgccaagaccaagcctagggaggagcagtataactccacctataggtggtgagcgtgctgaccgtgctgcaccaggattggctgaatggca<br>aggagtacaagtgcaaggtgagcaataaggctctgcctgctcctatcgagaagaccatctccaaggctaagggccagcccagggagcctcaggtgtacac<br>cctgcctcccagcagggacgagctgaccaagaatcaggtgagcctgacctgcctggtgaagggcttctacccctccgatatcgctgtggagtgggagagc<br>aacggccagcctgagaacaactacaagaccacccccccgtgctggattccgatggctccttcttcctgtacagcaagctgaccgtggataagagccggtg<br>gcagcagggcaacgtgttcagctgctccgtgatgcacgaggccctgcacaaccactacacccagaagagcctgagcctgagccctggcaag(SEQ<br>ID NO: 54) |
| 1B-1B6-V3<br><br>QVQLLESGGGSVQAGGSLRLSCAASGHTYSSGCMGWFRQAPGKGREGVATIYTRNDFTYYSDTVK<br>GRFTISRDNSKNTVYLQMNSLRAEDTAVYYCAAGSPFRYAGAWNVCGLKEEDYNYWGQGTLVTV<br>SSGEPKSSDKTHTCPPCPAPELLGGPSVFLFPPKPKDTLMISRTPEVTCVVVDVSHEDPEVKFNWYV<br>DGVEVHNAKTKPREEQYNSTYRVVSVLTVLHQDWLNGKEYKCKVSNKALPAPIEKTISKAKGQPR<br>EPQVYTLPPSRDELTKNQVSLTCLVKGFYPSDIAVEWESNGQPENNYKTTPPVLDSDGSFFLYSKLT<br>VDKSRWQQGNVFSCSVMHEALHNHYTQKSLSLSPGK(SEQ ID NO: 55)<br>caggtgcagctgctggagagcggcggcggaagcgtgcaggctggaggaagcctgaggctgagctgcgccgccagcggacacacctacagctccggc<br>tgtatgggctggttcaggcaggcccccggcaagggacgggagggagttgctaccatctacacccggaatgacttcacctactattccgataccgtgaaggg<br>caggttcaccatcagcagggacaactccaagaataccgtgtacctgcagatgaactccctgcgggccgaggacaccgccgtgtattactgtgctgctggct<br>ccccccttcaggtatgccggcgcttggaatgtgtgcggcctgaaggaggaggactataattattggggccagggcaccctggtgaccgtgagcagcggaga<br>gccaaagtccagcgataagacccacacctgcccccccttgccctgctcccgagctgctgggaggccctagcgtgttcctgttcccccctaagcctaaggaca<br>ccctgatgatcagccggaccccccgaggtgacctgtgtggtggtggatgtgagccacgaggatcctgaggtgaagttcaactggtatgtggacggcgtgga<br>ggtgcacaacgccaagaccaagcctagggaggagcagtataactccacctataggtggtgagcgtgctgaccgtgctgcaccaggattggctgaatgg<br>caaggagtacaagtgcaaggtgagcaataaggctctgcctgctcctatcgagaagaccatctccaaggctaagggccagcccagggagcctcaggtgtac<br>accctgcctcccagcagggacgagctgaccaagaatcaggtgagcctgacctgcctggtgaagggcttctacccctccgatatcgctgtggagtgggaga<br>gcaacggccagcctgagaacaactacaagaccacccccccgtgctggattccgatggctccttcttcctgtacagcaagctgaccgtggataagagccg<br>gtggcagcagggcaacgtgttcagctgctccgtgatgcacgaggccctgcacaaccactacacccagaagagcctgagcctgagccctggcaag(SEQ<br>ID NO: 56) |
| 1B-1C7-V1 |

| Description |
| --- |
| Sequence (SEQ ID NO:) |

QVQLVESGGGSVQPGGSLRLSCAASGYSYSANCVGWFRQAPGKGREGVSAINTGGETATYADFVK
GRFTISRDNAKNTVYLQMNSLRAEDTAVYYCAAVGTRKYWDECSLSQHAYKNWGQGTLVTVSSG
EPKSSDKTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYSKLTVDK
SRWQQGNVFSCSVMHEALHNHYTQKSLSLSPGK(SEQ ID NO: 57)

caggtgcagctggtggagtccggcggcggaagcgtgcagcctggaggaagcctgaggctgtcctgcgctgcctccggctatagctacagcgctaactgc
gtgggctggttccggcaggctcctggcaagggccgggagggagtgtccgctatcaacaccggcggcgagaccgctacctacgccgacttcgtgaaggg
ccggttcaccatctcccgggataacgctaagaataccgtgtacctgcagatgaatagcctgcgggctgaggataccgccgtgtattattgtgctgccgtggg
cacccggaagtactgggacgagtgcagcctgagccagcacgcctacaagaattggggccagggcaccctggtgaccgtgtccagcggagagcaaagt
ccagcgataagacccacacctgcccccccttgccctgctcccgagctgctggggaggccctagcgtgttcctgttccccctaagcctaaggacaccctgatg
atcagccggacccccgaggtgacctgtgtggtggtggatgtgagccacgaggatcctgaggtgaagttcaactggtatgtggacggcgtggaggtgcaca
acgccaagaccaagcctagggaggagcagtataactccacctataggtggtgagcgtgctgaccgtgctgcaccaggattggctgaatggcaaggagt
acaagtgcaaggtgagcaataaggctctgcctgctcctatcgagaagaccatctccaaggctaagggccagcccagggagcctcaggtgtacaccctgcc
tcccagcagggacgagctgaccaagaatcaggtgagcctgacctgcctggtgaagggcttctacccctccgatatcgctgtggagtgggagagcaacgg
ccagcctgagaacaactacaagaccacccccccccgtgctggattccgatggctccttcttcctgtacagcaagctgaccgtggataagagccggtggcagc
agggcaacgtgttcagctgctccgtgatgcacgaggccctgcacaaccactacacccagaagagcctgagcctgagccctggcaag(SEQ ID NO: 58)

---

1B-1C7-V2

QVQLVESGGGSVQPGGSLRLSCAASGFTFSANCVGWFRQAPGKGREGVSAINTGGETATYADFVK
GRFTISRDNAKNTVYLQMNSLRAEDTAVYYCAAVGTRKYWDECSLSQHAYKNWGQGTLVTVSSG
EPKSSDKTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYSKLTVDK
SRWQQGNVFSCSVMHEALHNHYTQKSLSLSPGK(SEQ ID NO: 59)

caggtgcagctggtggagtccggcggcggcggatccgtgcagcctggaggatccctgaggctgagctgcgccgccagcggattcaccttcagcgccaactgc
gtgggctggttccggcaggctcctggcaagggccgggagggagtgagcgctatcaacaccggcggcgagaccgccacctatgccgacttcgtgaagg
gccggttcaccatctccaggataatgctaagaacaccgtgtacctgcagatgaatagcctgagggccgaggataccgccgtgtattactgtgccgccgtg
ggcaccaggaagtattgggacgagtgctccctgtcccagcacgcttacaagaactggggccagggcaccctggtgaccgtgagcagcggagagcaaa
gtccagcgataagacccacacctgcccccccttgccctgctcccgagctgctggggaggccctagcgtgttcctgttccccctaagcctaaggacaccctgat
gatcagccggacccccgaggtgacctgtgtggtggtggatgtgagccacgaggatcctgaggtgaagttcaactggtatgtggacggcgtggaggtgcac
aacgccaagaccaagcctagggaggagcagtataactccacctataggtggtgagcgtgctgaccgtgctgcaccaggattggctgaatggcaaggag
tacaagtgcaaggtgagcaataaggctctgcctgctcctatcgagaagaccatctccaaggctaagggccagcccagggagcctcaggtgtacaccctgc
ctcccagcagggacgagctgaccaagaatcaggtgagcctgacctgcctggtgaagggcttctacccctccgatatcgctgtggagtgggagagcaacg
gccagcctgagaacaactacaagaccacccccccccgtgctggattccgatggctccttcttcctgtacagcaagctgaccgtggataagagccggtggcag
cagggcaacgtgttcagctgctccgtgatgcacgaggccctgcacaaccactacacccagaagagcctgagcctgagccctggcaag(SEQ ID
NO: 60)

---

HGF-α

QRKRRNTIHEFKKSAKTTLIKIDPALKIKTKKVNTADQCANRCTRNKGLPFTCKAFVFDKARKQCL
WFPFNSMSSGVKKEFGHEFDLYENKDYIRNCIIGKGRSYKGTVSITKSGIKCQPWSSMIPHEHSFLPS
SYRGKDLQENYCRNPRGEEGGPWCFTSNPEVRYEVCDIPQCSEVECMTCNGESYRGLMDHTESGK
ICQRWDHQTPHRHKFLPERYPDKGFDDNYCRNPDGQPRPWCYTLDPHTRWEYCAIKTCADNTMN
DTDVPLETTECIQGQGEGYRGTVNTIWNGIPCQRWDSQYPHEHDMTPENFKCKDLRENYCRNPDG
SESPWCFTTDPNIRVGYCSQIPNCDMSHGQDCYRGNGKNYMGNLSQTRSGLTCSMWDKNMEDLH
RHIFWEPDASKLNENYCRNPDDDAHGPWCYTGNPLIPWDYCPISRCEGDTTPTIVNLDHPVISCAK
TKQLR(SEQ ID NO: 61)

---

HGF-β

(continued)

| Description |
|---|
| Sequence (SEQ ID NO:) |
| VVNGIPTRTNIGWMVSLRYRNKHICGGSLIKESWVLTARQCFPSRDLKDYEAWLGIHDVHGRGDEK CKQVLNVSQLVYGPEGSDLVLMKLARPAVLDDFVSTIDLPNYGCTIPEKTSCSVYGWGYTGLINYD GLLRVAHLYIMGNEKCSQHHRGKVTLNESEICAGAEKIGSGPCEGDYGGPLVCEQHKMRMVLGVI VPGRGCAIPNRPGIFVRVAYYAKWIHKIILTYKVPQS(SEQ ID NO: 62) |
| HCDR1 of anti-EGFR antibody |
| TYGMH (SEQ ID NO: 63) |
| HCDR2 of anti-EGFR antibody |
| VIWDDGSYKYYGDSVKG (SEQ ID NO: 64) |
| HCDR3 of anti-EGFR antibody |
| DGITMVRGVMKDYFDY (SEQ ID NO: 65) |
| LCDR1 of anti-EGFR antibody |
| RASQDISSALV (SEQ ID NO: 66) |
| LCDR2 of anti-EGFR antibody |
| DASSLES (SEQ ID NO: 67) |
| LCDR3 of anti-EGFR antibody |
| QQFNSYPLT (SEQ ID NO: 68) |
| VH of anti-EGFR antibody |
| QVQLVESGGGVVQPGRSLRLSCAASGFTFSTYGMHWVRQAPGKGLEWVAVIWDDGSYKYYGDS VKGRFTISRDNSKNTLYLQMNSLRAEDTAVYYCARDGITMVRGVMKDYFDYWGQGTLVTVSS (SEQ ID NO: 69) |
| VL of anti-EGFR antibody |
| AIQLTQSPSSLSASVGDRVTITCRASQDISSALVWYQQKPGKAPKLLIYDASSLESGVPSRFSGSESGT DFTLTISSLQPEDFATYYCQQFNSYPLTFGGGTKVEIK (SEQ ID NO: 70) |
| Anti-c-Met polypeptide chain anti-c-Met-$V_H$H-Fc of V12 |

(continued)

| Description |
| --- |
| Sequence (SEQ ID NO:) |

| QVQLVESGGDSVQAGGSLRLSCAASAHAYSSGCMGWFRQAPGKGREGVATIYTRNDFTYYSDSVK GRFTISRDDAKNTVYLQVNSLKAEDTAMYYCAAGSPFRYAGAWNVCGLKEEDYNYWGQGTLVT VSSGGGGSGGGGSQVQLVESGGGLVQPGGSLRLSCAASGYSYSANCVGWFYQAPGKEREGVAAIN TGGETATYADFVKGRFTISQDYAKNTVYLQMNSLKSEDTGMYYCAAVGTRKYWDECSLSQHAYK NWGQGTLVTVSSGEPKSSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSNKALPAPIE KTISKAKGQPREPQVCTLPPSREEMTKNQVSLSCAVKGFYPSDIAVEWESNGQPENNYKTTPPVLDS DGSFFLVSKLTVDKSRWQQGNVFSCSVMHEALHNRFTQKSLSLSPGK (SEQ ID NO: 71) |
| --- |
| caagtgcagctggtcgagtccggcggcgactccgtgcaggctggcggatctctgagactgtcttgcgctgcttctgcccacgcttactccagcggctgcatg ggctggttcagacaggcccctggcaagggcagagaaggagtggctaccatctacaccagaaacgactttacctattactccgattctgtcaagggcaggttc accatctcccgggacgacgccaagaacaccgtgacctgcaggtaacagcctgaaggccgaggataccgccatgtactactgcgccgctggctcccctt tccggtatgccggcgcctggaacgtgtgcggcctgaaagaagaagactacaactactggggccagggaaccttggtgaccgtctcatccggggggcggcg gtagcggtggcggaggctctcaagtgcagctggtggaatctggcggaggactggttcagcctggcggctctctccggctgtcctgcgccgcctctggctac tcctactctgccaactgcgtgggctggttctaccaggcccccggcaaggagagagagggcgtggccgccatcaacaccggcggcgagacagcaaccta cgccgacttcgtgaaaggccggtttacaatctctcaggattatgctaagaataccgtgtacctgcagatgaactccctgaagtccgaggacacaggcatgtac tactgtgctgctgtgggcacccggaagtactgggacgagtgctctctgagccagcacgcctacaaaaattggggccaaggcaccctggtgaccgtgtcca gtggcgaacccaagtcctccgacaagacccacacctgcccccccctgtcctgctccagagctgctgggcggcccttctgtgtttctgttccctcctaagcccaa ggacaccctgatgatctccagaacccctgaggtgacctgtgtggtggtggacgtgtctcacgaggatcctgaagtgaagttcaactggtacgtggacggcg tggaagtgcataacgccaagacaaagcctagagaggaacagtacaactctacctacagagtggtgtccgtgctgaccgtgctgcaccaggactggctgaa cggcaaagagtacaagtgcaaggtgtccaacaaggctctgcctgcccctatcgagaagaccatcagcaaggccaagggccagccccgcgagcctcagg tctgcaccctgcctccatccagagaagagatgaccaagaaccaggtttccctgtcttgtgctgtgaagggcttctaccctagcgacatcgccgtggaatggg agtctaatggccagcctgagaacaactacaagaccacacctcctgtgctcgactccgatggctccttcttcctggtgtccaagctgacggtggataagtctag atggcagcagggcaacgtgttctcctgctccgtgatgcacgaggccctgcataaccggttcacccagaagagcctgtccctgtctccaggaaaa (SEQ ID NO: 72) |

| Anti-c-Met polypeptide chain anti-c-Met-V$_H$H-Fc of V41 |
| --- |
| QVQLLESGGGSVQAGGSLRLSCAASGFTSSGCMGWFRQAPGKGREGVATIYTRNDFTYYSDSVK GRFTISRDNSKNTVYLQMNSLRAEDTAVYYCAAGSPFRYAGAWNVCGLKEEDYNYWGQGTLVTV SSGGGGSGGGGSQVQLVESGGGSVQPGGSLRLSCAASGYSYSANCVGWFRQAPGKGREGVSAINT GGETATYADFVKGRFTISRDNAKNTVYLQMNSLRAEDTAVYYCAAVGTRKYWDECSLSQHAYKN WGQGTLVTVSSGEPKSSDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPIEK TISKAKGQPREPQVCTLPPSREEMTKNQVSLSCAVKGFYPSDIAVEWESNGQPENNYKTTPPVLDSD GSFFLVSKLTVDKSRWQQGNVFSCSVMHEALHNRFTQKSLSLSPGK (SEQ ID NO: 73) |
| caggtgcagctgctggaatctggcggcggctctgtgcaggctggtggatccctgagactgtcttgtgctgcctccggctttaccttcagcagtggctgcatgg gctggtttagacaggcccccggcaaaggcagagaaggcgtggccaccatctacaccagaaacgacttcacctattattccgattccgtgaaaggcagattc accatcagccgcgacaactccaaaaacaccgtgacctgcagatgaactccctgagagccgaggataccgctgtgtactactgcgccgccggctctcctttc cggtacgccggagcttggaacgtgtgtggcctgaaggaagaggactacaactactggggacaaggcaccctggtgaccgtgtctagcggcggaggtgg ctcgggcggaggcggctctcaagtgcagctggtggaatccggcggcggctccgtgcaacccggaggctccctgcggttgtcctgtgctgcctctggctac tcctactccgccaactgcgtaggatggttcagacaggcccctggcaagggcagagagggcgtgtccgctatcaacaccggcggcgagacagccacctac gccgacttcgtgaagggccggttcaccatctctgggacaacgccaagaacactgtctatctgcagatgaactccctgagagcagaagataccgccgtgta ctactgtgctgctgtgggcacccggaagtactgggacgagtgctctctgtcccagcacgcctacaagaattggggccagggcacactagtcaccgtctcct ctggcgagcctaagtcctctgataagacccacacctgtcctcttgccctgctcctgagctgctgggcggcccttccgtcttcctgttccctccaaagcccaag gacacactgatgatctccaggacacctgaagtgacctgcgtggtggtggacgtgtctcatgaggaccccgaggtgaagttcaactggtacgtggatggcgt ggaagtgcacaatgccaagaccaagcctcgggaggaacagtacaactctacctacagagtggtgtccgtgctgaccgtcctgcaccaggactggctgaac ggcaaagagtacaagtgcaaggtgtccaacaaggccctgccagctcctatcgagaagacaatcagcaaggccaagggccagcctagagagcctcaggt gtgcaccctgcctccttctcgggaagagatgaccaagaaccaggtgagcctgtcctgcgccgtgaaaggcttctacccctccgacatcgccgtcgagtggg agtctaatggcagcccgagaacaactacaagaccacccctcctgtgctggactccgacggctctttcttcctggtttctaagctgaccgtggacaagtccag |

| atggcagcagggcaacgtgttctcctgctccgtgatgcacgaggctctgcacaaccggtttacccagaagtctctgtctctcagcccaggcaag (SEQ ID NO: 74) |
| --- |
| Anti-c-Met polypeptide chain anti-c-Met-V$_H$H-Fc of V35 |

(continued)

| Description<br>Sequence (SEQ ID NO:) |
| --- |
| QVQLLESGGGSVQAGGSLRLSCAASGHTYSSGCMGWFRQAPGKGREGVATIYTRNDFTYYSDSVK GRFTISRDNSKNTVYLQMNSLRAEDTAVYYCAAGSPFRYAGAWNVCGLKEEDYNYWGQGTLVTV SSGGGGSGGGGSQVQLVESGGGSVQPGGSLRLSCAASGYSYSANCVGWFRQAPGKGREGVSAINT GGETATYADFVKGRFTISRDNAKNTVYLQMNSLRAEDTAVYYCAAVGTRKYWDECSLSQHAYKN WGQGTLVTVSSGEPKSSDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPIEK TISKAKGQPREPQVCTLPPSREEMTKNQVSLSCAVKGFYPSDIAVEWESNGQPENNYKTTPPVLDSD GSFFLVSKLTVDKSRWQQGNVFSCSVMHEALHNRFTQKSLSLSPGK (SEQ ID NO: 75)<br><br>caggttcagctgttggaatctggcggcggatctgttcaggctggcggatctctgagactgtcttgtgctgcttccggccacacctactcctctggctgtatggg atggttcagacaggcccctggcaagggaagagaaggcgtggccaccatctacacccggaacgacttcacctactactccgactccgtgaagggcagattc accatcagccgggacaactccaagaacaccgtgtacctgcagatgaactccctgagagccgaggacaccgccgtgtactattgtgccgctggcagcccct ttagatacgctggcgccttggaacgtgtgcggcctgaaagaagaggactacaactactggggccagggcaccctggtcacagtttctagcggaggcggag gatcaggtggcggtggatctcaggtgcagttggttgaaagtggcggaggctctgtgcaaccaggcggaagcctcagattgtcttgcgccgcttctggctact cctacagcgccaattgtgtcggctggtttaggcaggctcccggaaaaggcagagaaggggtgtccgctatcaacaccggcggagagacagccacctacg ccgatttcgtgaaaggccggtttaccatctctcgcgataacgctaagaacacagtctatctccaaatgaacagcctgcgcgctgaggataccgctgtgtattac tgcgctgccgtgggcaccagaaagtactgggacgagtgctccctgagccagcacgcctataagaattggggacagggaaccctcgtgaccgtgtcctctg gcgaacctaagtcctccgacaagacccacacctgtcctccatgtcctgctccagaactgctcggcggaccttccgtgttcctgtttcctccaaagcctaagga caccctgatgatctctcggacccctgaagtgacctgcgtggtggtggatgtgtctcacgaggatcccgaagtgaagttcaattggtacgtggacggcgtgga agtgcacaacgccaagaccaagcctagagaggaacagtacaactccacctacagagtggtgtccgtgctgaccgtgctgcaccaggattggctgaacgg caaagagtacaagtgcaaggtgtccaacaaggccctgcctgctcctatcgaaaagaccatctccaaggccaagggccagcctcgggaacctcaagtctgt accctgcctcctagccgggaagagatgaccaagaaccaggtgtccctgtcctgcgctgtgaagggcttctacccttccgatatcgccgtggaatgggagag caatggccagccagagaacaactacaagacaaccccctcctgtgctggactccgacggctcattcttcctggtgtccaagctgacagtggacaagtccagat ggcagcagggcaacgtgttctcctgctccgtgatgcacgaggccctgcacaacaggttcacccagaagtccctgtctctgagcccaggcaaa (SEQ ID NO: 76) |
| Anti-c-Met polypeptide chain anti-c-Met-V$_H$H-Fc of V42<br><br>QVQLLESGGGSVQAGGSLRLSCAASGHTYSSGCMGWFRQAPGKGREGVATIYTRNDFTYYSDTVK GRFTISRDNSKNTVYLQMNSLRAEDTAVYYCAAGSPFRYAGAWNVCGLKEEDYNYWGQGTLVTV SSGGGGSGGGGSQVQLVESGGGSVQPGGSLRLSCAASGYSYSANCVGWFRQAPGKGREGVSAINT GGETATYADFVKGRFTISRDNAKNTVYLQMNSLRAEDTAVYYCAAVGTRKYWDECSLSQHAYKN WGQGTLVTVSSGEPKSSDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPIEK TISKAKGQPREPQVCTLPPSREEMTKNQVSLSCAVKGFYPSDIAVEWESNGQPENNYKTTPPVLDSD GSFFLVSKLTVDKSRWQQGNVFSCSVMHEALHNRFTQKSLSLSPGK (SEQ ID NO: 77)<br><br>caggttcagctgctggaatctggcggaggctccgtgcaggccggcggaagcctgagactgtcctgcgccgcctctggccatacatattcttccggctgcat gggctggttccggcaggctcctggcaagggcagggagggcgtggccacaatctacaccagaaacgacttcacctattattctgataccgtcaaaggcagat tcaccatctctcgggacaactccaagaacaccgtgtacctgcagatgaactccctgcgggctgaggacaccgccgtctactactgcgccgctggctctccttt tagatacgctggcgcctggaacgtgtgtggcctgaaggaagaagactacaactactggggccaaggcaccctggtgaccgtgtcctccggcggcggagg ctctggaggcggcggcagccaagtgcagctggtggaaagcggcggtggctccgtgcagccaggcggctctctgcggctgtcctgtgccgcttctggcta ctcctactccgccaactgcgtgggctggttcagacaggcccccggcaagggccgggaaggcgtgtctgccatcaacaccggcggcgagacagctaccta cgccgacttcgtgaagggcagatttaccatctccagagataacgctaagaataccgtgtacctgcagatgaacagcctgcgggccgaggacaccgctgtgt actactgtgctgccgtgggcacccggaagtactgggacgagtgcagtctgagccagcacgcctacaagaattggggccagggaacactggtcaccgtgt ccagtggagaacctaagtcgtctgacaagacccacacctgtcctccatgccccgctcccgagctgctgggcggcccttctgtgttcctgttccctcctaagcc caggacacactgatgatcagcagaacccctgaagtgacctgcgtggtggtggacgtgtcccacgaggatcctgaggtgaagttcaactggtacgtggac ggcgtggaagtgcacaacgccaagaccaagcctcgcgaggagcagtacaactccacctacagagtggtctctgtactgaccgtgctgcaccaggactgg ctgaacggaaaagagtacaagtgcaaggtgtccaacaaggctctgcctgccccctatcgagaagaccatctccaaggccaaaggacagcctagagagcct caagtgtgcaccctgcctccatctctgggaagagatgactaagaaccaggtctccctgtcctgtgccgtcaagggcttctacccctccgacatcgccgtggag tgggagtccaacggccagcctgagaacaactacaaaaccacacctcctgtgctggacagcgatggctccttcttcctcgtgtccaagctgaccgttgataag tccagatggcagcagggcaacgtgttctcttgctccgtgatgcacgaggccctgcataatagatttacccagaagtctctgtcgctctctcccggcaaa (SEQ ID NO: 78) |
| Anti-c-Met polypeptide chain anti-c-Met-V$_H$H-Fc of V67 |

| Description |
| --- |
| Sequence (SEQ ID NO:) |

EVQLVESGGGSVQAQGSLRLSCTVSGSTYSSNFMAWFRQAPGKEREWVATITTGAGNTAYADSVK GRFTISEDNAKRTAYLQMNSLKPEDTAVYYCAAGRGGFWRPAEYDFWGQGTQVTVSSGGGGSQV QLVESGGGSVQPGGSLRLSCAASGYSYSANCVGWFRQAPGKGREGVSAINTGGETATYADFVKGR FTISRDNAKNTVYLQMNSLRAEDTAVYYCAAVGTRKYWDECSLSQHAYKNWGQGTLVTVSSGEP KSSDKTHTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPREPQV

CTLPPSREEMTKNQVSLSCAVKGFYPSDIAVEWESNGQPENNYKTTPPVLDSDGSFFLVSKLTVDKS RWQQGNVFSCSVMHEALHNRYTQKSLSLSPGK (SEQ ID NO: 79)

gaagtgcagctggtcgagtctggcggcggctctgtgcaggctcagggctccctgcggctgtcttgtaccgtttccgggtccacctactcttccaacttcatgg cctggttccggcaggcccctggaaaagagagagaatgggtggctaccatcaccacaggcgctggcaacaccgcttatgccgactccgtcaagggcagat tcaccatctctgaggacaacgccaagcggaccgcctacctgcagatgaacagcctgaagcctgaagataccgccgtgtactactgtgctgccggcagagg cggcttttggcggcccgctgagtacgacttctggggccaaggcactcaagtgacagtgtcctctggaggaggaggctctcaggtgcagctggtggaatctg gcggcggctccgtgcaaccaggcggatctctgagactgtcctgcgctgcttccggctactcctactccgccaactgcgtgggctggttcagacaggcccct ggcaaaggccgcgagggcgtctctgccatcaacaccggcggagagacagctacctacgccgattttgttaaggggccggttcactatctccagagacaatg ctaagaacaccgtgtacctgcagatgaactctctgagagccgaggacaccgctgtgtactactgcgccgctgtgggcacccggaagtactgggacgagtg ctctctgtcccagcacgcctataagaattggggccagggcaccctcgtgaccgtgtcttcaggcgagcctaaaagcagcgataagacccacacctgccctc cttgccccgctcctgagctgctgggcggaccttctgtctttctgttcccacctaagcccaaggacaccctgatgatcagccggacacctgaggtgacctgtgt ggtggtggacgtgtctcatgaggatcctgaggtgaagttcaactggtacgtggatggcgtggaagtgcacaatgccaagaccaagcctagagaagagcag tacaactccacatacagagtggtctcggtgctgaccgtgctgcaccaggactggctgaacggcaaagaatacaagtgcaaggtgtccaacaaggctctgcc tgcccccatcgagaagacaatctccaaggccaagggccagcctcgggaacctcaggtgtgcaccctgcccccctccagggaagaaatgacaaagaacc aggtgtccctatcctgtgccgtgaaaggcttctaccttccgacatcgccgtggagtgggagtccaacggccagcccgagaacaactacaaaaccacccct ccagtgctggactctgacggctccttcttcctggtgagcaagctgaccgtggacaagtctagatggcaacagggaaacgtgttcagctgctccgtcatgcac gaggccctgcacaacagatacacccagaagtccctgagcctgtctcctggcaag (SEQ ID NO: 80)

Anti-c-Met polypeptide chain anti-c-Met-V$_H$H-Fc of V73

EVQLVESGGGSVQAGGSLRLSCAVSGFTVSSNFMAWFRQAPGKGREWVSTITTGAGNTAYADSVK GRFTISEDNSKNTLYLQMNSLRAEDTAVYYCAAGRGGFWRPAEYDFWGQGTTVTVSSGGGGSQV QLVESGGGSVQPGGSLRLSCAASGYSYSANCVGWFRQAPGKGREGVSAINTGGETATYADFVKGR FTISRDNAKNTVYLQMNSLRAEDTAVYYCAAVGTRKYWDECSLSQHAYKNWGQGTLVTVSSGEP KSSDKTHTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPREPQV CTLPPSREEMTKNQVSLSCAVKGFYPSDIAVEWESNGQPENNYKTTPPVLDSDGSFFLVSKLTVDKS RWQQGNVFSCSVMHEALHNRYTQKSLSLSPGK (SEQ ID NO: 81)

gaggtgcagctggtggaatcaggcggaggctccgtgcaggccggcggctctttgagactgtcttgtgccgtgtccggcttcaccgtgtcttctaacttcatgg cctggttcagacaggctcctggcaagggcagagagtgggtcagcaccatcaccaccggcgctggaaacaccgcctacgccgactccgtgaaagggcgc tttacaatcagcgaggacaactccaagaataccctgtacctgcagatgaactctctgcgggccgaggataccgccgtgtactactgtgctgctggcagaggc ggcttttggcggccagctgagtacgacttctggggccaaggcaccacagtcaccgtgtcctctggcggaggtggcagccaagtgcagctggtcgaatctg gaggtggctccgttcagcctggcggctctctgagactgtcttgcgccgcttccggctactcttactccgccaactgcgtgggctggttccggcaggccccg gcaaaggcagagaaggcgtgagcgccatcaacaccggcggcgaaaccgctacctatgccgatttcgtgaagggaagattcaccatctctcgggacaacg ccaagaacaccgtgtatctgcagatgaactctctgagagccgaggatacagctgtgtactactgcgctgctgtgggcaccagaaagtactgggacgagtgc tccctgtcccagcacgcctataagaactggggacagggcacactcgtgaccgtgtctagcggcgagcctaaaagctctgacaaaacccacacctgtcctcc ttgccccgctcctgagctgctgggcggcccttccgtgtttctgttccctcccaagcccaaggacacactgatgatctccagaacccccggaagtgacctgcgtg gtggtggacgtgtctcacgaggaccctgaggtcaagttcaactggtacgtggatggcgtggaagtgcataatgctaagaccaagcctcgggaggaacagt acaactccacctacagagtggttccgtgctgaccgtgctgcaccaagactggctgaacggcaaagtacaagtgcaaggtgtccaacaaggctctgcct gcccccatcgagaagacgatctccaaggccaagggccagcctagggaacctcaggtgtgcaccctgcctccttcccgggaagagatgaccaaaaacca ggtcagcctgtcttgcgccgtgaaaggcttctacccctccgacatcgccgtggaatgggagtccaacggccagcccgagaataactacaagaccacccct cctgtgctcgatagcgacggctccttcttcctggtgtctaagctgacagtggacaagtctagatggcagcagggcaacgtgttctcctgttccgtgatgcacg aggccctgcacaaccggtacacccagaagtccctgagcctgtctccaggcaag (SEQ ID NO: 82)

Anti-c-Met polypeptide chain anti-c-Met-V$_H$H-Fc of V74

(continued)

| Description<br>Sequence (SEQ ID NO:) |
| --- |
| EVQLVESGGGSVQAGGSLRLSCAVSGFTVSSNFMAWFRQAPGKGREWVSTITTGAGNTAYADSVK<br>GRFTISEDNSKNTAYLQMNSLRAEDTAVYYCAAGRGGFWRPAEYDFWGQGTTVTVSSGGGGSQV<br>QLVESGGGSVQPGGSLRLSCAASGYSYSANCVGWFRQAPGKGREGVSAINTGGETATYADFVKGR<br>FTISRDNAKNTVYLQMNSLRAEDTAVYYCAAVGTRKYWDECSLSQHAYKNWGQGTLVTVSSGEP<br>KSSDKTHTCPPCPAPELLGGPSVFLFPPKPKDTLMISRTPEVTCVVVDVSHEDPEVKFNWYVDGVE<br>VHNAKTKPREEQYNSTYRVVSVLTVLHQDWLNGKEYKCKVSNKALPAPIEKTISKAKGQPREPQV<br>CTLPPSREEMTKNQVSLSCAVKGFYPSDIAVEWESNGQPENNYKTTPPVLDSDGSFFLVSKLTVDKS<br>RWQQGNVFSCSVMHEALHNRYTQKSLSLSPGK (SEQ ID NO: 83)<br>gaagtgcagctggtggaatccggcggcggatctgtgcaggccggggggctccctgagactgtcttgcgccgtgtccggcttcaccgtgtcttctaacttcatg<br>gcctggtttcgccaggcacctggcaagggcagagagtgggtgtctaccatcaccaccggcgctgggaacaccgcttacgccgattctgtgaaaggccggt<br>tcaccatctccgaggataattccaagaacacagcttatctgcagatgaacagcctgagagccgaggacaccgccgtatactactgtgctgctggcagaggc<br>ggcttttggcggcctgctgagtacgacttctgggggccagggcaccacagtcacagtttcctctggaggcggtggctctcaagtgcagctggtggagtctgg<br>gggaggctctgtccaacctggaggatctctgcggctgagctgcgccgcctctggctactcctacagcgccaactgcgtgggctggttcagacaggcccctg<br>gaaaagggcagagaaggcgtttctgccatcaacaccggcggcgagacagccacatacgccgacttcgtgaagggcagattcaccatcagtcgggacaac<br>gccaaaaacaccgtgtacctgcagatgaactccctccgggccgaggacaccgctgtgtactactgcgctgctgtcggcaccagaaagtactgggacgagt<br>gctctctgagccagcacgcctacaaaaattgggggccaaggcaccctcgtgacagtgagcagcggcgaacccaagtccagcgacaagacccacacctgc<br>cctccttgtcctgccccagagctgctggcggcgcttccgtgtttctgttcccacccaagcctaaggacactctgatgatcagcagaacccctgaggtgacct<br>gtgtggtggtggacgtgtctcacgaagatcctgaagtcaagttcaactggtacgtggacggcgtggaagtgcacaacgctaagaccaagcccagagaaga |
| gcagtacaacagcacctacagagtggtctccgtgctgaccgtgctgcaccaggactggctgaacggcaaagagtataagtgcaaggtgtccaacaaggct<br>ctgcccgctcctatcgagaagaccatctccaaggccaagggccagcctcgggagcctcaggtgtgcaccctgcctccctcccgggaagagatgaccaag<br>aaccaggtgtccttgtcttgtgctgtgaagggcttctacccttcggacatcgccgtggaatgggagtccaatggacagcccgagaacaactacaagacaacc<br>cctcctgtgctggactccgatggctccttcttcctggtgtccaagctgaccgtggataagtctaggtggcagcagggcaacgtgttctcctgctccgtgatgca<br>cgaggccctgcataacagatacacccagaagtccctgtccctgtctcctggcaaa (SEQ ID NO: 84) |
| Heavy chain anti-EGFR-HC-Fc of anti-EGFR antibody |
| QVQLVESGGGVVQPGRSLRLSCAASGFTFSTYGMHWVRQAPGKGLEWVAVIWDDGSYKYYGDS<br>VKGRFTISRDNSKNTLYLQMNSLRAEDTAVYYCARDGITMVRGVMKDYFDYWGQGTLVTVSSAS<br>TKGPSVFPLAPSSKSTSGGTAALGCLVKDYFPEPVTVSWNSGALTSGVHTFPAVLQSSGLYSLSSVV<br>TVPSSSLGTQTYICNVNHKPSNTKVDKRVEPKSCDKTHTCPPCPAPELLGGPSVFLFPPKPKDTLMIS<br>RTPEVTCVVVDVSHEDPEVKFNWYVDGVEVHNAKTKPREEQYNSTYRVVSVLTVLHQDWLNGK<br>EYKCKVSNKALPAPIEKTISKAKGQPREPQVYTLPPCREEMTKNQVSLWCLVKGFYPSDIAVEWES<br>NGQPENNYKTTPPVLDSDGSFFLYSKLTVDKSRWQQGNVFSCSVMHEALHNHYTQKSLSLSPGK<br>(SEQ ID NO: 85)<br>caggtgcagctggtggagtccggcggcggagtggtgcagccaggaagatccctgcggctgagctgtgccgcttccggcttcaccttctccacctacggca<br>tgcactgggtgcggcaggcccccggaaagggactggagtgggtggctgtgatctgggacgacggctcctacaagtattacggcgattccgtgaagggcc<br>ggttcaccatctcccgggacaactccaagaacaccctgtacctgcagatgaatagcctgagggccgaggacaccgctgtgtactactgtgcccgggacgg<br>catcaccatggtgcggggagtgatgaaggattacttcgactactggggccagggcaccctggtgaccgtgagctccgccagtaccaaaggcccttccgtgt<br>ttcctctggccccatcctccaagagtacatctggaggcaccgctgctctgggctgcctggtgaaggactacttccccgagcccgtgaccgtcagctggaact<br>ccggcgccctgacctctggcgttcatacctttcccgccgttctgcagagctctggcctgtacagcctgtcttccgtcgtgaccgtgccttcagctctctgggaa<br>cacagacctacatctgcaacgtgaaccacaagcccagcaacaccaaggtggacaagcgggtggaacccaagtctgcgacaaaacccacacctgtcctc<br>cttgtccagcccctgagctgctggcggcccttctgtctttctgttccctcctaagcctaaggacacgctcatgatctcccggacacctgaagtgacctgcgtg<br>gtggtggatgtgtctcacgaggaccctgaggtgaagttcaactggtacgtggacggcgtggaagtccataacgccaagaccaagcctagagaggagcagt<br>acaattccacctataggtggtgagcgtgctgaccgtgctgcaccaggattggctgaatggcaaggagtacaagtgcaaggtgagcaataaggctctgcct<br>gctcccatcgagaagaccatctccaaggctaagggccagcctaggagcccccaggtgtataccctgcctccctgccgggaggagatgaccaagaaccag<br>gtgtccctgtggtgcctggtgaagggcttcacccttccgacatcgctgtggagtgggagtccaatggccagcccgagaataattacaagaccaccccctccc<br>gtgctggattccgacggctccttcttcctgtactccaagctgaccgtggacaagagcaggtggcagcagggcaacgtgttcagctgtagcgtgatgcacga<br>ggccctgcacaaccactacacccagaagtccctgagcctgagcccggcaag (SEQ ID NO: 86) |
| Light chain anti-EGFR-LC of anti-EGFR antibody |

(continued)

| Description Sequence (SEQ ID NO:) |
|---|
| AIQLTQSPSSLSASVGDRVTITCRASQDISSALVWYQQKPGKAPKLLIYDASSLESGVPSRFSGSESGT DFTLTISSLQPEDFATYYCQQFNSYPLTFGGGTKVEIKRTVAAPSVFIFPPSDEQLKSGTASVVCLLNN FYPREAKVQWKVDNALQSGNSQESVTEQDSKDSTYSLSSTLTLSKADYEKHKVYACEVTHQGLSS PVTKSFNRGEC (SEQ ID NO: 87) |
| gctatccagctgacccagtccctagctccctgagcgcctccgtgggcgacagggtgaccatcacctgcagggccagccaggatatctcctccgccctggt gtggtaccagcagaagcccggcaaggccccaagctgctgatctatgacgctagctccctggagtccggcgtgccctccaggttcagcggctccgagtcc ggcaccgacttcaccctgaccatctccagcctgcagcccgaggacttcgccacctattattgtcagcagttcaatagctaccccctgaccttcggcggcggc accaaggtggagatcaagaggaccgtggccgcccctagcgtgttcatcttccctcctagcgacgagcagctgaagagcggcaccgcctccgtggtgtgcc tgctgaacaacttctatcccagggaggctaaggtgcagtggaaggtggataacgccctgcagtccggcaatagccaggagagcgtgaccgagcaggatt ccaaggattccacctactccctgagcagcaccctgaccctgagcaaggctgattacgagaagcacaaggtgtatgcttgtgaggtgacccaccagggcctg agcagccccgtgaccaagtccttcaacagggggcgagtgt (SEQ ID NO: 88) |
| First hinge GEPKSSDKTHTCPPCP (SEQ ID NO: 89) |
| Second hinge EPKSCDKTHTCPPCP (SEQ ID NO: 90) |
| Peptide linker GGGGS (SEQ ID NO: 91) |
| Heavy chain constant region of human IgG1 ASTKGPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKSLSLSPGK (SEQ ID NO: 92) |

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the contents of these documents are based on the information available to the applicant and do not constitute any admission as to the correctness of the dates or the content of these documents. Moreover, in any country or region, any reference to these publications herein should not be construed as an admission that the publications form part of the commonly recognized knowledge in the art.

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the claimed scope of the present disclosure.

**Claims**

1. A multispecific antibody, comprising

> (i) a first antigen-binding moiety binding to a first antigen;
> (ii) a second antigen-binding moiety binding to a second antigen; and
> (iii) a third antigen-binding moiety binding to the first antigen,

wherein the first antigen is c-Met, and the second antigen is EGFR; and the first antigen-binding moiety and the third antigen-binding moiety are both single variable domains and each independently comprise any one of the following:

> (1) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6,

wherein $X_1$ is selected from S and T, preferably T;

(2) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;

(3) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;

(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;

(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;

(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;

(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

**2.** The multispecific antibody according to claim 1, wherein the first antigen-binding moiety and the third antigen-binding moiety each independently comprise any one of the following:

(1) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein, $X_1$ is selected from S and T, preferably T;

(2) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; or

(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24;

preferably, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is selected from S and T; preferably, $X_1$ is T; or

preferably, the first antigen-binding moiety comprises: a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9; and the third antigen-binding moiety comprises: a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO: 24.

**3.** The multispecific antibody according to claim 1 or 2, wherein the first antigen-binding moiety and the third antigen-binding moiety each independently comprise an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40, 41, 35, 36, 26, 27, 25, 28, 29, 30, 31, 32, 33, 34, 37, 38, 39, or 42.

**4.** The multispecific antibody according to any one of claims 1-3, wherein the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41;

preferably, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 27, 36, or 37;

more preferably, the first antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 36.

5. The multispecific antibody according to any one of claims 1-4, wherein the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40;

preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 26, 33, 34, or 35; more preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35; or
preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 42;
more preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32, 38, or 39; more preferably, the third antigen-binding moiety comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 38.

6. The multispecific antibody according to any one of claims 1-5, wherein the first antigen-binding moiety and the third antigen-binding moiety are selected from any one of the following:

(1) the first antigen-binding moiety comprises the amino acid sequence set forth in SEQ ID NO: 36, and the third antigen-binding moiety comprises the amino acid sequence set forth in SEQ ID NO: 35;
(2) the first antigen-binding moiety comprises the amino acid sequence set forth in SEQ ID NO: 27, and the third antigen-binding moiety comprises the amino acid sequence set forth in SEQ ID NO: 26;
(3) the first antigen-binding moiety comprises the amino acid sequence set forth in SEQ ID NO: 36, and the third antigen-binding moiety comprises the amino acid sequence set forth in SEQ ID NO: 33;
(4) the first antigen-binding moiety comprises the amino acid sequence set forth in SEQ ID NO: 36, and the third antigen-binding moiety comprises the amino acid sequence set forth in SEQ ID NO: 34;
(5) the first antigen-binding moiety comprises the amino acid sequence set forth in SEQ ID NO: 36, and the third antigen-binding moiety comprises the amino acid sequence set forth in SEQ ID NO: 32;
(6) the third antigen-binding moiety comprises the amino acid sequence set forth in SEQ ID NO: 36, and the first antigen-binding moiety comprises the amino acid sequence set forth in SEQ ID NO: 38; or
(7) the third antigen-binding moiety comprises the amino acid sequence set forth in SEQ ID NO: 36, and the first antigen-binding moiety comprises the amino acid sequence set forth in SEQ ID NO: 39.

7. The multispecific antibody according to any one of claims 1-6, wherein the single variable domain is derived from a camelid or is humanized.

8. The multispecific antibody according to any one of claims 1-7, wherein the second antigen-binding moiety comprises a heavy chain variable region and a light chain variable region; the heavy chain variable region comprises: an HCDR1 comprising the amino acid sequence set forth in SEQ ID NO: 63, an HCDR2 comprising the amino acid sequence set forth in SEQ ID NO: 64, and an HCDR3 comprising the amino acid sequence set forth in SEQ ID NO: 65, and the light chain variable region comprises: an LCDR1 comprising the amino acid sequence set forth in SEQ ID NO: 66, an LCDR2 comprising the amino acid sequence set forth in SEQ ID NO: 67, and an LCDR3 comprising the amino acid sequence set forth in SEQ ID NO: 68.

9. The multispecific antibody according to any one of claims 1-8, wherein the second antigen-binding moiety comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 70;
preferably, the heavy chain variable region of the second antigen-binding moiety comprises the amino acid sequence set forth in SEQ ID NO: 69, and the light chain variable region thereof comprises the amino acid sequence set forth in SEQ ID NO: 70.

10. The multispecific antibody according to any one of claims 1-9, wherein the second antigen-binding moiety is murine, chimeric, or humanized.

11. The multispecific antibody according to any one of claims 1-10, wherein the multispecific antibody further comprises:
(iv) an Fc domain composed of two Fc polypeptides;

preferably, the Fc domain is an IgG Fc domain, more preferably an IgG1 Fc domain;
preferably, the IgG Fc domain is a human IgG Fc domain, more preferably a human IgG1 Fc domain.

12. The multispecific antibody according to any one of claims 1-11, wherein the Fc domain comprises an amino acid substitution that promotes the association of the two Fc polypeptides of the Fc domain and/or an amino acid substitution that reduces or eliminates the binding of the CH3 region of one Fc polypeptide in the Fc domain to protein A;

preferably, according to the EU numbering, one of the Fc polypeptides of the Fc domain comprises amino acid substitutions 349C, 366S, 368A, 407V, 435R, and 436F, and the other Fc polypeptide comprises amino acid substitutions 354C and 366W; or
preferably, according to the EU numbering, one of the Fc polypeptides of the Fc domain comprises amino acid substitutions 349C, 366S, 368A, 407V, and 435R, and the other Fc polypeptide comprises amino acid substitutions 354C and 366W.

13. The multispecific antibody according to any one of claims 1-12, wherein the third antigen-binding moiety and the first antigen-binding moiety are fused to each other, optionally via a peptide linker;

preferably, the third antigen-binding moiety, at the C-terminus, is fused to the N-terminus of the first antigen-binding moiety;
preferably, the first antigen-binding moiety, at the C-terminus, is fused to the N-terminus of one of the Fc polypeptides of the Fc domain, the second antigen-binding moiety is a Fab, and the second antigen-binding moiety, at the C-terminus of the Fab heavy chain, is fused to the N-terminus of the other Fc polypeptide of the Fc domain.

14. The multispecific antibody according to any one of claims 1-13, wherein the multispecific antibody consists of three polypeptide chains, wherein:

(1) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;
(2) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;
(3) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;
(4) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(5) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(6) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87; or

(7) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

more preferably, the multispecific antibody consists of three polypeptide chains, wherein:

(1) one polypeptide chain comprises the amino acid sequence set forth in SEQ ID NO: 77, another polypeptide chain comprises the amino acid sequence set forth in SEQ ID NO: 85, and the other polypeptide chain comprises the amino acid sequence set forth in SEQ ID NO: 87;

(2) one polypeptide chain comprises the amino acid sequence set forth in SEQ ID NO: 71, another polypeptide chain comprises the amino acid sequence set forth in SEQ ID NO: 85, and the other polypeptide chain comprises the amino acid sequence set forth in SEQ ID NO: 87;

(3) one polypeptide chain comprises the amino acid sequence set forth in SEQ ID NO: 73, another polypeptide chain comprises the amino acid sequence set forth in SEQ ID NO: 85, and the other polypeptide chain comprises the amino acid sequence set forth in SEQ ID NO: 87;

(4) one polypeptide chain comprises the amino acid sequence set forth in SEQ ID NO: 75, another polypeptide chain comprises the amino acid sequence set forth in SEQ ID NO: 85, and the other polypeptide chain comprises the amino acid sequence set forth in SEQ ID NO: 87;

(5) one polypeptide chain comprises the amino acid sequence set forth in SEQ ID NO: 79, another polypeptide chain comprises the amino acid sequence set forth in SEQ ID NO: 85, and the other polypeptide chain comprises the amino acid sequence set forth in SEQ ID NO: 87;

(6) one polypeptide chain comprises the amino acid sequence set forth in SEQ ID NO: 81, another polypeptide chain comprises the amino acid sequence set forth in SEQ ID NO: 85, and the other polypeptide chain comprises the amino acid sequence set forth in SEQ ID NO: 87; or

(7) one polypeptide chain comprises the amino acid sequence set forth in SEQ ID NO: 83, another polypeptide chain comprises the amino acid sequence set forth in SEQ ID NO: 85, and the other polypeptide chain comprises the amino acid sequence set forth in SEQ ID NO: 87;

preferably, the multispecific antibody is afucosylated.

15. An isolated nucleic acid, comprising a nucleotide sequence encoding the multispecific antibody according to any one of claims 1-14.

16. A method for preparing the multispecific antibody according to any one of claims 1-14, comprising: culturing a host cell comprising the nucleic acid according to claim 15 to express the multispecific antibody, and isolating and purifying the multispecific antibody from the system.

17. An antibody-drug conjugate, comprising the multispecific antibody according to any one of claims 1-14 and a cytotoxic drug.

18. A pharmaceutical composition, comprising the multispecific antibody according to any one of claims 1-14 or the antibody-drug conjugate according to claim 17, and a pharmaceutically acceptable excipient.

19. A method for treating a disease expressing c-Met or/and EGFR, comprising: administering to a subject in need a therapeutically effective amount of the multispecific antibody according to any one of claims 1-14, the antibody-drug conjugate according to claim 17, or the pharmaceutical composition according to claim 18, wherein preferably, the disease is a tumor;

preferably, the tumor is epithelial cell cancer, breast cancer, ovarian cancer, lung cancer, lung adenocarcinoma, colorectal cancer, anal cancer, prostate cancer, kidney cancer, liver cancer, bladder cancer, head and neck cancer, gastric cancer, pancreatic cancer, skin cancer, oral cancer, pharyngeal cancer, nasal cancer, tongue cancer, esophageal cancer, testicular cancer, vaginal cancer, cervical cancer, spleen cancer, testicular cancer, thyroid cancer,
salivary gland cancer, or thymus cancer;
preferably, the lung cancer is non-small cell lung cancer or small cell lung cancer;
optionally, the method further comprises: further administering to the subject in need one or more additional therapeutic agents.

20. A c-Met-binding antibody, comprising a single variable domain, wherein the single variable domain comprises:

(1) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6, wherein $X_1$ is S or T;
(2) a CDR1 comprising the amino acid sequence set forth in SEQ ID NO: 7, a CDR2 comprising the amino acid sequence set forth in SEQ ID NO: 8, and a CDR3 comprising the amino acid sequence set forth in SEQ ID NO: 9;
(3) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;
(4) a CDR1 comprising the amino acid sequence set forth in SEQ ID NO: 10, a CDR2 comprising the amino acid sequence set forth in SEQ ID NO: 11, and a CDR3 comprising the amino acid sequence set forth in SEQ ID NO: 12;
(5) a CDR1 comprising the amino acid sequence set forth in SEQ ID NO: 13, a CDR2 comprising the amino acid sequence set forth in SEQ ID NO: 14, and a CDR3 comprising the amino acid sequence set forth in SEQ ID NO: 15;
(6) a CDR1 comprising the amino acid sequence set forth in SEQ ID NO: 16, a CDR2 comprising the amino acid sequence set forth in SEQ ID NO: 17, and a CDR3 comprising the amino acid sequence set forth in SEQ ID NO: 18;
(7) a CDR1 comprising the amino acid sequence set forth in SEQ ID NO: 19, a CDR2 comprising the amino acid sequence set forth in SEQ ID NO: 20, and a CDR3 comprising the amino acid sequence set forth in SEQ ID NO: 21; or
(8) a CDR1 comprising the amino acid sequence set forth in SEQ ID NO: 22, a CDR2 comprising the amino acid sequence set forth in SEQ ID NO: 23, and a CDR3 comprising the amino acid sequence set forth in SEQ ID NO:24;
preferably, the single variable domain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40, 41, 35, 36, 26, 27, 25, 28, 29, 30, 31, 32, 33, 34, 37, 38, 39, or 42;
more preferably, the single variable domain comprises the amino acid sequence set forth in SEQ ID NO: 35, 36, 33, 34, 37, 38, or 39;
further preferably, the single variable domain comprises the amino acid sequence set forth in SEQ ID NO: 40, 41, or 42, but does not comprise the amino acid sequence set forth in SEQ ID NO: 29, 30, or 31;
further preferably, the single variable domain is derived from a camelid or is humanized;
further preferably, the c-Met-binding antibody comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 44, 45, 43, 46, 47, 48, 49, 50, 51, 53, 55, 57, or 59.

FIG. 1A

FIG. 1B

FIG. 1C

FIG. 1D

## MKN45

FIG. 1E

## NCI-H1975

FIG. 1F

## NCI-H292

FIG. 1G

FIG. 2

A431

FIG. 3A

NCI-H1975

FIG. 3B

NCI-H1993

FIG. 3C

FIG. 4

FIG. 5

FIG. 6A

HGF Conc.: 10 ng/mL

FIG. 6B

HGF Conc.: 1 ng/mL

FIG. 6C

NCI-H292

FIG. 7A

**KP4**

FIG. 7B

**NCI-H292**

FIG. 8A

**KP4**

FIG. 8B

## NCI-H441

FIG. 9A

## A431

FIG. 9B

## NCI-H1975

FIG. 9C

## INTERNATIONAL SEARCH REPORT

| International application No. |
| --- |
| **PCT/CN2024/089297** |

| A. | CLASSIFICATION OF SUBJECT MATTER |
| --- | --- |

C07K16/46(2006.01)i; C07K16/28(2006.01)i; C07K16/30(2006.01)i; A61K39/395(2006.01)i; C12N15/13(2006.01)i; A61P35/00(2006.01)i

According to International Patent Classification (IPC) or to both national classification and IPC

| B. | FIELDS SEARCHED |
| --- | --- |

Minimum documentation searched (classification system followed by classification symbols)

IPC: C07K A61K C12N A61P

Documentation searched other than minimum documentation to the extent that such documents are included in the fields searched

Electronic data base consulted during the international search (name of data base and, where practicable, search terms used)

CNABS, CNTXT, ENTXTC, ENTXT, WOTXT, USTXT, EPTXT, CNKI, 万方, WANFANG, 百度学术, Baidu Scholar, PUBMED, ISI WEB OF KNOWLEDGE, BING, GENBANK, EMBL, STN, 中国专利生物序列检索, China Patent Biological Sequence Search, c-Met, 间质表皮转化因子, 肝细胞生长因子受体, 多特异性, 三特异性, 多价, 单抗, 抗原结合, EGFR, 表皮生长因子受体, 单可变结构域, 单域抗体, 纳米抗体, 骆驼抗体, multispecific, antibody, antigen binding, nanobody, VHH, SEQ ID NOs: 1-88

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | WO 2023078391 A1 (CHIA TAI TIANQING PHARMACEUTICAL GROUP CO., LTD.) 11 May 2023 (2023-05-11) description, page 1, line 30 to page 2, line 24, and tables S1 and S2 | 20 |
| PY | WO 2023078391 A1 (CHIA TAI TIANQING PHARMACEUTICAL GROUP CO., LTD.) 11 May 2023 (2023-05-11) description, page 1, line 30 to page 2, line 24, and tables S1 and S2 | 1-19 |
| PX | WO 2023078393 A1 (CHIA TAI TIANQING PHARMACEUTICAL GROUP CO., LTD.) 11 May 2023 (2023-05-11) description, page 1, line 30 to page 2, line 21, and tables S1 and S2 | 20 |
| PY | WO 2023078393 A1 (CHIA TAI TIANQING PHARMACEUTICAL GROUP CO., LTD.) 11 May 2023 (2023-05-11) description, page 1, line 30 to page 2, line 21, and tables S1 and S2 | 1-19 |

✓ Further documents are listed in the continuation of Box C.  ✓ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 July 2024** | **02 August 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/089297** |

### C. DOCUMENTS CONSIDERED TO BE RELEVANT

| Category* | Citation of document, with indication, where appropriate, of the relevant passages | Relevant to claim No. |
|---|---|---|
| Y | WO 2022104236 A2 (AB THERAPEUTICS, INC.) 19 May 2022 (2022-05-19) description, page 3, line 10 to page 5, line 6, page 13, lines 9-30, page 19, lines 28-29, and page 47, line 5 to page 48, line 8 | 1-19 |
| A | CN 103889451 A (ABLYNX NV) 25 June 2014 (2014-06-25) description, paragraphs 12-18 and 143-146 | 1-20 |
| A | CN 1610695 A (GENMAB INC.) 27 April 2005 (2005-04-27) claims 1-52 | 1-20 |
| A | CN 113840842 A (JANSSEN BIOTECH, INC.) 24 December 2021 (2021-12-24) claims 1-70 | 1-20 |
| A | CN 114702590 A (XIDIAN UNIVERSITY) 05 July 2022 (2022-07-05) description, paragraphs 4-22 | 1-20 |
| A | WO 2022218380 A1 (CHIA TAI TIANQING PHARMACEUTICAL GROUP CO., LTD.) 20 October 2022 (2022-10-20) claims 1-17 | 1-20 |
| A | CN 112794916 A (CHIA TAI TIANQING PHARMACEUTICAL GROUP NANJING SHUNXIN PHARMACEUTICAL CO., LTD. et al.) 14 May 2021 (2021-05-14) description, paragraphs 8-18 | 1-20 |
| A | DU, Q.L. et al. "Abstract LB538: Characterization of GB263T, a Tri-specificantibody against EGFR/cMET/cMET for NSCLC" *Cancer Research*, Vol. 82, No. (12_Supplement), 15 June 2022 (2022-06-15), LB538 | 1-20 |
| A | MOORES, S.L. et al. "A Novel Bispecific Antibody Targeting EGFR and cMet Is Effective against EGFR Inhibitor-Resistant Lung Tumors" *Cancer Research*, Vol. 76, No. (13), 01 July 2016 (2016-07-01), pp. 3942-3953 | 1-20 |

Form PCT/ISA/210 (second sheet) (July 2022)

**INTERNATIONAL SEARCH REPORT**

International application No.

**PCT/CN2024/089297**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**EP 4 703 390 A1**

| INTERNATIONAL SEARCH REPORT | International application No. |
|---|---|
| | **PCT/CN2024/089297** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **19**
   because they relate to subject matter not required to be searched by this Authority, namely:

   Claim 19 relates to a method for treating a disease expressing c-Met or/and EGFR, that is, claim 19 relates to subject matter for which no search is required by the International Searching Authority as defined in PCT Rule 39.1: (4) methods for treatment of the human or animal body by surgery or therapy, as well as diagnostic methods. The international search is carried out on the basis of the use of a multispecific antibody, an antibody-drug conjugate or a pharmaceutical composition in the preparation of a drug for treating a disease expressing c-Met or/and EGFR.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/089297** |

| Patent document cited in search report | | | Publication date (day/month/year) | Patent family member(s) | | | Publication date (day/month/year) |
|---|---|---|---|---|---|---|---|
| WO | 2023078391 | A1 | 11 May 2023 | KR | 20240099302 | A | 28 June 2024 |
| | | | | TW | 202325729 | A | 01 July 2023 |
| | | | | CN | 118139889 | A | 04 June 2024 |
| WO | 2023078393 | A1 | 11 May 2023 | CN | 118159567 | A | 07 June 2024 |
| WO | 2022104236 | A2 | 19 May 2022 | WO | 2022104236 | A3 | 09 March 2023 |
| | | | | EP | 4243938 | A2 | 20 September 2023 |
| | | | | US | 2023399408 | A1 | 14 December 2023 |
| | | | | JP | 2023549537 | A | 27 November 2023 |
| CN | 103889451 | A | 25 June 2014 | AU | 2013201446 | A1 | 02 May 2013 |
| | | | | AU | 2013201446 | A8 | 20 June 2013 |
| | | | | AU | 2013201446 | B2 | 10 March 2016 |
| | | | | EP | 2747783 | A2 | 02 July 2014 |
| | | | | EP | 2747783 | B1 | 14 June 2017 |
| | | | | CA | 2850261 | A1 | 04 April 2013 |
| | | | | CA | 2850261 | C | 20 April 2021 |
| | | | | JP | 2015501135 | A | 15 January 2015 |
| | | | | JP | 6219287 | B2 | 25 October 2017 |
| | | | | WO | 2013045707 | A2 | 04 April 2013 |
| | | | | WO | 2013045707 | A3 | 13 June 2013 |
| CN | 1610695 | A | 27 April 2005 | AU | 2002345673 | B2 | 26 April 2007 |
| | | | | CA | 2450285 | A1 | 19 December 2002 |
| | | | | CA | 2450285 | C | 02 August 2016 |
| | | | | KR | 20040016883 | A | 25 February 2004 |
| | | | | KR | 100945108 | B1 | 02 March 2010 |
| | | | | HUP | 0600225 | A2 | 28 June 2006 |
| | | | | HUP | 0600225 | A3 | 28 January 2010 |
| | | | | MXPA | 03011365 | A | 07 March 2005 |
| | | | | KR | 20090125840 | A | 07 December 2009 |
| | | | | JP | 2005501529 | A | 20 January 2005 |
| | | | | JP | 4298498 | B2 | 22 July 2009 |
| | | | | JP | 2009148282 | A | 09 July 2009 |
| | | | | IL | 159225 | A0 | 01 June 2004 |
| | | | | HK | 1064685 | A1 | 04 February 2005 |
| | | | | CZ | 200438 | A3 | 16 June 2004 |
| | | | | BR | 0210405 | A | 19 April 2005 |
| | | | | BRPI | 0210405 | B1 | 27 November 2018 |
| | | | | BRPI | 0210405 | B8 | 25 May 2021 |
| | | | | WO | 02100348 | A2 | 19 December 2002 |
| | | | | WO | 02100348 | A3 | 27 February 2003 |
| | | | | WO | 02100348 | A8 | 10 April 2003 |
| | | | | NZ | 530212 | A | 29 September 2006 |
| | | | | IL | 159225 | A | 01 September 2009 |
| | | | | EP | 1417232 | A2 | 12 May 2004 |
| | | | | EP | 1417232 | A4 | 27 April 2005 |
| | | | | EP | 1417232 | B1 | 03 December 2014 |
| | | | | RU | 2004100834 | A | 27 March 2005 |
| | | | | RU | 2335507 | C2 | 10 October 2008 |
| | | | | US | 2003091561 | A1 | 15 May 2003 |
| | | | | US | 7247301 | B2 | 24 July 2007 |

Form PCT/ISA/210 (patent family annex) (July 2022)

## INTERNATIONAL SEARCH REPORT
### Information on patent family members

International application No.

**PCT/CN2024/089297**

| Patent document cited in search report | | | Publication date (day/month/year) | Patent family member(s) | | | Publication date (day/month/year) |
|---|---|---|---|---|---|---|---|
| CN | 113840842 | A | 24 December 2021 | US | 2023130600 | A1 | 27 April 2023 |
| | | | | MX | 2021010265 | A | 23 September 2021 |
| | | | | AU | 2020229467 | A1 | 12 August 2021 |
| | | | | SG | 11202108311 | RA | 29 September 2021 |
| | | | | JOP | 20210233 | A1 | 30 January 2023 |
| | | | | IL | 285718 | A | 31 October 2021 |
| | | | | BR | 112021016149 | A2 | 13 October 2021 |
| | | | | EP | 3931224 | A2 | 05 January 2022 |
| | | | | EP | 3931224 | A4 | 01 March 2023 |
| | | | | CA | 3131654 | A1 | 03 September 2020 |
| | | | | US | 2020270351 | A1 | 27 August 2020 |
| | | | | US | 11459391 | B2 | 04 October 2022 |
| | | | | JP | 2022521610 | A | 11 April 2022 |
| | | | | MA | 55089 | A | 05 January 2022 |
| | | | | WO | 2020174370 | A2 | 03 September 2020 |
| | | | | WO | 2020174370 | A3 | 03 December 2020 |
| | | | | KR | 20210132117 | A | 03 November 2021 |
| CN | 114702590 | A | 05 July 2022 | None | | | |
| WO | 2022218380 | A1 | 20 October 2022 | KR | 20230170721 | A | 19 December 2023 |
| | | | | US | 2024182592 | A1 | 06 June 2024 |
| | | | | TW | 202304963 | A | 01 February 2023 |
| | | | | MX | 2023011923 | A | 23 October 2023 |
| | | | | EP | 4324853 | A1 | 21 February 2024 |
| | | | | IL | 307308 | A | 01 November 2023 |
| | | | | BR | 112023020341 | A2 | 06 February 2024 |
| | | | | CA | 3214012 | A1 | 20 October 2022 |
| | | | | AU | 2022256638 | A1 | 09 November 2023 |
| | | | | JP | 2024516098 | A | 12 April 2024 |
| CN | 112794916 | A | 14 May 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019195535 A
- US 20200079872 A1

- CN 100497389 C

**Non-patent literature cited in the description**

- **ELVIN A. KABAT et al.** Sequences of Proteins of Immunological Interest. 1991
- **CYRUS CHOTHIA et al.** Canonical Structures for the Hypervariable Regions of Immunoglobulins. *J. Mol. Biol.*, 1987, vol. 196, 901-917

- **KABAT, E.A. et al.** Sequences of Proteins of Immunological Interest. NIH Publication, 1991, 91-3242